# EUROPEAN PATENT APPLICATION

(11) **EP 2 308 959 A1**
(43) Date of publication of application: **13.04.2011**
(21) Application number: 09172567.1
(22) Date of filing: 08.10.2009
(51) Int. Cl.: C12N 1/20, C12N 15/31, C12N 15/52, C12N 9/00, C12N 9/02, C12N 9/16, C12N 9/88, C07K 14/195, C12P 19/32, A62D 3/02, C12R 1/01

(54) **Novel microorganism and its use in lignocellulose detoxifixcation**

(71) Applicant: Shell Internationale Research Maatschappij B.V., 2596 HR The Hague (NL)
(72) Inventor: The designation of the inventor has not yet been filed
(74) Representative: Matthezing, Robert Maarten

(57) **Abstract**

The present invention relates to an isolated microorganism which is Cupriavidus basilensis strain HMF14 Deposit number DSM 22875, and its use in a process for the in-situ detoxification of lignocelluloses hydrolysate

SEQUENCE LISTING

<110>  Shell Internationale Research Maatschappij B.V.

<120>  A novel microorganism and its use in lignocellulose
       detoxification

<130>  TS9726

<160>  46

<170>  PatentIn version 3.5

<210>  1
<211>  20
<212>  DNA
<213>  Artificial Sequence

<220>
<223>  Primer

<400>  1
gcacgcgcct gagttacgac                                                    20


<210>  2
<211>  19
<212>  DNA
<213>  Artificial Sequence

<220>
<223>  Primer

<400>  2
catgctcggc gctggtgac                                                     19


<210>  3
<211>  29
<212>  DNA
<213>  Artificial Sequence

<220>
<223>  Primer

<400>  3
catgaattcc gacccaggag tcacgccat                                          29


<210>  4
<211>  36
<212>  DNA
<213>  Artificial Sequence

<220>
<223>  Primer

<400>  4
cggcggccgc ggatataccg acaatgatgc gtctct                                  36


<210>  5
<211>  34
<212>  DNA
<213>  Artificial Sequence

<220>
<223>  Primer

<400>  5
cggcggccgc tgtctcctgc ctgttcagca ttca                                34

<210>  6
<211>  33
<212>  DNA
<213>  Artificial Sequence

<220>
<223>  Primer

<400>  6
gcgggcccct tactccttga tggtatcgac agg                                 33

<210>  7
<211>  26
<212>  DNA
<213>  Artificial Sequence

<220>
<223>  Primer

<400>  7
gcggtaccgg gagggccggt catgag                                         26

<210>  8
<211>  28
<212>  DNA
<213>  Artificial Sequence

<220>
<223>  Primer

<400>  8
gcggtaccgg cgtagatacc caggaggc                                       28

<210>  9
<211>  28
<212>  DNA
<213>  Artificial Sequence

<220>
<223>  Primer

<400>  9
gcgggccccc acgctttgca ggaaggtg                                       28

<210>  10
<211>  26
<212>  DNA
<213>  Artificial Sequence

<220>
<223>  Primer

<400>  10
cggaattccg gcgcatgtgt tcacgc                                         26

<210>  11
<211>  29
<212>  DNA

<213>  Artificial Sequence

<220>
<223>  Primer

<400>  11
gcgcggccgc cgcccgcgat ttccatcag                                          29


<210>  12
<211>  32
<212>  DNA
<213>  Artificial Sequence

<220>
<223>  Primer

<400>  12
gcggtacccc atcaaggagt aagacatgac cc                                      32


<210>  13
<211>  28
<212>  DNA
<213>  Artificial Sequence

<220>
<223>  Primer

<400>  13
cggaattcca catgacaagg ggagaccg                                           28


<210>  14
<211>  30
<212>  DNA
<213>  Artificial Sequence

<220>
<223>  Primer

<400>  14
cggaattcgc ttcggtcttc aactcggatg                                         30


<210>  15
<211>  500
<212>  PRT
<213>  Aldehyde Dehydrogenase, Species Cupriavidus basilensis HMF 14

<400>  15

Met Asn Ala Gln His Trp Ile Ala Gly Ala Trp Thr Gly Glu Pro Ser
1               5                   10                  15


Ala Asp Ser Val Asn Pro Ala Asp Gly Thr Leu Ile Gly Gln Phe Ala
            20                  25                  30


Asp Gly Gly Thr Trp Gln Ala Glu Ala Ala Ile Ala Ala Ala Arg His
        35                  40                  45


Val Phe Glu Arg Thr Thr Trp Gly Gln Asp Ala Arg Leu Arg Gln Asp
    50                  55                  60

Val Leu Leu Ala Trp Ala Gly Ala Leu Glu Ala Glu Arg Glu Arg Leu
65                  70                  75                  80

Ala Ser Leu Leu Thr Ala Glu Asn Gly Lys Pro Val Ala Gln Ala Arg
                85                  90                  95

Gly Glu Val Gly Ala Ala Ile Ser Glu Val Arg Tyr Tyr Ala Gly Leu
            100                 105                 110

Ala Arg His Ile Pro Gly His Val Leu Glu Pro Glu Pro Gly Thr Ile
        115                 120                 125

Ser Thr Ile Leu Arg Glu Pro Ala Gly Val Ala Ala Ile Ile Val Pro
    130                 135                 140

Trp Asn Ala Pro Ala Val Leu Leu Val Arg Ser Leu Ala Pro Ala Leu
145                 150                 155                 160

Ala Ala Gly Cys Thr Ala Val Val Lys Ser Ala Ala Gln Thr Thr Leu
                165                 170                 175

Phe Thr Ala Ala Met Leu Arg Leu Phe Glu Arg Thr Ala Leu Pro Ala
            180                 185                 190

Gly Ala Val Asn Leu Val Cys Glu Thr Gly Tyr Ala Ala Ala Asp His
        195                 200                 205

Leu Val Arg Ser Arg Asp Val Asp Val Val Ser Phe Thr Gly Ser Thr
    210                 215                 220

Ala Thr Gly Lys Lys Ile Met Ile Ala Ala Ala Asp Ser Val Lys Lys
225                 230                 235                 240

Leu Ser Leu Glu Leu Gly Gly Lys Ser Cys Cys Leu Val Phe Asp Asp
                245                 250                 255

Val Asp Ala Gln Ala Val Ala Lys Arg Leu Ala Leu Ala Ala Thr Val
            260                 265                 270

Ile Ser Gly Gln Gln Cys Thr Ala Ala Arg Arg Val Leu Val His Glu
        275                 280                 285

Ala Ile Ala Pro Gln Met Arg Arg His Leu Thr Glu Ala Leu Ala Ala
    290                 295                 300

Leu Arg Leu Gly Pro Gly Ile Glu Pro Asp Thr Gln Ile Gly Pro Leu
305                 310                 315                 320

Ile Asp His Pro Thr Arg Ala Met Val Ser Ala Gln Val Glu Arg Ala
                325                 330                 335

Cys Asp Glu Ala Asp Thr Val Leu Leu Arg Gly Thr Met Pro Gly Gly
            340                 345                 350

Ala Leu Ala Arg Gly Ala Phe Leu Ser Pro Thr Leu Val Glu His Ser
        355                 360                 365

Asp Pro Gly Ala Phe Phe Cys Gln Glu Glu Ile Phe Gly Pro Phe Val
    370                 375                 380

Thr Phe Glu Thr Phe Ala Thr Glu Asp Glu Ala Leu Ala Lys Ala Asn
385                 390                 395                 400

Asn Thr Val Phe Gly Leu Ser Ala Ser Val Trp Thr His His Gly Glu
                405                 410                 415

Arg Ala Ile Arg Leu Ala Arg Ala Leu Arg Asn Gly Thr Val Trp Val
            420                 425                 430

Asn Asp His Asn Arg Leu Phe Ala Glu Ala Glu Thr Gly Gly Tyr Arg
        435                 440                 445

Gln Ser Gly Leu Gly Arg Leu His Gly Tyr Asp Ala Leu Ala Asp Phe
    450                 455                 460

Thr Glu Leu Lys His Ile Cys Ile Gln Ala Gly Leu Pro Lys Gly Met
465                 470                 475                 480

Ser Gln Ala Gly Cys Arg Leu Ser Gly Val Ala Ala Arg Glu Arg Met
                485                 490                 495

Gly Val Ser Val
            500

<210>  16
<211>  1503
<212>  DNA
<213>  Aldehyde Dehydrogenase, Species Cupriavidus basilensis HMF 14

<400>  16
atgaacgcgc aacactggat tgccggcgcc tggaccggcg agccttccgc cgatagcgtc      60

aaccccgccg acgggaccct gatcgggcag ttcgcggacg gcggcacctg gcaagccgaa     120

gccgccatcg ccgccgcgcg ccatgtcttc gagcgcacca cctggggcca ggatgcccgc     180

ctgcgccagg acgtgcttct agcctgggct ggtgcgctcg aggcagagcg agagcgcctg     240

gccagcctgc tcaccgcgga aaacggcaag ccggtcgcac aagcccgagg cgaggtcggc     300

gccgcaattt cagaggtccg ctattacgcc gggctggcgc ggcacatccc gggtcacgtg     360

ctggagcccg agccaggcac gatatcgacc atcctgcgcg agccggccgg cgtcgccgcc     420

atcatcgtcc cctggaacgc gccggcggtg ctgctcgtgc gctccctcgc gccagcgctt     480

gccgcgggct gcacggcagt ggtcaaatcg gcagcgcaaa ccacgctgtt cacagccgca     540

atgctgcgct tgttcgagcg cacggccctg ccggccggcg ccgtcaatct ggtctgcgaa 600
acgggctatg cggcagcgga ccacctggtg cgttcgcgcg acgtggacgt agtgagcttc 660
acaggatcga ccgcaaccgg caagaagatc atgatcgccg ctgcggacag cgtgaaaaaa 720
ctctcgctgg aactcggcgg gaaatcgtgc tgcctggtgt tcgacgacgt cgatgcgcaa 780
gcggtcgcga aacggcttgc gcttgccgcc accgtcatct cgggccagca atgcaccgcc 840
gcgcggcgag tactggttca cgaagccatc gcgccacaga tgcgccggca cctgaccgag 900
gccctcgccg cgctgcgcct cgggcccggc atcgagcccg acacccaaat cggcccgctg 960
atcgaccacc cgacgcgcgc gatggtgagc gcgcaagtcg agcgcgcctg cgacgaggcg 1020
gacacggtcc tgctgcgcgg cacgatgccg ggcggcgcgc tagcgcgcgg cgccttcctc 1080
agccccacac tagtggaaca cagcgacccc ggtgccttct tctgccagga ggagatcttc 1140
gggcccttcg tcacattcga gaccttcgcg accgaagacg aggcgctagc caaggccaac 1200
aacaccgtct tcggcctgtc cgccagcgtc tggacgcacc acggcgagcg cgccatacgc 1260
ctagcgcggg cgctgcgcaa cggcacggtc tgggtcaacg accacaaccg cctgttcgcc 1320
gaagcggaga cgggcggcta tcggcaaagc ggccttggac ggctccacgg ttatgacgcc 1380
ctcgcggact tcaccgagtt gaagcacatc tgcatccagg cgggcctgcc gaaagggatg 1440
tcgcaggcgg gctgcaggct cagtggggta gcagcgcgcg agcggatggg agtttccgtc 1500
tag 1503

<210> 17
<211> 320
<212> PRT
<213> LysR-type transcriptional regulator, Species Cupriavidus basilensis HMF
14

<400> 17

Met Gly Gln Leu Gly His Met Asp Leu Lys Gln Ile Gln Tyr Phe Ile
1               5                   10                  15

Ala Leu Phe Glu Asp Gly Ser Val Thr Arg Ala Ala Lys Arg Leu His
            20                  25                  30

Ile Val Gln Pro Ala Leu Ser Met Gln Ile Ala Arg Leu Glu Glu Glu
        35                  40                  45

Leu Asn Gln Lys Leu Phe Glu Arg Gly Ala His Gly Met Ser Pro Thr
    50                  55                  60

Ala Ala Gly Arg Gln Met Tyr Arg Leu Phe Leu Pro Ile Met Arg Asp
65                  70                  75                  80

Ile Thr His Ala Arg Glu Gln Leu Val Gln Arg Asp Glu Val Val Ser
                85                  90                  95

Gly Asn Ile Thr Ile Gly Leu Ile Ala Ser Ile Ala Glu Gly Ala Leu

            100                 105                 110

Ala Glu Ala Leu Thr Ser Phe Arg Met Arg Tyr Pro Gln Val Glu Val
        115                 120                 125

Thr Val Ala Asp Gly Tyr Ser Thr Thr Leu Ile Asp Trp Val Ala Gly
    130                 135                 140

Gly Gln Leu Asp Val Ala Ile Ile Asn Lys Pro Arg Ala Gln Leu Ser
145                 150                 155                 160

Leu Asp Ser Arg Pro Leu Leu Asp Glu Glu Met Val Leu Ala Thr Ser
                165                 170                 175

Ala Ala His Gly Leu Asp Leu Pro Gly Ser Val Gln Leu Ala Ser Leu
            180                 185                 190

Pro Ala Val Glu Leu Val Leu Pro Thr Lys Arg His Gly Leu Arg Gly
        195                 200                 205

Val Leu Asp Ser Ala Ala His Gln Leu Asp Met Met Leu Thr Pro Lys
    210                 215                 220

Tyr Glu Ile Asp Ala Leu Gly Thr Ile Val Lys Leu Val Ala Ser Thr
225                 230                 235                 240

Asn Met Ala Thr Ile Leu Pro Arg Ile Ala Val Gln Arg Ala Val Asp
                245                 250                 255

Arg Gly Thr Leu Arg Val His Ala Ile Leu Ala Pro Arg Leu Ile Arg
            260                 265                 270

His Ile Val Arg Ile Ser His Pro Arg Arg Pro Leu Ser Thr Ala Ala
        275                 280                 285

Glu Ala Leu Val Ser Ile Ile Ala Ser Glu Ile Gln Arg Thr Ser Asp
    290                 295                 300

Ala Ala Thr Pro Ala Gly Gly Gln Asp Leu Asn Lys Glu Lys Met Glu
305                 310                 315                 320

<210> 18
<211> 963
<212> DNA
<213> LysR-type transcriptional regulator, Species Cupriavidus basilensis HMF
14

<400> 18
atggggcaac tcgggcatat ggatttaaag cagatccagt acttcatcgc gctcttcgag 60

gacggatcag tcactcgggc cgccaagcgg ctgcatatcg tgcagcccgc gctgagcatg 120

cagatcgcca ggctggaaga agagctgaat caaaaactgt tcgagcgcgg cgcgcatggc 180

atgtcgccga ccgccgcggg ccgccagatg tatcggctgt tcctgcccat catgcgcgac 240

atcacgcatg cgcgcgaaca actggtgcaa cgggacgagg tcgtctccgg aaacatcacg 300

atcggactga tcgcctccat cgccgagggt gctctcgccg aagcactgac cagctttcgt 360

atgcgctatc cccaggtcga ggtgaccgtg gccgacggct acagcaccac gctgatcgac 420

tgggtagcgg gcggccagct ggacgtggcc attatcaaca agccgcgcgc ccagttgtca 480

ctcgactccc ggccgctgct cgacgaagaa atggtgctgg caacgagcgc ggcccacggc 540

ctggacctgc ccggctcggt ccagctagcc agcctccccg cggtggaact ggtgctgccc 600

accaaacgcc acggcctgcg cggcgtgctc gacagcgcgg cgcaccagtt ggacatgatg 660

ctcacgccca agtacgagat cgacgcgctc ggcaccatcg tcaagcttgt tgcatcaacc 720

aacatggcca cgatcctgcc gcgcatcgcg gtgcagcgcg cggtggaccg gggcacgttg 780

cgcgtgcacg ccatcctggc accacggcta atccggcaca tcgtgcgcat cagccacccc 840

cgccgcccct tgagcacagc ggccgaggcg ctggtcagca tcattgccag cgagatccag 900

cgtacgtcgg acgcggcaac gcccgccggc ggccaagact taaacaagga gaagatggaa 960

tga 963

<210> 19
<211> 497
<212> PRT
<213> 5-hydroxymethyl-2-furoic acid decarboxylase I, Species Cupriavidus
basilensis HMF 14

<400> 19

Met Ser Arg Gln Pro Ala Glu Lys Thr Asn Ser Ala Ser Gln Ala Ala
1               5                   10                  15

Pro Leu Pro Glu Gly Pro Leu Thr Leu Arg Ser Trp Leu Arg His Leu
            20                  25                  30

Gly Asn Thr Asp Arg Leu Ala Ala Ile Asp Glu Pro Val Ala Leu Glu
        35                  40                  45

His Thr Leu Ala Ala Val Ala Lys Arg Leu Asp Gly Glu Arg Ala Val
    50                  55                  60

Leu Phe Arg Arg Pro Gly Gly His Ala Val Pro Val Val Ser Gly Phe
65                  70                  75                  80

Met Ser Arg Arg Ala Trp Ile Ala Glu Ala Met Gly Val Pro Glu Ala
                85                  90                  95

Gly Leu Leu Glu Arg Met Arg Ser Ala Ala Gln Pro Leu Pro Val
            100                 105                 110

Ser Glu Val Ala Gln Gly Glu Ala Ala Cys Gln Gln Val Ile His Leu
            115                 120                 125

Asp Lys Val Asp Leu His Lys Leu Leu Pro Ile Pro Thr His Ser Glu
    130                 135                 140

His Asp Asn Gly Pro Tyr Ile Thr Ala Gly Leu Ala Ile Ala Arg Asn
145                 150                 155                 160

Pro Arg Thr Gly Val Gln Asn Val Ser Ile His Arg Ile Gln Val His
                165                 170                 175

Ala Ala Asp Arg Met Ala Ile Leu Leu Leu Pro Arg His Leu Asp Ala
            180                 185                 190

Phe Tyr Arg Ala Ala Glu Glu Cys Gly Glu Ala Leu Pro Ile Ala Ile
        195                 200                 205

Val Ile Gly Val Asp Pro Leu Thr Met Leu Ala Ser Gln Ala Ile Thr
    210                 215                 220

Pro Ile Asp Tyr Asp Glu Leu Glu Ile Ala Gly Ala Leu His Gly Ala
225                 230                 235                 240

Pro Leu Glu Val Ile Lys Cys Arg Thr Ser Asp Val Arg Val Pro Ala
                245                 250                 255

Asn Ala Glu Ile Val Ile Glu Gly Arg Leu Leu Pro Gly Glu Arg Glu
            260                 265                 270

Met Glu Gly Pro Phe Gly Glu Phe Pro Lys Tyr Tyr Ser Ser Ala Glu
        275                 280                 285

Pro Arg Glu Val Ile Gln Val Asp Ala Val Thr His Arg His Arg Pro
    290                 295                 300

Ile Tyr His Thr Ile Val Pro Ala Glu Met Glu His Leu Leu Leu Gly
305                 310                 315                 320

Ala Ile Pro Arg Glu Ala Thr Leu Leu Ala His Leu Gln Arg Ser His
                325                 330                 335

Pro Gly Val Gln Asp Val His Leu Ser Val Gly Gly Val Cys Arg Tyr
            340                 345                 350

His Leu Tyr Val Lys Leu Asp Lys Lys Arg Glu Gly Glu Ala Lys Asn
        355                 360                 365

Val Ile Leu Ser Ala Phe Gly Ala His Tyr Asp Ile Lys Gln Val Val
    370                 375                 380

Val Val Asp Thr Asp Val Asp Val His Asp Pro Ala Glu Val Glu Trp
385                 390                 395                 400

Ala Val Ala Thr Arg Phe Gln Ala Asp Gln Asp Leu Val Val Ile Ala
405 410 415

Gly Ala Gln Gly Ser Val Leu Asp Pro Ser Thr Thr Val Ala Ala Asn
                420                 425                 430

Leu Ala Gly Ile Asp Asn Pro Glu Pro His Leu Gln Gly Ile Cys Ala
            435                 440                 445

Lys Met Gly Leu Asp Ala Thr Arg Pro Val Lys Tyr Ala Ala His Val
        450                 455                 460

Phe Thr Arg Val Arg Ile Pro Gly Glu Ser Thr Ile Asp Leu Gln Ala
465 470 475 480

Leu Val Ser Val Asp Pro Ser Gln Trp Glu Ala Tyr Leu Gly Glu Gly
485 490 495

Ala

<210> 20
<211> 1494
<212> DNA
<213> 5-hydroxymethyl-2-furoic acid decarboxylase I, Species Cupriavidus basilensis HMF 14

<400> 20
atgtccaggc aacccgccga aaagacgaac agcgcgtcgc aagccgcgcc attgcccgaa 60

gggccgctga ccctgcgcag ctggctgcgc cacctcggca acacggaccg gctggctgca 120

atcgacgagc cggtggcact agagcacacc cttgcagccg tcgccaagcg gctcgatggc 180

gagcgcgcgg tgctcttccg ccggcctggc ggccatgccg ttccggttgt gagcgggttc 240

atgtcgcgcc gcgcgtggat cgccgaggcg atgggcgtgc ccgaggccgg cctgctcgag 300

cgcatgcgca gcgccgccgc acagcccttg ccggtgagcg aggtggccca gggcgaggcc 360

gcatgccagc aggtcatcca cctggacaag gtggacctac acaagctgtt gcccatcccg 420

acccacagcg agcatgacaa cggcccctat atcactgcag gcctggccat cgcgcgcaac 480

ccgcgcaccg gcgtgcagaa cgtatcgatc caccgcatcc aggtgcatgc cgcggatcgc 540

atggccatcc tgctgctgcc gcggcatctc gatgcgttct accgcgcggc ggaggaatgc 600

ggcgaggcgc tgccgattgc cattgtcatc ggtgtcgatc cactcaccat gcttgcttcg 660

caggccatca cgcctatcga ctatgacgag ttggagatcg ccggggcatt gcacggcgcg 720

ccgcttgaag tgatcaagtg ccgcaccagc gatgtgcgtg tgccggccaa tgccgagatc 780

gtgatcgagg gccggcttct gcccggcgag cgcgagatgg aagggccctt tggcgagttt 840

cccaagtact acagcagcgc cgagccgcgc gaggtcatcc aggtcgacgc cgtcacgcac 900

cgtcatcggc cgatctatca caccatcgtg ccggcggaga tggagcacct gctgctcggg 960

gcgattccgc gcgaagcgac cttgctggcg catctgcagc gcagccatcc cggggtgcag 1020

gatgtgcatc tgtcggtggg cggcgtatgc cggtaccacc tgtatgtaaa gctcgacaag 1080

aagcgcgagg gcgaagcgaa gaacgtcatt ctctcggcgt ttggcgcgca ctacgacatc 1140

aagcaggtgg tcgtggtgga taccgatgtc gacgtccacg acccggccga ggtggaatgg 1200

gccgtcgcga cccgcttcca ggcagaccag gacctggtgg tgatcgccgg ggcgcagggc 1260

tcggtgctcg acccctccac gaccgtcgcc gccaacctcg ccggcatcga caatcccgag 1320

ccgcacctgc aaggcatctg cgccaagatg gggctggacg cgacccgccc ggtcaagtac 1380

gcggcgcatg tgttcacgcg cgtgcggatt cccggcgagt caaccatcga tttgcaggcg 1440

ctggtgtcgg tcgacccatc gcaatgggaa gcgtatctcg gcgaaggagc ttga 1494

<210> 21
<211> 234
<212> PRT
<213> 5-hydroxymethyl-2-furoic acid decarboxylase II, Species Cupriavidus basilensis HMF 14

<400> 21

Met Cys Ser Arg Ala Cys Gly Phe Pro Ala Ser Gln Pro Ser Ile Cys
1               5                   10                  15

Arg Arg Trp Cys Arg Ser Thr His Arg Asn Gly Lys Arg Ile Ser Ala
            20                  25                  30

Lys Glu Leu Asp Val Met Ala Ser Gln Arg Arg Ile Ile Val Gly Ile
        35                  40                  45

Ser Gly Ala Ser Gly Ala Ala Ile Gly Val Asn Leu Leu Lys Ala Met
    50                  55                  60

Arg Gly Leu Asp Gly Val Glu Ser His Leu Ile Val Ser Ala Ser Gly
65                  70                  75                  80

Met Leu Thr Ala Thr Gln Glu Leu Gly Ile Lys Arg Ser Glu Leu Glu
                85                  90                  95

Ala Leu Ala Asp Val Val His Asn Val Arg Asp Ile Gly Ala Ala Val
            100                 105                 110

Ala Ser Gly Ser Phe Val Thr Glu Gly Met Val Val Ala Pro Cys Ser
        115                 120                 125

Met Lys Thr Leu Ala Ser Val Ala Asn Gly Phe Ser Asp Asn Leu Leu
    130                 135                 140

Thr Arg Ala Ala Asp Val Val Leu Lys Glu Arg Arg Arg Leu Val Leu
145                 150                 155                 160

Val Ala Arg Glu Thr Pro Leu Asn Leu Ala His Leu Arg Asn Met Leu

165                 170                 175

His Ala Thr Glu Met Gly Ala Ile Val Met Pro Pro Val Pro Ala Phe
            180                 185                 190

Tyr Ser His Pro Thr Ser Ile Glu Asp Val Val Asn His Thr Val Gly
        195                 200                 205

Arg Ile Leu Asp Leu Phe Gln Ile Glu His Gly Thr Leu Val Ser Arg
    210                 215                 220

Trp Ser Gly Leu Ala His Asp Phe Ala Arg
225                 230

<210>  22
<211>  705
<212>  DNA
<213>  5-hydroxymenthyl-2-furoic acid decarboxylase II, Species Cupriavidus
basilensis HMF 14

<400>  22
atgtgttcac gcgcgtgcgg attcccggcg agtcaaccat cgatttgcag gcgctggtgt      60

cggtcgaccc atcgcaatgg gaagcgtatc tcggcgaagg agcttgatgt catggccagc     120

cagagacgca tcattgtcgg tatatccggc gccagcggcg cggccatcgg cgtcaatctc     180

ctcaaggcga tgcgcggcct ggacggcgtc gagtcgcacc tcatcgtgtc ggcctccggc     240

atgctcaccg cgacccagga actcggcatc aagcgcagcg aactcgaggc gcttgccgac     300

gtagtgcata acgtgcgcga tattggcgca gcggtggcca gcggctcttt cgtgaccgag     360

ggcatggtgg tcgcgccgtg ctcgatgaag acactagcgt cggtggccaa cgggttttcc     420

gacaacctgc tgacccgcgc cgcggacgtg gtgctcaagg agcggcggcg cctggtgctg     480

gtggcgcgcg aaaccccgct gaacctcgcg cacctgcgca acatgctgca cgccaccgag     540

atgggcgcga tcgtgatgcc gcccgtgccc gccttctact cgcatccgac cagcatcgag     600

gatgtggtca atcacaccgt gggccgcatc ctggacctgt tccagatcga gcacggcacg     660

ctggtcagcc gctggtcggg cctcgctcac gactttgccc gctga                     705

<210>  23
<211>  328
<212>  PRT
<213>  Extracytoplasmic solute receptor; probable orphan gene, Species
Cupriavidus basilensis HMF 14

<400>  23

Met Leu Asn Arg Gln Glu Thr Asp Met Lys Thr Trp Leu Ala His Ala
1               5                   10                  15

Gly Leu Ala Leu Leu Leu Leu Thr Gly Leu Ala His Ala Gln Gly Tyr
            20                  25                  30

Pro Thr Lys Pro Ile Arg Ile Val Val Pro Tyr Pro Pro Gly Gly Phe

35                  40                  45

Asn Asp Thr Leu Ala Arg Ile Val Gly Ser Arg Leu Thr Ala Ala Trp
    50                  55                  60

Gly Gln Pro Val Val Val Asp Asn Lys Pro Gly Ala Gly Thr Ile Ile
65                  70                  75                  80

Gly Thr Ser Phe Val Ala Lys Ala Ala Pro Asp Gly Tyr Thr Leu Leu
                85                  90                  95

Val Val Gln Phe Pro Phe Gly Ala Asn Pro Trp Leu Tyr Lys Ser Leu
            100                 105                 110

Pro Tyr Asp Thr Leu Lys Asp Phe Thr Pro Val Ile Leu Ala Gly Glu
        115                 120                 125

Ser Pro Met Thr Leu Val Val Thr Asn Gly Ser Pro Ile Arg Ser Val
    130                 135                 140

Asp Asp Leu Val Lys Ser Ala Lys Gly Thr Pro Gly Lys Ile Asn Tyr
145                 150                 155                 160

Gly Ser Ser Gly Ser Gly Ser Ser Asn His Leu Ala Met Ala Leu Phe
                165                 170                 175

Glu Arg Ser Ala Gly Ile Thr Leu Ala Gln Val Pro Tyr Lys Gly Ser
            180                 185                 190

Thr Pro Met Leu Thr Asp Leu Ala Gly Gly Gln Val Glu Val Ala Phe
        195                 200                 205

Asp Ala Leu Pro His Val Leu Pro Phe Val Arg Ser Gly Lys Val Arg
    210                 215                 220

Ala Leu Ala Val Ala Asp Arg Ser Arg Phe Ala Ser Leu Ser Thr Val
225                 230                 235                 240

Pro Thr Met Ala Glu Ser Gly Leu Pro Gly Tyr Asp Ala Ser Ser Trp
                245                 250                 255

His Gly Ile Val Ala Pro Ala Gly Thr Pro Pro Glu Ile Val Arg Lys
            260                 265                 270

Leu Asn Ala Gln Ile Asn Asp Ala Leu Arg Thr Ala Asp Val Arg Asn
        275                 280                 285

Leu Phe His Glu Gln Gly Val Arg Pro Asp Gly Gly Ser Pro Ala Asp
    290                 295                 300

Phe Ser Ala Phe Ile Gly Arg Glu Leu Ala Lys Trp Lys Gln Val Val

305                 310                 315                 320

His Asp Ala Ala Ile Pro Leu Gln
                325

<210>  24
<211>  987
<212>  DNA
<213>  Extracytoplasmic solute receptor; probable orphan gene, Species
Cupriavidus basilensis HMF 14

<400>  24
   atgctgaaca ggcaggagac agacatgaaa acttggctcg cccatgccgg gctcgctttg        60
   ctgttgctca cggggcttgc ccatgcgcaa gggtatccca ccaagccgat ccgcatcgtg       120
   gtgccgtacc cgcccggcgg cttcaatgac acgctggccc gcatcgtcgg cagcaggctc       180
   accgcagcct ggggccagcc cgtggtggtg gacaacaagc ccggtgcggg caccatcatc       240
   ggcacctcgt tcgtggccaa ggccgcgccc gatggctaca cgctgctggt ggtgcagttc       300
   ccgttcggtg ccaatccctg gctctataag tcgctgccgt acgacaccct caaggacttc        360
   acgcccgtca tcctggcagg cgagtcgccg atgacccttg tggtgaccaa cggatcgccg       420
   atacgctccg tggacgatct tgtcaaatcc gctaagggca cgcccggcaa gatcaactac       480
   ggctcgtcgg gcagcggctc gtccaaccac cttgccatgg cgctgttcga gcgcagtgcc       540
   ggcatcacgc tcgcccaggt tccctacaag ggcagcacgc ccatgctgac cgatctggcc       600
   ggcggccagg tcgaagtggc cttcgacgcg ttgccccatg tgttgccttt cgtgaggtcc       660
   ggcaaggtgc gcgcacttgc cgtcgccgac cggagccgct ttgcgtcgct ttccacggtg       720
   cccaccatgg ccgagagcgg cctgcccggc tacgacgcgt catcctggca cggcatcgtc       780
   gcgccggccg gcacgccccc ggagatcgtg cgaaagctga acgcgcagat caacgacgcg       840
   ctgcgcacgg cggatgtgcg caatctcttc cacgagcagg gcgtgcgtcc cgacggcggc       900
   agccctgccg acttctctgc gtttatcggc agggaactgg cgaagtggaa gcaggtggtg       960
   catgacgcgg ccatcccctt gcaatga                                           987

<210>  25
<211>  579
<212>  PRT
<213>  HMF/furfural oxidoreductase, Small orf, Species Cupriavidus basilensis
HMF 14

<400>  25

Met Asp Thr Pro Arg Glu Arg Phe Asp Tyr Val Ile Val Gly Gly Gly
1               5                   10                  15

Ser Ala Gly Cys Val Leu Ala Asn Arg Leu Ser Gln Asp Pro Ala Ile
            20                  25                  30

Arg Val Ala Leu Ile Glu Ala Gly Val Asp Thr Pro Pro Asp Ala Val
        35                  40                  45

Pro Ala Glu Ile Leu Asp Ser Tyr Pro Met Pro Leu Phe Phe Gly Asp
    50                  55                  60

Arg Tyr Ile Trp Pro Ser Leu Gln Ala Arg Ala Val Ala Gly Gly Arg
65                  70                  75                  80

Ser Lys Val Tyr Glu Gln Gly Arg Val Met Gly Gly Gly Ser Ser Ile
                85                  90                  95

Asn Val Gln Ala Ala Asn Arg Gly Leu Pro Arg Asp Tyr Asp Glu Trp
            100                 105                 110

Ala Ala Ser Gly Ala Ser Gly Trp Ser Trp Gln Asp Val Leu Pro Tyr
        115                 120                 125

Phe Arg His Leu Glu Arg Asp Val Asp Tyr Gly Asn Ser Pro Leu His
    130                 135                 140

Gly Ser His Gly Pro Val Pro Ile Arg Arg Ile Leu Pro Gln Ala Trp
145                 150                 155                 160

Pro Pro Phe Cys Thr Glu Phe Ala His Ala Met Gly Arg Ser Gly Leu
                165                 170                 175

Ser Ala Leu Ala Asp Gln Asn Ala Glu Phe Gly Asp Gly Trp Phe Pro
            180                 185                 190

Ala Ala Phe Ser Asn Leu Asp Asp Lys Arg Val Ser Thr Ala Ile Ala
        195                 200                 205

Tyr Leu Asp Ala Asp Thr Arg Arg Arg Ala Asn Leu Arg Ile Tyr Ala
    210                 215                 220

Glu Thr Thr Val Arg Lys Leu Val Val Ser Gly Arg Glu Ala Arg Gly
225                 230                 235                 240

Val Ile Ala Met Arg Ala Asp Gly Ser Arg Leu Ala Leu Asp Ala Gly
                245                 250                 255

Glu Val Ile Val Ser Ala Gly Ala Leu Gln Ser Pro Ala Ile Leu Met
            260                 265                 270

Arg Ala Gly Ile Gly Asp Ala Gly Ala Leu Gln Ala Leu Gly Ile Glu
        275                 280                 285

Val Val Ala Asp Arg Pro Gly Val Gly Arg Asn Leu Gln Asp His Pro
    290                 295                 300

Ala Leu Thr Phe Cys Gln Phe Leu Ala Pro Gln Tyr Arg Met Pro Leu
305                 310                 315                 320

Ser Arg Arg Arg Ala Ser Met Thr Ala Ala Arg Phe Ser Ser Gly Val
                325                 330                 335

Pro Gly Gly Glu Ala Ser Asp Met Tyr Leu Ser Ser Ser Thr Arg Ala
            340                 345                 350

Gly Trp His Ala Leu Gly Asn Arg Leu Gly Leu Phe Phe Leu Trp Cys
        355                 360                 365

Asn Arg Pro Phe Ser Arg Gly Gln Val Ser Leu Ala Gly Ala Gln Pro
    370                 375                 380

Asp Val Pro Pro Met Val Glu Leu Asn Leu Leu Asp Asp Glu Arg Asp
385                 390                 395                 400

Leu Arg Arg Met Val Ala Gly Val Arg Lys Leu Val Gln Ile Val Gly
                405                 410                 415

Ala Ser Ala Leu His Gln His Pro Gly Asp Phe Phe Pro Ala Thr Phe
            420                 425                 430

Ser Pro Arg Val Lys Ala Leu Ser Arg Val Ser Arg Gly Asn Val Leu
        435                 440                 445

Leu Thr Glu Leu Leu Gly Ala Val Leu Asp Val Ser Gly Pro Leu Arg
    450                 455                 460

Arg Ser Leu Ile Ala Arg Phe Val Thr Gly Gly Ala Asn Leu Ala Ser
465                 470                 475                 480

Leu Leu Thr Asp Glu Ser Ala Leu Glu Gly Phe Val Arg Gln Ser Val
                485                 490                 495

Phe Gly Val Trp His Ala Ser Gly Thr Cys Arg Met Gly Ala His Ala
            500                 505                 510

Asp Arg Ser Ala Val Thr Asp Ala Ala Gly Arg Val His Asp Val Gly
        515                 520                 525

Arg Leu Arg Val Ile Asp Ala Ser Leu Met Pro Arg Leu Pro Thr Ala
    530                 535                 540

Asn Thr Asn Ile Pro Thr Ile Met Leu Ala Glu Lys Ile Ala Asp Thr
545                 550                 555                 560

Met Gln Ala Glu Arg Arg Ala Val Arg Pro Ala Ser Ser Glu Val Ala
                565                 570                 575

His Pro Ser

<210> 26
<211> 1740
<212> DNA
<213> HMF/furfural oxidoreductase, Small orf, Species Cupriavidus basilensis
HMF 14

<400> 26
atggatacgc cgagggagcg tttcgactac gtgattgttg gcggcgggtc cgccggttgc 60

gtactggcca atcgcctgtc gcaggacccg gccatccgcg tcgcgctgat cgaggcgggc 120

gtcgatacgc cgccggacgc tgtgccggcg gagatcctcg acagctatcc gatgcccttg 180

ttcttcggtg accggtatat ctggccatcg ctgcaagccc gcgccgtggc agggggcagg 240

tccaaggtct acgagcaagg gcgcgtcatg ggcggcggct ccagcatcaa cgtgcaggcg 300

gcaaaccgcg ggctgccgcg cgactacgat gagtgggccg cgtcgggcgc gtccggatgg 360

tcgtggcagg atgtgctgcc gtatttccgc caccttgagc gcgatgtgga ttacggcaac 420

agcccgctgc acggcagcca cggaccggtg ccgatccgcc gcatcctgcc gcaggcttgg 480

ccgccgttct gcacggagtt tgcgcacgcg atgggccgca gcggcttgtc cgcgctggcc 540

gaccagaacg cggagttcgg cgatggctgg tttccggccg ccttctcgaa cctggatgac 600

aagcgggttt cgaccgccat cgcctatctc gacgcggata cgcgccggcg ggccaatctg 660

cggatctatg ccgagacaac ggtgcgcaag ctcgtcgtat ccggccggga agcgcgtggg 720

gtgatcgcca tgcgggccga tgggtcgcgg ctggcgctgg acgccgggga ggtcatcgtg 780

tccgcgggcg ccttgcagtc gcccgccatc ctgatgcgcg cggggatcgg cgacgccggc 840

gcgctgcagg ccctcggcat cgaggtcgta gccgaccgac ccggcgttgg ccgcaatctc 900

caggatcatc ccgcgctgac gttctgccag ttcctcgcgc cccagtaccg catgccgctc 960

tcgcgccggc gcgctagcat gacggcggcg cggttctcat cgggggtgcc aggtggcgag 1020

gcgtcggaca tgtacctgtc cagttccaca cgggcaggct ggcatgcact cggtaatcgg 1080

ctcggcctct tcttcctgtg gtgcaatcgg ccattctcgc gcgggcaggt gagccttgcg 1140

ggagcccagc cggatgtgcc gcccatggtg gagctcaacc tgctcgacga cgagcgggat 1200

ctgcggcgca tggtggccgg cgtacgcaag ttggtgcaga tcgtgggtgc gtcggccttg 1260

catcagcatc ccggtgattt cttccccgct acgttttcgc cgcgcgtcaa ggcgctgagc 1320

cgcgtgagcc gcggcaatgt gttgctcacg gagttgctgg gggcagtgct tgatgtctcg 1380

gggccgctgc gcagaagcct gatcgcgcgc tttgtcacgg gcggcgcaaa cctggccagc 1440

ctgctgacgg atgagtccgc gctagagggc ttcgtgcgcc agagcgtctt cggggtctgg 1500

catgccagcg gcacttgccg gatgggcgcg catgcggacc ggagcgcggt gacggatgcg 1560

gcgggccgcg ttcacgatgt tggcaggctg cgcgttattg acgcctctct gatgccgcgg 1620

ctgccgacgg ccaataccaa catccccacc atcatgctcg cggaaaagat tgccgacacc 1680

atgcaagccg agcgccgcgc ggtccggccg gcatcgagcg aagttgccca tccgagttga 1740

<210> 27

<211>  231
<212>  PRT
<213>  Fatty acid hydroxylase, Species Cupriavidus basilensis HMF 14

<400>  27

Met Lys Tyr Asp Asp Glu Ile Arg Ala Arg Ser Tyr Arg Phe Arg Asp
1               5                   10                  15

Glu Tyr Val Ala Ala Thr Pro Ala Trp Tyr Arg Gly Glu Leu His Leu
            20                  25                  30

Ala Phe Thr Leu Leu Phe Thr Gly Gly Val Ile Ala Trp Cys Ala Met
        35                  40                  45

Lys Leu Gln Ala Pro Thr Leu Ala Gln Trp Leu Ala Ile Val Pro Ile
    50                  55                  60

Phe Leu Leu Gly Asn Trp Ala Glu Trp Ala Ala His Arg Tyr Ile Leu
65                  70                  75                  80

His Arg Pro Thr Arg Leu Phe Ser Ala Ile Tyr Lys Arg His Cys Ala
                85                  90                  95

Val His His Arg Phe Phe Thr His Leu Thr Leu Ser Thr Lys Ala Arg
            100                 105                 110

Ser Thr Gly Ala Pro Cys Cys Phe His Pro Leu Arg Gly Ser Leu Arg
        115                 120                 125

Ala Gly Ala Val Arg Arg Ala Val Ile Gly Val Ala Phe Ser Lys Asn
    130                 135                 140

Ala Gly Tyr Ile Ala Leu Met Thr Met Ala Ala Tyr Tyr Leu Met Tyr
145                 150                 155                 160

Glu Gly Leu His Thr Leu Ser His Ile Thr Asp Ser Pro Leu Leu Asp
                165                 170                 175

Arg Met Pro Phe Val Gly Thr Val Arg Arg Leu His Val Thr His His
            180                 185                 190

Asp Pro Glu Leu Met Ala Thr Gln Asn Phe Asn Leu Thr Phe Pro Ile
        195                 200                 205

Cys Asp Thr Leu Phe Gly Thr Arg Ser Asp Val Pro Arg Glu Val Arg
    210                 215                 220

Ser Pro Met Gln Gly Gln Gly
225                 230

<210>  28
<211>  696

<212>  DNA
<213>  Fatty acid hydroxylase, Species Cupriavidus basilensis HMF 14

<400>  28
atgaaatacg atgacgaaat ccgagcacgc tcttaccgct tccgcgacga atatgtcgcc      60

gccacccctg cgtggtatcg cggcgaattg catctggcct tcacgctgct attcaccggc     120

ggggtgattg cctggtgcgc gatgaaactg caagcaccga cgctggcgca gtggctcgcg     180

atcgtgccaa tcttcttgct cgggaactgg gccgagtggg ccgcgcaccg ctatatattg     240

catcgaccga cgcgcttgtt cagtgcgatc tataaacggc attgcgctgt gcatcatcgc     300

ttcttcacac atctgacgct gagtacaaag gccagaagca ctggcgcgcc ttgttgtttc     360

caccctttgc gcggtagcct tcgtgctggc gctgtgcgtc gcgctgtgat cggcgtggcg     420

ttctcgaaga acgcgggcta tatcgcgctg atgacgatgg cggcgtacta cctgatgtac     480

gagggcctgc atacgctgtc gcacatcacg gatagcccgc tgctggatcg gatgccgttc     540

gtggggaccg tgcggcgcct gcacgtcacg catcacgatc ctgagctgat ggccacccag     600

aacttcaacc tgaccttccc gatctgcgac acgctgttcg gcacgcgcag cgatgtgccg     660

cgcgaggtgc gaagcccgat gcaagggcag gggtag                               696

<210>  29
<211>  74
<212>  PRT
<213>  Truncated LysR-type transcriptional regulator, Species Cupriavidus basilensis HMF 14

<400>  29

Val Gln Phe Val Glu Ala Ser Arg Phe Ala Thr Val Leu Pro Arg Ile
1               5                   10                  15

Val Val Gln Arg Ala Val Asp Gln Gly Arg Leu Ser Met Tyr Pro Ile
            20                  25                  30

Leu Ala Pro Arg Ile Val Arg His Leu Val Cys Val Ser His Pro Gln
        35                  40                  45

Arg Pro Leu Ser Thr Ala Thr Val Ala Pro Ile Ala Ile Val Ala Ala
    50                  55                  60

Glu Ile Arg Arg Val Ser Gly Thr Ala Gly
65                  70

<210>  30
<211>  225
<212>  DNA
<213>  Truncated LysR-type transcriptional regulator, Species Cupriavidus basilensis HMF 14, DNA sequence

<400>  30
tcagccggcg gtgcctgaca ccctgcggat ctccgcggcc acgatcgcga tcggcgcaac      60

ggttgccgtg ctgagcggac gctgcggatg gctcacgcac accaggtggc gcacgatgcg     120

cggcgccagg atggggtaca tgctcagccg gccctggtcc accgcgcgtt gcaccacgat     180

gcgcggcagc accgtggcaa aacgcgaggc ctcgacgaac tggac                    225

<210>  31
<211>  441
<212>  PRT
<213>  major facilitator superfamily transporter, Species Cupriavidus basilensis
HMF 14

<400>  31

Met Glu Ala Val Ala Lys Lys Arg Thr Glu Thr Ile Ser Glu Ala Leu
1               5                   10                  15

Pro Ala Ala Thr Asn Arg Gln Val Phe Gly Ala Val Thr Ala Ser Cys
            20                  25                  30

Met Gly Trp Ala Leu Asp Leu Phe Asp Leu Phe Ile Leu Leu Phe Val
        35                  40                  45

Ala Pro Val Ile Gly Arg Leu Phe Phe Pro Ser Glu His Ala Met Leu
    50                  55                  60

Ser Leu Ala Ala Val Tyr Ala Ser Phe Ala Val Thr Leu Leu Met Arg
65                  70                  75                  80

Pro Leu Gly Ser Ala Ile Phe Gly Thr Tyr Ala Asp Arg His Gly Arg
                85                  90                  95

Lys Gly Ala Met Val Val Ala Val Thr Gly Val Gly Leu Ser Thr Ala
            100                 105                 110

Ala Phe Gly Leu Leu Pro Thr Val Gly Gln Val Gly Leu Leu Ala Pro
        115                 120                 125

Ala Leu Phe Ile Leu Leu Arg Leu Val Gln Gly Ile Phe Val Gly Gly
    130                 135                 140

Val Val Ala Ser Thr His Thr Ile Gly Thr Glu Ser Val Pro Pro Ser
145                 150                 155                 160

Trp Arg Gly Ala Val Ser Gly Leu Val Gly Gly Gly Gly Ala Gly Ile
                165                 170                 175

Gly Ala Leu Leu Ala Ser Ile Thr Tyr Met Ala Met Thr Ala Leu Phe
            180                 185                 190

Pro Gly Glu Ala Phe Asp Ala Trp Gly Trp Arg Cys Met Phe Phe Ser
        195                 200                 205

Gly Ile Ile Ser Ser Val Leu Gly Leu Phe Ile Phe Asn Ser Leu Glu
    210                 215                 220

Glu Ser Pro Leu Trp Lys Gln Leu Gln Ala Ala Lys Gly His Ala Ala
225                 230                 235                 240

Pro Val Glu Asn Pro Leu Arg Val Ile Phe Ser Arg Gln Tyr Arg Gly
                245                 250                 255

Val Leu Phe Val Asn Ile Leu Leu Thr Val Gly Gly Gly Ser Ala Tyr
            260                 265                 270

Tyr Leu Thr Ser Gly Tyr Leu Pro Thr Phe Leu Lys Val Val Val Lys
        275                 280                 285

Ala Pro Ala Gly Ala Ser Ala Ala Ile Leu Met Ala Ser Ser Val Gly
    290                 295                 300

Val Ile Val Ala Ser Ile Ile Ala Gly His Leu Ser Thr Leu Ile Gly
305                 310                 315                 320

Arg Lys Arg Ala Phe Leu Leu Ile Gly Ala Leu Asn Val Val Leu Leu
                325                 330                 335

Pro Leu Ile Tyr Gln Arg Met Pro Ala Ala Pro Asp Val Thr Thr Leu
            340                 345                 350

Gly Ile Tyr Ala Val Ala Leu Ala Met Leu Gly Ser Thr Gly Phe Ala
        355                 360                 365

Pro Ile Leu Ile Phe Leu Asn Glu Arg Val Ser His Gln His Pro Cys
    370                 375                 380

Tyr Gly Asn Trp Pro Val Met Glu Tyr Arg Leu Cys His Arg Arg His
385                 390                 395                 400

Asp Ala Thr Phe Ala Ser Leu Cys Ala Ala Pro Pro Arg Leu Pro Lys
                405                 410                 415

Cys Trp Gly Ser Arg Arg Gly Val Lys Ala Phe Thr Ala Gly Ala Ala
            420                 425                 430

Ile Val Trp Asn Ala Pro Leu Gly Glu
        435                 440

<210>  32
<211>  1326
<212>  DNA
<213>  Major facilitator superfamily transporter, Species Cupriavidus basilensis

<400>  32
atggaagccg tagcaaagaa gcgtacagag acgatcagcg aggcgctgcc agcggcgacc      60

aatcgccagg tgtttggtgc cgtgacggcg tcgtgcatgg gatgggcgct ggacctgttc     120

gacctgttca tcctgctgtt cgtggcgccc gtgatcggca ggctgttttt cccgtcggag     180

cacgccatgc tgtcgctggc ggcggtgtat gcgtcgtttg ccgtgacgct gctgatgcgg 240

ccgctcggct cggcgatctt cggcacttat gccgaccgcc acggccgcaa gggggcgatg 300

gtagttgccg tcactggcgt tggcttgtcc acggcggcgt tcggcctgct gcctacggtg 360

ggtcaggtgg ggctgcttgc gccagccttg tttatcctgc tgcggctggt gcagggcatc 420

ttcgtaggtg gcgtggtggc atccacccac accatcggta ccgaatcggt gcccccgtcc 480

tggcgcggcg ccgtctccgg gctggtcggt ggcggtggcg cgggcatcgg ggcactgctg 540

gcttccatta cctacatggc gatgaccgcg ctgtttccgg gggaagcgtt cgatgcctgg 600

ggttggcgct gcatgttctt ctccggcatc atcagctcgg tgctcggcct gttcatcttc 660

aactcgctgg aggagtctcc gctgtggaag cagttgcagg cggccaaggg gcacgccgcg 720

ccggttgaga acccgctgcg cgtgatcttc tcccgccagt accgtggcgt cctcttcgtc 780

aacatcctgc tcaccgtggg cggtggcagc gcctactacc tgacctccgg ctatctgccg 840

accttcctca aggtggtggt gaaggcaccg gctggcgcat ccgcagccat cctgatggcc 900

agcagtgttg gcgttatcgt ggcatcgata attgccggtc acctcagcac gctgattggt 960

cgcaagcgag ccttcctgct gatcggcgcc ttgaacgtgg tgctgctgcc gttgatctac 1020

caacggatgc ccgcggcgcc ggatgtcacc acgcttggca tttatgccgt ggcgctggcg 1080

atgctgggca gcaccggctt cgccccgatc ctcattttcc tgaacgaacg ggtttcccac 1140

cagcatccgt gctacgggaa ctggcctgtc atggaatatc ggctttgcca tcggcggcat 1200

gatgcgacgt ttgcgtcgct gtgcgcagca cccccgcgac tgccaaagtg ctggggatct 1260

cgtcgcggtg tcaaagcatt tactgccggt gcggcgatcg tctggaacgc gccgctgggg 1320

gagtga 1326

<210> 33
<211> 314
<212> PRT
<213> LysR-type regulator, Species Cupriavidus basilensis HMF 14

<400> 33

Met Asp Ile Lys Gln Ile Gln Tyr Phe Ile Ala Leu Phe Glu Asp Gly
1               5                   10                  15

Ser Val Thr Arg Ala Ala Lys Arg Leu Asn Ile Val Gln Pro Ala Leu
            20                  25                  30

Ser Met Gln Ile Ala Lys Leu Glu Glu Glu Phe Gly Gln Lys Leu Phe
        35                  40                  45

Asp Arg Ile Pro His Gly Met Val Pro Thr Ala Ala Gly Arg Met Met
    50                  55                  60

Tyr Arg Leu Cys Leu Pro Ile Thr Arg Asp Leu Ala Asn Ala Arg Gln
65                  70                  75                  80

Gln Leu Met Gln Arg Glu Glu Gln Val Ala Gly Asn Ile Ser Ile Gly
                85                  90                  95

Met Val Ala Ser Glu Thr Glu Ser Val Leu Val Gly Ser Leu Val Arg
            100                 105                 110

Phe Asn Ala Arg Tyr Pro Asn Val Glu Val Ser Val Ala Asp Gly Phe
        115                 120                 125

Ser Ala Thr Leu Ile Asp Leu Val Ser Ala Gly Gln Leu Asp Ala Ala
    130                 135                 140

Val Val His Lys Pro Arg Gly Lys Leu Ser Leu His Val Gln Ser Leu
145                 150                 155                 160

His Asp Glu Glu Met Val Leu Val Thr Ser Ala Glu His Gly Pro Gly
                165                 170                 175

Thr Ala Gly Gly Gly Gly Thr His Gln Ala Ala Arg Ala Gly Thr Gly
            180                 185                 190

Ala Ala His Gln Ala Pro Arg Leu Ala Arg Arg Ala Gly Arg Gly His
        195                 200                 205

Ala Thr Gly Arg Arg Asp Pro Ala Ala Glu Val Arg Asp Arg His Pro
    210                 215                 220

Arg His His Arg Pro Val Arg Arg Gly His Ala Phe Cys His Gly Ala
225                 230                 235                 240

Ala Ala His Arg Gly Ala Thr Arg Gly Gly Arg Gly Pro Ala Ala His
                245                 250                 255

Val Pro His Pro Gly Ala Ala His Arg Ala Pro Pro Gly Val Arg Glu
            260                 265                 270

Pro Ser Ala Ala Pro Ala Gln His Gly Ser Arg Cys Ala Asp Arg Asp
        275                 280                 285

Arg Gly Arg Gly Asp Pro Gln Gly Val Arg His Arg Arg Leu Ile Arg
    290                 295                 300

Val Pro Gly Ile Arg Lys Gly Thr Ile Arg
305                 310

<210>  34
<211>  945
<212>  DNA
<213>  LysR-type regulator, Species Cupriavidus basilensis HMF 14

<400>  34
atggacatca aacagatcca gtacttcatt gcgctgttcg aagatggctc cgtcacgcgc        60

gcggccaagc ggctgaatat cgtgcagccg gcgctcagca tgcagatcgc caagctggaa 120

gaggagttcg gacagaagct gttcgatcgc atcccgcacg ggatggtgcc gacagcggcc 180

ggccgcatga tgtaccggct gtgcctgccg atcacgcggg acctcgccaa cgcccggcag 240

caactgatgc aacgcgagga gcaggtggcc ggcaacatct ccatcggcat ggtggcgtcg 300

gaaacggaaa gcgtgctggt tggctcgctg gtgcgcttca acgcgcgcta cccgaatgtg 360

gaggtatcgg tggcggacgg attcagcgcg acgctgatcg acctggtctc cgccggccag 420

ctcgacgcgg ccgtggtgca taagccccgt ggcaagctct cgctccatgt gcaatccctg 480

catgacgagg aaatggtgct ggtcaccagc gccgagcatg ggcccggaac tgccggcggc 540

ggtggaactc accaagctgc ccgggctgga actggtgctg cccaccaagc gccacggctt 600

gcgcggcgtg ctggacgcgg ccacgcaact ggaagacgtg accctgcagc cgaagttcga 660

gatcgacatc ctcggcacca tcgtccagtt cgtcgaggcc acgcgttttg ccacggtgct 720

gccgcgcatc gtggtgcaac gcgcggtgga cgagggccgg ctgcgcatgt accccatcct 780

ggcgccgcgc atcgtgcgcc acctggtgtg cgtgagccat ccgcagcgcc cgctcagcac 840

ggcagccgat gcgctgatcg cgatcgtggc cgaggagatc cgcagggtgt caggcaccgc 900

cggctgatcc gggtgcccgg catcaggaag ggcacgatac ggtag 945

<210> 35
<211> 1015
<212> PRT
<213> Furoyl-CoA dehydrogenase large subunit, Species Cupriavidus basilensis
HMF 14

<400> 35

Met Ser Asp Lys Arg Ser Leu Pro Ala Ala Ala Ala Ala Ile His Leu
1               5                   10                  15

Glu Ser Ala Ala Ala Gly Ala Ala Ser Arg Gln Arg His Val Gly Arg
            20                  25                  30

Ser Met Glu Arg Leu Glu Asp Ala Ala Ile Leu Thr Gly Arg Gly Arg
        35                  40                  45

Tyr Gly Asp Asp Leu Gly Val Lys Pro Gly Thr Leu His Ala Ala Ile
    50                  55                  60

Val Arg Ser Pro His Ala His Ala Glu Leu Gly Thr Ile Asp Ala Thr
65                  70                  75                  80

Ala Ala Leu Ala Ala Pro Gly Val His Ala Val Leu Thr Gly Ala Asp
                85                  90                  95

Leu Ala Ala Trp Ser Arg Pro Phe Val Val Ala Val Lys Ser Pro Met
            100                 105                 110

Glu Gln Trp Ala Leu Ala Met Asp Arg Val Arg Tyr Val Gly Glu Pro
        115                 120                 125

Val Ala Val Val Met Ala Glu Ser Arg Ala Leu Ala Glu Asp Ala Leu
    130                 135                 140

Asp Leu Val Arg Val Asn Tyr Arg Val Leu Pro Pro Val Val Ser Ile
145                 150                 155                 160

Glu Ala Ala Leu Ala Asp Asp Ala Pro Ile Leu His Pro Gly Val Gly
                165                 170                 175

Ala Asn Val Val Ser Asp Arg His Phe Arg Tyr Gly Glu Pro Glu Ala
            180                 185                 190

Ala Phe Ala Ala Ala Pro His Arg Val Thr Leu Thr Ala His Tyr Pro
        195                 200                 205

Arg Asn Thr Cys Thr Pro Ile Glu Cys Gly Val Val Ile Ala Glu Phe
    210                 215                 220

Leu Pro Gly Asp Glu Gly Tyr Asp Val Thr Ser Asn Phe Met Gly Pro
225                 230                 235                 240

Phe Ser Leu His Ala Val Met Ala Met Ala Leu Lys Val Pro Ala Asn
                245                 250                 255

Arg Leu Arg His Lys Ala Pro Arg Asp Ser Gly Gly Ser Phe Gly Val
            260                 265                 270

Lys Gln Ala Val Phe Pro Tyr Ala Val Leu Met Cys Leu Ala Ser Arg
        275                 280                 285

Lys Ala Gly Ala Pro Val Lys Trp Val Glu Asp Arg Leu Glu His Leu
    290                 295                 300

Ser Ala Ala Thr Ser Ala Thr Ala Arg Leu Ser Thr Leu Glu Ala Ala
305                 310                 315                 320

Val Glu Ser Asp Gly Arg Ile Lys Ala Leu Thr Tyr Asp Gln Ile Glu
                325                 330                 335

Asp Cys Gly Gly Tyr Leu Arg Ala Pro Glu Pro Ala Thr Phe Tyr Arg
            340                 345                 350

Met His Gly Cys Leu Thr Gly Ala Tyr Asp Ile Pro Asn Leu Leu Val
        355                 360                 365

Arg Asn Arg Val Val Met Thr Asn Lys Thr Pro Thr Gly Leu Val Arg
    370                 375                 380

Gly Phe Gly Gly Pro Gln Val Tyr Phe Ala Leu Glu Arg Leu Val His
385                 390                 395                 400

Arg Ile Ala Thr Gln Leu Gly Leu Asp Pro Leu Asp Val Tyr Arg Arg
                405                 410                 415

Asn Phe Val Ala Ala Asp Ala Phe Pro Tyr Arg Ala Ala Ala Gly Ala
            420                 425                 430

Leu Leu Asp Ser Gly Asn Tyr Gln Leu Ala Leu Ala Arg Ala Leu Glu
        435                 440                 445

Glu Gly Gly Tyr Tyr Glu Leu Thr Cys Arg Arg Asp Val Ala Arg Ala
    450                 455                 460

Glu Gly Arg Leu Tyr Gly Ile Gly Phe Ala Ala Ile Val Glu Pro Ser
465                 470                 475                 480

Val Ser Asn Met Gly Tyr Ile Thr Thr Ala Met Pro Ala Glu Ala Arg
                485                 490                 495

Lys Lys Ala Gly Pro Lys Asn Gly Ala Ile Ala Ser Ala Thr Val Ser
            500                 505                 510

Val Asp Leu Leu Gly Gly Val Val Val Thr Ile Ala Ser Thr Pro Ala
        515                 520                 525

Gly Gln Gly His Met Thr Val Cys Ala Gln Val Val Ala Asp Val Leu
    530                 535                 540

Gly Val Asn Pro Ala Asp Val Val Val Asn Val Glu Phe Asp Thr His
545                 550                 555                 560

Lys Asp Ala Trp Ser Val Ala Ala Gly Asn Tyr Ser Ser Arg Phe Ala
                565                 570                 575

Gly Ala Val Ala Gly Thr Val His Leu Ala Ala Glu Arg Val Arg Asp
            580                 585                 590

Lys Leu Ala Arg Ile Val Ala Pro Gln Phe Gly Cys Thr Pro Ala Glu
        595                 600                 605

Val Val Phe Glu Asp Gly Arg Ile Ala Arg Lys Gly Ala Pro Glu Ser
    610                 615                 620

Gly Leu Pro Phe Thr Arg Val Ala Ser Asn Ala Pro His Trp Ser Pro
625                 630                 635                 640

Gln Gln Leu Pro Ala Gly Glu Glu Pro Gly Leu Arg Glu Thr Val Phe
                645                 650                 655

Trp Ser Pro Pro Asn Leu Glu Ala Pro Asp Glu Asn Asp Arg Ile Asn
            660                 665                 670

Thr Ser Ala Ala Tyr Gly Phe Ala Phe Asp Met Cys Gly Val Glu Ile
        675                 680                 685

Asp Arg Ala Thr Gly Arg Val Arg Ile Asp Arg Tyr Val Thr Ala His
    690                 695                 700

Asp Ala Gly Thr Leu Leu Asn Pro Ala Leu Ala Asp Gly Gln Ile Arg
705                 710                 715                 720

Gly Ala Phe Ala Gln Gly Leu Gly Ala Ala Leu Met Glu Glu Phe Arg
                725                 730                 735

Tyr Gly Ala Asp Gly Ser Phe Gln Ser Gly Thr Leu Ala Asp Tyr Leu
            740                 745                 750

Met Pro Thr Thr Cys Glu Val Pro Asp Pro Val Ile Val His Leu Glu
        755                 760                 765

Thr Pro Ser Pro Phe Thr Pro Leu Gly Ala Lys Gly Leu Gly Glu Gly
    770                 775                 780

Asn Asn Met Ser Thr Pro Pro Cys Ile Ala Asn Ala Val Ala Asp Ala
785                 790                 795                 800

Leu Gly Glu Gln Asp Ile Arg Leu Pro Leu Thr Pro Ala Lys Val Met
                805                 810                 815

Ala Met Val Gly Phe Asp Asp Pro Pro Pro Ser Arg Pro Glu Leu Leu
            820                 825                 830

Glu Ala Met Arg Glu Ala Ala Val Pro Ala Ala Arg Lys Gly Ser Ala
        835                 840                 845

Lys Ala Leu Thr Ala Arg Gly Ser Val Asp Leu Asp Ala Thr Pro Glu
    850                 855                 860

Ala Ile Phe Ala Val Leu Met Asp Pro Gln Ala Leu Ala Arg Val Val
865                 870                 875                 880

Pro Gly Cys His Ala Leu Glu Arg Thr Ala Glu Asn His Tyr Arg Ala
                885                 890                 895

Asp Val Thr Val Gly Val Gly Met Ile Lys Ala Arg Phe Glu Ala Glu
            900                 905                 910

Ile Ala Leu Ser Asp Leu Asp Pro Pro Arg Arg Leu Arg Leu Ala Gly
        915                 920                 925

Ala Gly Met Ser Ser Leu Gly Ser Ala Arg Gly Ala Gly Leu Val Glu
    930                 935                 940

Leu Val Pro His Gly Ser Gly Thr Arg Leu Ser Tyr Asp Tyr Glu Ala
945                 950                 955                 960

Glu Val Ser Gly Lys Val Ala Ala Val Gly Gly Arg Met Leu Glu Gly
                965                 970                 975

Ala Ala Lys Val Val Leu Arg Gln Leu Phe Glu Ser Leu Gly Arg Gln
            980                 985                 990

Ala Ala Gly Lys Pro Val Arg Pro Gln Gly Trp Leu Ala Arg Leu Leu
        995                 1000                1005

Ala Arg Leu Gly Val Arg Arg
    1010                1015

<210> 36
<211> 3048
<212> DNA
<213> Furoyl-CoA dehydrogenase large subunit, Species Cupriavidus basilensis
HMF 14

<400> 36
atgagcgaca agcgcagcct gcccgccgca gcggcggcca tccaccttga atccgcagcc 60
gccggcgcgg cctcgcgcca gcgtcacgtc ggccgctcga tggagcggct cgaggacgcc 120
gcgatcctca ccggccgtgg ccgctatggc gacgacctgg gcgtgaagcc gggcaccctg 180
cacgcggcca tcgtgcgttc cccgcatgca catgccgaac ttggcaccat cgatgccacc 240
gccgcgcttg ccgcgccggg cgtgcatgcc gtgctgaccg gcgccgacct ggcagcctgg 300
tcgcgtccct tcgtggtcgc cgtgaagtcg ccaatggagc aatgggcact ggccatggac 360
cgcgtgcgct atgtgggcga gccggtggcc gtggtaatgg ccgaaagccg tgccctggcc 420
gaagacgcgc tcgacctggt gcgggtgaat taccgcgtgt tgccgccggt ggtatcgatc 480
gaggctgcgc tggccgacga tgcgcccatc ctgcatcccg gcgtaggcgc caatgtggtg 540
agcgaccgcc acttccgcta cggcgagcca gaggccgcct ttgccgccgc gccgcaccgg 600
gtcacgctga ccgcgcacta tccgcgcaac acctgcacgc cgatcgaatg cggcgtggtg 660
attgccgagt tcctgcccgg tgacgaaggc tacgacgtca cctccaattt catggggccg 720
ttctcgctgc atgcggtgat ggcgatggcg ctcaaggtgc cggccaaccg gctgcgccac 780
aaggccccgc gcgattccgg cggcagcttt ggcgtgaagc aggcggtgtt tccttacgcg 840
gtgctgatgt gcctggcgtc ccgcaaggcc ggcgcgcctg taaagtgggt ggaggaccga 900
ctcgaacatc tcagcgcggc cacctccgcc accgcgcggc tgtccacgct ggaagccgcg 960
gtggaatcag acggccgcat caaagcgctg acctacgacc agatcgaaga ctgcggcggc 1020
tatctgcgcg cgccggagcc cgccactttc taccgcatgc acggctgcct gactggcgcc 1080
tacgacatcc ccaacctgct ggtgcgcaac cgcgtggtga tgaccaacaa gacgcccacc 1140

ggcctggtgc ggggctttgg cggcccgcag gtgtattttg cgctggagcg gctggtgcat 1200
cgcatcgcga cacaactcgg gctcgatcca ctcgatgtgt atcgccgcaa ctttgtcgcc 1260
gccgatgcct ttccctatcg cgccgcggcg ggcgcgttgc tggactccgg caactaccag 1320
ctggcgctgg cgcgcgcgct ggaagaaggc ggctactacg agctgacgtg ccgccgcgat 1380
gtcgcacgcg ccgagggccg gctctatggc atcggctttg ccgccatcgt cgagccgtcg 1440
gtgtcgaaca tgggctatat caccaccgcc atgcccgccg aggcgcgcaa gaaggcgggg 1500
ccgaagaacg gcgccatcgc cagcgccacg gtcagcgtcg acctgctcgg cggcgtggtg 1560
gtcaccattg cctcgacgcc ggccggacag ggccatatga cagtgtgcgc gcaggtggtg 1620
gccgatgtgc tcggcgtgaa tccggccgac gtggtcgtaa acgtcgagtt cgatacgcac 1680
aaggatgcat ggtcggtcgc cgccggcaac tactccagcc gtttcgccgg cgccgtggcc 1740
ggcaccgtgc acctggccgc cgagcgggtg cgcgacaagc tggcgcgcat cgtggcgccg 1800
cagttcggct gcacgcccgc cgaggtggtg ttcgaagacg gccgcattgc ccgcaaaggc 1860
gcgcccgaat ccggcctgcc cttcacgcgc gtggccagca atgcgccgca ctggtcaccg 1920
cagcagttgc cggcgggaga agagcctggc ctgcgcgaga cggtgttctg gtcgccgccc 1980
aacctggaag cgccggatga gaacgaccgc atcaacacct cggccgccta cggctttgcc 2040
ttcgatatgt gcggcgtgga gatcgaccgc gccacggggc gcgtgcgcat cgaccgctac 2100
gtgaccgcgc acgacgccgg cacgctgctc aacccggcgc tggccgatgg ccagatccgc 2160
ggcgccttcg cccaaggcct gggcgcggcg ctgatggagg agttccgcta tggcgccgac 2220
ggcagcttcc agtccggcac gctggccgac tacctgatgc cgaccacctg cgaggtgccg 2280
gacccggtga tcgtgcacct ggagacgccc agccccttca cgccgctcgg cgccaagggc 2340
ctgggcgagg gcaacaacat gagcacgccg ccctgcatcg ccaacgcggt agccgacgcg 2400
ctcggcgagc aggatatccg cctgccgctg accccggcca aggtgatggc catggtgggc 2460
ttcgacgatc cgccgccgtc gcgccccgag ctgctcgaag cgatgcgcga ggccgccgtg 2520
cccgcggccc gcaagggcag cgcaaaggcg ctcaccgcgc gcggctcggt ggatctggat 2580
gccacgcccg aagccatctt cgccgtgctg atggacccgc aggccctggc cagggtagtg 2640
ccaggctgcc acgcgctgga gcgcaccgcc gagaaccact accgcgccga tgtgacggtg 2700
ggggttggca tgatcaaggc ccgcttcgag gcggagatcg cactgtcaga cctcgatccc 2760
ccgcgccggc tgcggctggc aggcgcaggc atgtcctcgc tgggcagtgc acgcggcgcc 2820
gggctggtgg agttggtccc gcatggcagc ggcacgcgcc tgagttacga ctacgaggcc 2880
gaggtatccg gcaaggtcgc tgccgtgggc ggccgcatgc tggagggcgc cgccaaggtg 2940
gtgctgcgcc agttgtttga atcgctcggc cgccaggccg ccggcaagcc ggtacggccg 3000
caaggctggc ttgcccgact gctggcccgc ttgggagtac gccgatga 3048

<210> 37
<211> 271

<212>  PRT
<213>  Furoyl-CoA dehydrogenase FAD binding subunit, , Species Cupriavidus
basilensis HMF 14

<400>  37

Met Lys Pro Ser Ala Phe Asp Tyr Leu Arg Ala Glu Thr Thr Gln His
1               5                   10                  15

Ala Leu Glu Ala Leu Ala Arg Gly Gly Glu Gly Ala Arg Val Leu Ala
            20                  25                  30

Gly Gly Gln Ser Leu Met Ala Val Leu Asn Met Arg Leu Ala Gln Pro
        35                  40                  45

Gln Leu Leu Ile Asp Ile Ser Arg Thr Ala Glu Leu Asp Thr Val Arg
    50                  55                  60

Val Glu Asp Ala His Leu Val Val Gly Ala Ala Ala Thr Gln Gly Ser
65                  70                  75                  80

Val Glu Trp Arg Arg Ser Leu Ala Asp Glu Val Pro Leu Leu Ala Met
                85                  90                  95

Ala Phe Pro His Ile Ser His Phe Gln Ile Arg Asn Arg Gly Thr Val
            100                 105                 110

Cys Gly Ser Val Ala His Ala Asp Pro Ser Ala Glu Leu Pro Leu Val
        115                 120                 125

Leu Thr Ala Leu Gly Gly Glu Val Val Leu Arg Ser Ala Arg Arg Arg
    130                 135                 140

Arg Val Leu Pro Ala Ala Ser Phe Phe Gln Gly Met Leu Met Thr Ala
145                 150                 155                 160

Arg Glu Pro Asp Glu Leu Val Glu Ala Val Arg Phe Pro Leu Arg Arg
                165                 170                 175

Pro Gly Ala Arg Tyr Gly Phe Ala Glu Phe Ser Ala Arg His Gly Asp
            180                 185                 190

Phe Ala Leu Val Ala Cys Ala Ala Thr Val Thr Asp Asp Ala Ile Ala
        195                 200                 205

Leu Ala Val Gly Gly Val Ala Asp Arg Pro Val Leu Glu Thr Trp Pro
    210                 215                 220

Arg Leu Gln Gly Lys Asp Leu Glu Gln Ala Ile Asn Asp Phe Ser Trp
225                 230                 235                 240

Lys Leu Gly Ala Gln Asp Asp Ala His Ile Ser Ala Gln Tyr Arg Arg
                245                 250                 255

His Leu Val Arg Gln Leu Ser Met Arg Val Ile Glu Glu Ala Lys
            260                 265                 270

<210>  38
<211>  816
<212>  DNA
<213>  Furoyl-CoA dehydrogenase FAD binding subunit, Species Cupriavidus
basilensis HMF 14

<400>  38
atgaaaccgt ctgctttcga ttacctgcgc gccgagacca cgcagcacgc gctcgaggcg      60

ctggcccgtg gcggcgaggg cgcgcgcgtg ctggccggcg gccagtcgct gatggcggtg     120

ctcaatatgc gcctggcgca gccgcaactg ctgatcgata tctcgcgcac cgccgagctg     180

gacacggtgc gggtggaaga cgctcacctc gtggtgggtg ccgcggccac gcagggcagc     240

gtcgaatggc gccgctcgct ggccgacgag gtgccgctgc tggccatggc ctttccgcat     300

atctcgcatt tccagatccg gaatcgcggc accgtgtgcg gctcggtcgc ccatgccgac     360

ccgagcgcgg aattgcccct ggtgctgacc gcgctgggcg gcgaggtggt gctgcgttca     420

gcccgccgcc gccgcgtgct gcctgcggcc agcttcttcc agggcatgtt gatgacggcg     480

cgcgagcccg acgagctggt ggaggccgtg cgctttccgc tgcggcgccc cggggcgcgc     540

tacggctttg ccgaattctc cgcgcgccac ggcgattttg cgctggtggc ctgcgcggcc     600

accgtgaccg atgacgccat cgcgctggcg gttggcggcg tggcggacag gccggtgctg     660

gaaacctggc cgcgcctgca gggcaaggac ctggagcagg ccatcaacga tttcagttgg     720

aaactgggcg cgcaggacga cgcccatatc agcgcgcagt accgccggca cctggtgcgg     780

caactgagca tgcgtgtgat cgaggaggca aaatga                               816

<210>  39
<211>  192
<212>  PRT
<213>  Furoyl-CoA dehydrogenase 2Fe-2S iron sulfer subunit, Species Cupriavidus
basilensis HMF 14

<400>  39

Met Ser Lys Thr Asn Lys Gly Ala Val Ser Gly Lys Pro Ala Glu Val
1               5                   10                  15

Met Gln Arg Gln Glu Gln Arg Arg Ile Thr Leu Thr Leu Asn Gly Arg
            20                  25                  30

Glu Arg Ser Gly His Cys Glu Pro Arg Glu Leu Leu Ser Asp Phe Leu
        35                  40                  45

Arg His Glu Leu Gly Ala Thr Gly Thr His Val Gly Cys Glu His Gly
    50                  55                  60

Val Cys Gly Ala Cys Thr Val Arg Val Asp Gly Val Ala Ala Arg Ser
65                  70                  75                  80

Cys Leu Met Leu Ala Val Gln Ala Glu His Arg Ala Ile Asp Thr Val
                85                  90                  95

Glu Gly Leu Ala Pro Ala Glu Gly Leu Gly Asp Leu Gln Glu Ala Phe
            100                 105                 110

Arg Arg His His Ala Leu Gln Cys Gly Phe Cys Thr Ala Gly Ile Leu
        115                 120                 125

Met Ser Cys Ala Asp Tyr Leu Glu Arg Val Pro Glu Pro Ser Glu Ala
    130                 135                 140

Gln Val Arg Asp Met Leu Ser Gly His Leu Cys Arg Cys Thr Gly Tyr
145                 150                 155                 160

Thr Pro Ile Val Ala Ala Val Leu Asp Val Ala Ala Ile Arg Ala Arg
                165                 170                 175

Ala Arg His Ala Ala Ala Gly Val Asp Thr Gln Glu Ala Arg Asn Ala
            180                 185                 190

<210> 40
<211> 579
<212> DNA
<213> Furoyl-CoA dehydrogenase 2Fe-2S iron sulfer subunit, Species Cupriavidus
basilensis HMF 14

<400> 40
atgagcaaga ccaacaaggg cgccgtgtcc ggcaagcccg ccgaggtaat gcagcgccag 60

gagcaacgcc gcatcaccct gacgctgaac ggccgcgagc gcagcggcca ttgcgagccg 120

cgcgagctgc tgtcggactt cctgcgccac gagctcggcg ccaccggcac ccatgtgggt 180

tgcgagcacg gcgtctgcgg cgcatgcacg gtacgcgttg acggcgttgc cgcgcgctcg 240

tgcctgatgc tggcggtgca ggccgagcac cgggccatcg ataccgtcga agggctggcg 300

ccggccgagg gactgggcga cctgcaagaa gccttccgcc gccaccacgc gctgcagtgc 360

ggcttctgca ccgcaggcat cctgatgtcg tgcgcggact acctggagcg cgtgccggaa 420

cccagcgagg cgcaggtgcg cgacatgctg tccggccacc tgtgccgctg tacgggctac 480

acccccattg tggccgccgt gctcgacgta gccgcgatcc gtgcacgggc tcgtcatgct 540

gctgccggcg tagataccca ggaggcccgc aatgcttga 579

<210> 41
<211> 532
<212> PRT
<213> Furoyl-CoA syntethase, Species Cupriavidus basilensis HMF 14

<400> 41

Met Leu Asp Leu Gly Arg Thr Phe Leu Gln Ser Val Glu Arg Ser Pro
1               5                   10                  15

His Thr Pro Ala Ile Val Asp Gly Asp Leu Met Leu Thr Tyr Ala Gln
            20                  25                  30

Trp Tyr Glu Arg Ile Arg Cys Val Ala Ser Gly Leu Arg Glu Ile Gly
        35                  40                  45

Leu Ala Pro Gly Asp Arg Leu Leu Ala Val Leu Gln Asn Arg Trp Glu
    50                  55                  60

Met Ala Thr Leu Tyr Trp Ala Cys Gln Phe Ala Gly Ile Val Met Val
65                  70                  75                  80

Pro Leu Asn Trp Arg Ala Lys Pro Glu Glu Leu Asp Tyr Cys Val Gln
                85                  90                  95

Asp Ala Gly Val Lys Ala Leu Val Phe Glu Pro Val Ser Ala Asp Ala
            100                 105                 110

Val Leu Gly Ser Pro Ala Ala Gln Ala Val Pro Cys Ile Ala Leu Asp
        115                 120                 125

Cys Ala Ala Gly Gly Ser Met Ser Phe Ala Ser Leu Leu Asp Ser Val
    130                 135                 140

Ala Leu His Gly Gly Pro Val Ala Glu Ala Gly Asp Val Ser Leu Met
145                 150                 155                 160

Leu Tyr Thr Ser Gly Thr Thr Gly Lys Pro Lys Gly Val Pro Arg Arg
                165                 170                 175

His Gln His Glu Arg Ala Ala Ala Leu Ala His Val Ala Gln Asn Leu
            180                 185                 190

Tyr Arg His Gly Glu Arg Thr Leu Gly Val Met Pro Leu Tyr His Thr
        195                 200                 205

Met Gly Val Arg Ser Leu Leu Ala Met Ala Leu Val Asp Gly Leu Phe
    210                 215                 220

Val Cys Val Arg Arg Trp Asn Ala Gly Gln Ala Leu Glu Glu Ile Asn
225                 230                 235                 240

Thr His Arg Ile Ser Cys Leu Tyr Leu Val Pro Thr Leu Tyr His Asp
                245                 250                 255

Leu Leu Ala Asp Pro Gly Phe Asp Ala Cys Ile Val Arg Ser Val Thr
            260                 265                 270

Lys Leu Gly Phe Ala Gly Ala Ser Met Asn Asp Gly Leu Leu Arg Arg
        275                 280                 285

Leu Ala Leu Ala Phe Glu Pro Glu Leu Phe Val Asn His Tyr Gly Ser
    290                 295                 300

Ser Glu Val Tyr Thr Phe Ser Val Asp Gln Arg Ala Thr Arg Lys Pro
305                 310                 315                 320

Gly Ser Ala Gly Arg Ala Gly Ile Asn Thr Arg Leu Arg Val Val Arg
                325                 330                 335

Leu Asp Ala Arg Ser Pro Asp Asp Leu Ala Ala Thr Gly Glu Glu Gly
            340                 345                 350

Gln Ile Ile Ala Asp Leu Arg Gly Asp Glu Ala Phe Glu Gly Tyr Trp
        355                 360                 365

Asn Arg Asp Asp Ala Asn Ala Lys Ser Leu Arg Asp Gly Trp Tyr Phe
    370                 375                 380

Thr Gly Asp Thr Gly Tyr Phe Asp Ala Glu Gly Asp Leu Phe Val Ser
385                 390                 395                 400

Gly Arg Val Asp Asp Met Ile Ile Ser Gly Gly Glu Asn Ile Ser Pro
                405                 410                 415

Val Asp Ile Glu Ser Val Leu Ser Leu His Pro Ala Val Asp Glu Val
            420                 425                 430

Ala Val Ala Gly Val Pro Asp Pro Arg Trp Gly Gln Lys Val Val Ala
        435                 440                 445

Phe Val Lys Pro Arg Gly Asn Ile Asp Ala Gln Ala Leu Asp Thr Tyr
    450                 455                 460

Cys Arg Gly Ser Asp Leu Val Asn Phe Lys Arg Pro Arg Asp Tyr Val
465                 470                 475                 480

Phe Val Glu Glu Ile Pro Lys Ser Pro Val Gly Lys Ile Leu Arg Arg
                485                 490                 495

Lys Leu Ser Ala Gly Glu Tyr Ala Leu Ala Pro His Ser Met Ser Pro
            500                 505                 510

Asp Pro Asn Thr Asn Pro Asn Gln Ala Ala Asp Ala Ala Pro Val Asp
        515                 520                 525

Thr Ile Lys Glu
    530

<210>  42
<211>  1599

<212>  DNA
<213>  Furoyl-CoA syntethase, Species Cupriavidus basilensis HMF 14

<400>  42
atgcttgatc taggccgcac cttcctgcaa agcgtggagc gcagcccgca cacgcccgcc      60

attgtcgacg gcgacctgat gctcacctat gcgcaatggt acgagcgcat ccggtgcgtg     120

gcgtccggcc tgcgcgagat cggcctcgcg ccgggcgatc gcctgctggc cgtgttgcaa     180

aaccgctggg aaatggccac gctgtactgg gcctgccagt tcgccggcat cgtgatggtg     240

ccgctgaact ggcgcgccaa gccggaggag ctcgattact gtgtgcagga tgccggcgtc     300

aaggcgctgg tgttcgagcc ggtcagcgcc gatgcggtgc tgggcagccc cgcggcgcag     360

gccgtgccct gcattgcgct ggactgcgcg gctggcggct cgatgtcctt cgcttcgctg     420

ctggacagcg tcgcgctgca tggcggcccg gtggcggaag cgggcgatgt ctcgctgatg     480

ctctacacct cgggcaccac cggcaagccc aagggcgtgc cgcgccgcca ccagcacgag     540

cgcgccgcgg cgctggcgca cgtggcgcag aacctgtatc gccatggtga gcgcaccctt     600

ggcgtgatgc cgctctacca caccatgggc gtgcggtccc tgctggccat ggcgctggtg     660

gacggcctgt tcgtctgcgt gcggcgctgg aacgccgggc aggcgctcga ggagatcaac     720

acccaccgaa tcagctgcct gtacctggtg ccgacgctgt accacgacct gctggccgat     780

ccggggttcg atgcctgcat cgtgcgcagc gtgaccaagc tcggctttgc cggcgcctcg     840

atgaacgacg gcctgctgcg ccgccttgcg ctggccttcg agccagagct gttcgtgaac     900

cactacggct catccgaggt gtacaccttc agcgtggacc agcgcgccac ccgcaagccc     960

ggcagcgccg ggcgcgccgg catcaacacg cgcctgcgcg tggtgcgcct ggatgcccgc    1020

tcacccgacg atctggcggc taccggcgag gaaggccaga tcattgccga cctgcgcggc    1080

gacgaggcct tcgagggcta ctggaaccgc gacgacgcca acgccaaatc gctgcgcgat    1140

ggctggtact tcaccggtga caccggctac ttcgatgccg agggcgatct cttcgtcagc    1200

ggccgggtgg acgacatgat catcagcggc ggcgagaaca tctccccggt cgatatcgaa    1260

tcggtgctgt cgctgcatcc ggcggtcgat gaggtggcgg tggccggcgt gccggatccg    1320

cgctggggcc agaaggtggt ggctttcgtc aagccgcgcg gcaacatcga cgcgcaagcc    1380

ctggatacct actgccgcgg ctcggacctg gtcaatttca agcgcccgcg cgactacgtc    1440

ttcgtggagg agattcccaa gtcgccggtt ggcaagatcc tgcggcgcaa gttgtccgcc    1500

ggcgaatacg cgctggcccc gcattccatg agccccgacc ctaataccaa ccccaaccag    1560

gctgcggacg ccgcgcctgt cgataccatc aaggagtaa                           1599

<210>  43
<211>  271
<212>  PRT
<213>  2-oxoglutaroyl-CoA hydrolase, Cupriavidus basilensis HMF 14

<400>  43

Met Thr Gln Ala Thr Glu Met Ile His Pro Asp Gln Gln Arg Leu Gln
1               5                   10                  15

Gln Leu Asp Gly Phe Ser Val Glu Ile Asp Ala Gly Arg Glu Arg Ala
            20                  25                  30

Asp Ile Ile Leu His Arg Pro Pro Tyr Asn Val Ile Ala Met Ala Ala
        35                  40                  45

Arg Asp Gln Leu Arg Ala Val Ile Glu Ala Leu Asp Ala Asp Asp Arg
    50                  55                  60

Val Arg Val Ile Val Leu Arg Ser Gln Gly Glu His Phe Ser Ser Gly
65                  70                  75                  80

Gly Asp Ile Lys Gly Phe Leu Glu Ala Ser Pro Glu His Val Ser Gln
                85                  90                  95

Leu Ala Trp Asn Val Ala Ala Pro Ala Arg Cys Ser Lys Pro Val Ile
            100                 105                 110

Ala Ala Asn Arg Gly Tyr Cys Phe Gly Val Gly Phe Glu Leu Ser Leu
        115                 120                 125

Ala Cys Asp Phe Arg Ile Ala Thr Glu Thr Thr Gln Tyr Ala Leu Pro
    130                 135                 140

Glu Gln Lys Leu Gly Gln Ile Pro Gly Ser Gly Gly Ser Ala Arg Leu
145                 150                 155                 160

Gln Lys Met Val Gly Ile Gly Arg Thr Lys Asp Ile Val Met Arg Ser
                165                 170                 175

Arg Arg Ile Ser Gly Lys Gln Ala Tyr Glu Trp Gly Ile Ala Val Glu
            180                 185                 190

Cys Val Ala Asp Ala Glu Leu Glu Ala Ala Thr Asp Ala Leu Val Asp
        195                 200                 205

Glu Leu Arg Gly Phe Ser Pro Leu Ala Gln Arg Thr Ala Lys Lys Leu
    210                 215                 220

Leu Asn Asp Thr Glu Asp Ala Pro Leu Ser Ile Ala Ile Glu Leu Glu
225                 230                 235                 240

Gly His Cys Tyr Ser Arg Leu Arg Ser Ser Asp Asp Phe Arg Glu Gly
                245                 250                 255

Val Glu Ala Phe His Gly Lys Arg Lys Pro Ala Phe Arg Gly Ser
            260                 265                 270

<210>  44
<211>  816

<212>  DNA
<213>  2-oxoglutaroyl-CoA hydrolase, Cupriavidus basilensis HMF 14

<400>  44
atgacccagg caaccgagat gatccatccc gaccagcagc ggctccagca actcgacggc      60

ttctccgtgg agatcgatgc cgggcgcgag cgtgcggaca tcatcctgca ccgtccaccc     120

tacaacgtga tcgccatggc ggcgcgcgac cagttgcgtg ccgtcattga agcgctggat     180

gccgacgatc gcgtgcgcgt gatcgtgctt cgttcgcaag gcgagcattt ttccagcggc     240

ggcgatatca agggcttcct ggaggcatcg cccgagcatg tctcgcaact ggcctggaac     300

gtggcggcgc cggcgcgctg cagcaagccg gtgattgccg ccaaccgcgg ctactgcttt     360

ggcgtgggct tcgagctgtc gctggcgtgc gacttccgca tcgccaccga gaccacgcag     420

tacgcgctgc cggaacagaa gctcggccag atccccggct cgggcggctc ggcgcgcctg     480

cagaagatgg tgggcatcgg ccgcaccaag gacatcgtga tgcgctcgcg ccgcatctcg     540

ggcaagcagg cctatgagtg gggcatcgcc gtggaatgcg tggcagacgc cgagctggaa     600

gccgccaccg atgcgctggt cgacgagctg cgcggcttct cgccgctggc gcagcgcacc     660

gccaagaagc tgctcaacga caccgaggac gcaccgctgt cgattgccat cgagctggaa     720

gggcattgct atagccgcct gcgcagctcg gacgatttcc gcgaaggcgt ggaagccttc     780

cacggcaagc gcaagccggc gttccgcggc agctga                               816

<210>  45
<211>  449
<212>  PRT
<213>  major facilitator superfamily transporter, Cupriavidus basilensis HMF 14

<400>  45

Met Glu Ala Val Ala Lys Lys Ser Ala Ala Thr Ile Ser Glu Ala Leu
1               5                   10                  15

Pro Ala Ala Ser Asn Arg Gln Val Phe Gly Ala Val Ala Ala Ser Cys
            20                  25                  30

Met Gly Trp Ala Leu Asp Leu Phe Asp Leu Phe Ile Leu Leu Phe Val
        35                  40                  45

Ala Pro Val Ile Gly Arg Leu Phe Phe Pro Ser Glu His Ala Met Leu
    50                  55                  60

Ser Leu Ala Ala Val Tyr Ala Ser Phe Ala Val Thr Leu Leu Met Arg
65                  70                  75                  80

Pro Leu Gly Ser Ala Ile Phe Gly Ser Tyr Ala Asp Arg His Gly Arg
                85                  90                  95

Lys Gly Ala Met Val Val Ala Val Thr Gly Val Gly Leu Ser Thr Ala
            100                 105                 110

Ala Phe Gly Leu Leu Pro Thr Val Gly Gln Val Gly Leu Leu Ala Pro
        115                 120                 125

Ala Leu Phe Ile Leu Leu Arg Leu Val Gln Gly Ile Phe Val Gly Gly
    130                 135                 140

Val Val Ala Ser Thr His Thr Ile Gly Thr Glu Ser Val Pro Pro Ser
145                 150                 155                 160

Trp Arg Gly Ala Val Ser Gly Leu Val Gly Gly Gly Gly Ala Gly Leu
                165                 170                 175

Gly Ala Leu Leu Ala Ser Ile Thr Tyr Met Ala Met Thr Ala Leu Phe
            180                 185                 190

Pro Gly Glu Ala Phe Asp Ala Trp Gly Trp Arg Cys Met Phe Phe Ser
        195                 200                 205

Gly Ile Ile Ser Ser Val Leu Gly Leu Phe Ile Phe Asn Ser Leu Glu
    210                 215                 220

Glu Ser Pro Leu Trp Lys Gln Leu Gln Ala Ala Lys Gly His Ala Ala
225                 230                 235                 240

Pro Val Glu Asn Pro Leu Arg Val Ile Phe Ser Arg Gln Tyr Arg Gly
                245                 250                 255

Val Leu Phe Val Asn Ile Leu Leu Thr Val Gly Gly Gly Ser Ala Tyr
            260                 265                 270

Tyr Leu Thr Ser Gly Tyr Leu Pro Thr Phe Leu Lys Val Val Val Lys
        275                 280                 285

Ala Ser Ala Gly Glu Ser Ala Ala Ile Leu Met Ala Ser Ser Leu Gly
    290                 295                 300

Val Ile Val Ala Ser Ile Leu Ala Gly His Leu Ser Thr Met Ile Gly
305                 310                 315                 320

Arg Lys Arg Ala Phe Leu Leu Ile Gly Ala Leu Asn Val Val Val Leu
                325                 330                 335

Pro Leu Leu Tyr Gln Trp Met Pro Ala Ala Pro Asp Thr Thr Thr Leu
            340                 345                 350

Gly Leu Tyr Ala Val Val Leu Ser Met Leu Gly Cys Ser Gly Phe Ala
        355                 360                 365

Pro Ile Leu Ile Phe Leu Asn Glu Arg Phe Pro Thr Ser Ile Arg Ala
    370                 375                 380

Thr Gly Thr Gly Leu Ser Trp Asn Ile Gly Phe Ala Val Gly Gly Met
385                 390                 395                 400

Met Pro Thr Phe Ala Ser Leu Cys Ala Ser Thr Pro Ala Glu Leu Pro
                405                 410                 415

Met Val Leu Gly Ile Phe Leu Ala Val Val Thr Ile Ile Tyr Leu Val
            420                 425                 430

Gly Ala Phe Ile Val Pro Glu Thr Val Gly Arg Leu Gly Asp Asn Gly
        435                 440                 445

Ala

<210>  46
<211>  1350
<212>  DNA
<213>  major facilitator superfamily transporter, Cupriavidus basilensis HMF 14

<400>  46
   atggaagccg tagcaaagaa gagtgcagcg acgatcagcg aggcgctgcc agcggcgagc      60
   aatcgccagg tgtttggtgc cgtggcggcg tcgtgcatgg gatgggcgct ggacctgttc     120
   gacctgttca tcctgctgtt cgtggcgccc gtgatcggca ggctgttttt cccgtcggag     180
   cacgcgatgc tgtcgctggc ggcggtgtat gcgtcgtttg ccgtgacgct gctgatgcgg     240
   ccgctcggct cggcgatctt cggctcttat gccgaccgcc acggccgcaa gggggcgatg     300
   gtggttgccg tcactggcgt tggcttgtcc acggcggcgt tcggcctgct gccgacggtg     360
   ggtcaggtgg ggctgcttgc gccagccttg tttatcctgc tgcggctggt gcagggcatc     420
   ttcgtgggtg gcgtggtggc atccacccac accatcggta ccgaatcggt gcccccgtcc     480
   tggcgcggcg ccgtttccgg gctggttggt ggcggtggcg cgggtctcgg ggcgctgctg     540
   gcttccatta cctacatggc gatgaccgcg ctgtttccgg gggaagcgtt cgatgcctgg     600
   ggttggcgct gcatgttctt ctccggcatc atcagctcgg tgctcggcct gttcatcttc     660
   aactcgctgg aggagtctcc gctgtggaag cagttgcagg cggccaaggg gcacgccgcg     720
   ccggttgaga acccgctgcg cgtgatcttc tcccgccagt accgtggtgt cctcttcgtc     780
   aacatcctgc tcaccgtggg cggtggcagc gcctactacc tgacctccgg ctatctgccg     840
   accttcctca aggtggtggt gaaggcatcg gctggcgagt ctgccgccat cctgatggcc     900
   agcagtctgg gtgtgatcgt ggcatcgatt cttgccggcc acctcagtac gatgatcggc     960
   cgcaagcgag ccttcctgtt gatcggcgcg ctgaacgtgg tagtactgcc gctgctctac    1020
   cagtggatgc cggcggcgcc ggacaccacc acgctcggcc tgtatgctgt ggtgctgtcc    1080
   atgctgggct gcagcggctt cgccccgatc ctcattttcc tgaacgaacg gttccccacc    1140
   agcatccgtg ccacggggac cggcctgtca tggaatatcg gatttgccgt cggtggcatg    1200
   atgccgacgt ttgcttcgct gtgcgccagc acccctgccg aactgcccat ggtgctgggc    1260
   atcttcctgg cggttgtcac catcatctac ctggtgggtg cgttcatcgt tccggagacg 1320
   gtagggcgcc ttggcgacaa tggagcgtag 1350

## Description

### Field of the invention

The present invention relates to the isolation of a novel Cupriavidus species, and to novel polynucleotide sequences encoding novel polypeptides derived from this organism. The enzymes described herein are useful in the detoxification of lignocellulose hydrolysates in recombinant organisms.

### Background of the invention

The use of lignocellulose-containing material for the production of second-generation biofuels and biochemicals is well known in the art. Commonly, the process comprises the steps of pretreatment, hydrolysis, and fermentation.

A significant drawback of the pretreatment process step, in particular if involving acidic conditions, is the release of degradation products from the lignocellulose-containing material that may evolve into fermentation inhibitors during the hydrolysis process step. Specifically furanic compounds, such as furfural, furfuryl alcohol, 5-(hydroxymethyl)-furfural (HMF) and/or furoic acid pose problems that are difficult to mitigate, since they inhibit the growth of e.g. ethanologenic microorganisms employed in the fermentation step, thereby inhibiting a suitable performance of these organisms and reducing yields.

Approaches to remove the degradation products at least in part by chemical, physical or biological methods have been discussed in detail in Bioresource Technology 93 (2004), 1-10, and Microbiol. Biotechnol, DOI 10.1007/s00253-009-1875-1, published 31st January 2009.

WO2009/030713 discloses the washing of pre-treated lignocellulose-containing material, further referred to as lignocellulose hydrolysate. However, the inhibitors have a comparatively low solubility in water, while recycling of the wash water is only possible to a very limited extent due to build-up of the inhibitors, thus requiring large amounts of water. This makes the disclosed process cumbersome and difficult to apply on a commercial scale.

WO2009/017441 discloses a process for biological detoxification of lignocellulosic hydrolysate with genetically modified yeasts, while US 7,067,303 discloses the use of a fungus for the same purpose.

While such microorganisms can metabolize the fermentation inhibitors or convert them into less toxic compounds, their preferred carbon source are fermentable sugars, thereby reducing the fermentable sugar content of the lignocellulosic hydrolysate, and thus reducing the overall yield of the desired fermentation products.

Consequently, there is a need for providing a process for detoxifying pre-treated lignocellulose-containing material to obtain substrates suitable for hydrolysis, fermentation or thermochemical conversion, while preserving the fermentable sugar content for subsequent or simultaneous process steps.

### SUMMARY OF THE INVENTION

The present invention relates to an isolated microorganism of the family Burkholderiaceae, genus Cupriavidus, and the species and strain designation basilensis HMF 14. This strain has been deposited at the Deutsche Sammlung von Mikroorganismen und Zellkulturen GmbH (German Collection of Microorganisms and Cell Cultures), Inhoffenstraße 7B, 38124 Braunschweig, GERMANY as strain HMF 14, Deposit number DSM 22875.

The invention further relates to a bacterial culture comprising the Cupriavidus microorganism of Cupriavidus basilensis HMF 14 strain Deposit number DSM 22875. This strain preferably grows, when provided with HMF, Furfurylalcohol, Furfural and/or Furoic acid as a carbon source. The invention further relates to an isolated microorganism or culture according to the invention, which expresses the following enzymes: a Furoyl-CoA dehydrogenase (composed of three polypeptide subunits), a Furoyl-CoA synthetase, a 2-oxoglutaroyl-CoA hydrolase, a 2,5-furan-dicarboxylic acid decarboxylase 1, a 2,5-furan-dicarboxylic acid decarboxylase 2 and a HMF/furfural oxidoreductase. These enzymes form a novel pathway to selectively metabolize furanic compounds.

The present invention further provides polynucleotides encoding polypeptides having the following activities : a 2-furoyl-CoA:acceptor 5-oxidoreductase (hydroxylating), EC 1.3.99.8 (further referred to herein as Furoyl-CoA dehydrogenase), a Furoyl-CoA synthetase, EC 6.2.1.31, , a 2-oxoglutaroyl-CoA hydrolase (thioester hydrolase), EC 3.1.2, a first 2,5-furan-dicarboxylic acid decarboxylase (Carboxy-lyase), EC 4.1.1; a second 2,5-furan-dicarboxylic acid decarboxylase (Carboxy-lyase), EC 4.1.1 and a HMF/furfural oxido-reductase EC 1.1.3 and 1.2.3.

A polynucleotide of the invention typically encodes a polypeptide having Furoyl-CoA dehydrogenase activity (large subunit hmfA). Another polynucleotide of the invention typically encodes a polypeptide having Furoyl-CoA dehydrogenase activity (FAD binding subunit, hmfB). A further polynucleotide of the invention typically encodes a Furoyl-CaA dehydrogenase (2Fe-2S iron sulfur subunit, hmfC).Yet another polynucleotide of the invention typically encodes a polypeptide having Furoyl-CoA synthetase activity. A further polynucleotide of the invention typically encodes a polypeptide having 2-oxoglutaroyl-CoA hydrolase activity. A further polynucleotide of the invention typically encodes a polypeptide having 2,5-furan-dicarboxylic acid decarboxylase activity, while a further polynucleotide of the invention typically encodes a polypeptide having a second 2,5-furan-dicarboxylic acid decarboxylase activity.

### Brief Description of the Figures

Figure 1 shows the growth of C. basilensis HMF14 on mineral salts medium with furfural as the sole carbon source.
Figure 2 illustrates the growth of C. basilensis HMF14 on different concentrations of furfural (A) or HMF (B).
Figure 3 depicts the detection of PHA in cultures of C. basilensis HMF14 in minimal medium with 120 mM acetate.
Figure 4 illustrates the detoxification of lignocellulosic hydrolysate by C. basilensis HMF14.
Figure 5 illustrates a reaction scheme of reactions catalysed by Cupriavidus basilensis HMF 14.
Figure 6 is a schematic representation of the genetic organization of the furfural and HMF metabolic genes in C. basilensis HMF14 (A) and other species (B) that were identified as potential furfural and / or HMF utilizers.

### Description of the Sequences

Sequences SEQ ID NO: 1 to 14 set out the DNA sequences of several synthetic DNA primers used for PCR and/or for cloning or isolation of relevant genes. Underlined sequences indicate a restriction site.

### Synthetic DNA

SEQ ID NO: 1

Hybridizes to hmfA, forward primer.

GCACGCGCCTGAGTTACGAC

### Synthetic DNA

SEQ ID NO: 2

Hybridizes to hmfR2, forward primer.

CATGCTCGGCGCTGGTGAC

### Synthetic DNA

SEQ ID NO: 3

Hybridizes near start site of hmfF gene, forward primer.

CATGAATTCCGACCCAGGAGTCACGCCAT

### Synthetic DNA

SEQ ID NO: 4

Hybridizes near stop site of hmfF gene, reverse primer.

CGGCGGCCGCGGATATACCGACAATGATGCGTCTCT

### Synthetic DNA

SEQ ID NO: 5

Hybridizes near stop site of hmfG gene, reverse primer.

CGGCGGCCGCTGTCTCCTGCCTGTTCAGCATTCA

### Synthetic DNA

SEQ ID NO: 6

Hybridizes near stop site of hmfD gene, reverse primer.

GCGGGCCCCTTACTCCTTGATGGTATCGACAGG

### Synthetic DNA

SEQ ID NO: 7

Hybridizes near start site of hmfA gene, forward primer.

GCGGTACCGGGAGGGCCGGTCATGAG

### Synthetic DNA

SEQ ID NO: 8

Hybridizes near start site of hmfD gene, forward primer.

GCGGTACCGGCGTAGATACCCAGGAGGC

### Synthetic DNA

SEQ ID NO: 9

Hybridizes near stop site of hmfC gene, reverse primer.

GCGGGCCCCCACGCTTTGCAGGAAGGTG

SEQ ID NO: 10

Hybridizes near start site of hmfG gene, forward primer.

CGGAATTCCGGCGCATGTGTTCACGC

### Synthetic DNA

SEQ ID NO: 10

CGGAATTCCGGCGCATGTGTTCACGC

### Synthetic DNA

SEQ ID NO: 11

Hybridizes near stop site of hmfE gene, reverse primer.

GCGCGGCCGCCGCCCGCGATTTCCATCAG

### Synthetic DNA

SEQ ID NO: 12

Hybridizes near start site of hmfE gene, forward primer.

GCGGTACCCCATCAAGGAGTAAGACATGACCC

### Synthetic DNA

SEQ ID NO: 13

Hybridizes near start site of hmfH gene, forward primer.

CGGAATTCCACATGACAAGGGGAGACCG

### Synthetic DNA

SEQ ID NO: 14

Hybridizes near stop site of hmfH gene, reverse primer.

CGGAATTCGCTTCGGTCTTCAACTCGGATG

### SEQ ID NO: 15 sets out the amino acid sequence

of a putative furan aldehyde dehydrogenase (adh) of Cupriavidus basilensis HMF14.

SEQ ID NO: 16 sets out the coding sequence of a putative furan aldehyde dehydrogenase (adh).

SEQ ID NO: 17 sets out the amino acid sequence of LysR-type transcriptional regulator hmfR1.

SEQ ID NO: 18 sets out the coding sequence of hmfR1 SEQ ID NO: 19 sets out the amino acid sequence of 5-hydroxymethyl-2-furoic acid decarboxylase 1 hmfF.

SEQ ID NO: 20 sets out the coding sequence of hmfF.

SEQ ID NO: 21 sets out the amino acid sequence of 5-hydroxymethyl-2-furoic acid decarboxylase 2 hmfG.

SEQ ID NO: 22 sets out the coding sequence of hmfG.

SEQ ID NO: 23 sets out sets out the amino acid sequence of the extracytoplasmic solute receptor hmfH'.

SEQ ID NO: 24 sets out the coding sequence of hmfH'. SEQ ID NO: 25 sets out the amino acid sequence of HMF/furfural oxidoreductase hmfH.

SEQ ID NO: 26 sets out the coding sequence of hmfH.

SEQ ID NO: 27 sets out the amino acid sequence of fatty acid hydroxylase hyd.

SEQ ID NO: 28 sets out the coding sequence of hyd.

SEQ ID NO: 29 sets out the amino acid sequence of truncated LysR-type transcriptional regulator LysR hmfRt. SEQ ID NO: 30 sets out the coding sequence for LysR hmfRt .

SEQ ID NO: 31 sets out the amino acid sequence for major facilitator superfamily transportera putative furanic MFS-type transporter mfsl.

SEQ ID NO: 32 sets out the coding sequence for a putative furanic MFS-type transporter major facilitator superfamily transporter mfs1.

SEQ ID NO: 33 sets out the amino acid sequence for LysR-type regulator hmfR2.

SEQ ID NO: 34 sets out the coding sequence for LysR-type regulator hmfR2.

SEQ ID NO: 35 sets out the amino acid sequence for the furoyl-CoA dehydrogenase large subunit hmfA.

SEQ ID NO: 36 sets out the coding sequence for hmfA.

SEQ ID NO: 37 sets out the amino acid sequence for the Furoyl-CoA dehydrogenase FAD binding subunit, hmfB.

SEQ ID NO: 38 sets out the coding sequence for hmfB.

SEQ ID NO: 39 sets out the amino acid sequence for the Furoyl-CoA dehydrogenase 2Fe-2S iron sulfur subunit, hmfC SEQ ID NO: 40 sets out the coding sequence for hmfC.

SEQ ID NO: 41 sets out the amino acid sequence for the Furoyl-CoA synthetase hmfD.

SEQ ID NO: 42 sets out the coding sequence for hmfD.

SEQ ID NO: 43 sets out the amino acid sequence for the 2-oxoglutaroyl-CoA hydrolase hmfE.

SEQ ID NO: 44 sets out the coding sequence for hmfE.

SEQ ID NO: 45 sets out the amino acid sequence for a putative furanic MFS-type transporterthe major facilitator superfamily transporter mfs2.

SEQ ID NO: 46 sets out the coding sequence for a putative furanic MFS-type transporter mfs2.

### Definition of Terms

The following terms will be understood as defined herein unless otherwise stated. Such definitions include without recitation those meanings associated with these terms known to those skilled in the art.

Within the context of the subject invention, the term "furanic compound may be selected from one or more of the following compounds: Furfural, furfurylalcohol, furoic acid, hydroxymethylfurfural, hydroxymethylfurancarboxylic acid, furandimethanol, diformylfuran, formylfuran carboxylic acid, furandicarboxylic acid, formylthiophene, thiophene methanol, thiophene carboxylic acid, hydroxymethyl formylthiaphene, hydroxymethyl thiopene carboxylic acid, thiophene dimethanol, diformyl thiophene, formylthiopene carboxylic acid, thiophene dicarboxylic acid, formylpyrrole, pyrrolylmethanol, pyrrole carboxylic acid, hydroxymethyl formylpyrrole, hydroxymethyl pyrrole carboxylic acid, pyrroledimethanol, diformylpyrrole, formylpyrrole carboxylic acid and pyrrole dicarboxylic acid.

Preferred furanic compounds are furfural, furfurylalcohol, furoic acid, hydroxymethylfurfural, hydroxymethylfurancarboxylic acid, furandimethanol, diformylfuran, formylfuran carboxylic acid, furandicarboxylic acid, more preferably at least one of hydroxymethylfurfural (HMF), hydroxymethylfuran carboxylic acid (HMF acid), 2,5-dihydroxymethylfuran (HMF alcohol). At the furanic compound, the furan ring or any or its substitutable side-group may be substituted, e.g. with OH, alkyl (e.g. C1-C10 alkyl), allyl, aryl or RO-ether moiety, including cyclic groups, in the furan ring on any available position. More preferably, Furanic compounds are herein understood to be any compound having a furan group that may be oxidized to 2,5-furan-dicarboxylic acid or a precursor thereof.

Expression of polypeptides according to the invention: Regardless of the exact mechanism utilized for expression of enzymes, it is contemplated that such expression is transferable by the introduction of genes encoding these enzymes into another host cell by methods known in the art. Genetic elements as herein defined include nucleic acids (generally DNA or RNA) having expressible coding sequences for products such as proteins, specifically enzymes, apoproteins or antisense RNA, which express or regulate expression of relevant enzymes. The expressed proteins can function as enzymes, repress or derepress enzyme activity or control expression of enzymes. Recombinant DNA encoding these expressible sequences can be either chromosomal (integrated into the host cell chromosome by, for example, homologous recombination) or extra-chromosomal (for example, carried by one or more plasmids, cosmids and other vectors capable of self replication). It is understood that the recombinant DNA utilized for transforming the host cell in accordance with this invention can include, in addition to structural genes and transcription factors, expression control sequences, including promoters, repressors and enhancers, that act to control expression or derepression of coding sequences for proteins, apoproteins or antisense RNA. For example, such control sequences can be inserted into wild-type host cells to promote overexpression of selected enzymes already encoded in the host cell genome, or alternatively they can be used to control synthesis of extrachromosomally encoded enzymes.

Recombinant DNA can be introduced into the host cell by any means, including, but not limited to, plasmids, cosmids, phages, yeast artificial chromosomes or other vectors that mediate transfer of genetic elements into a host cell. These vectors can include an origin of replication, along with cis-acting control elements that control replication of the vector and the genetic elements carried by the vector. Selectable markers can be present on the vector to aid in the identification of host cells into which genetic elements have been introduced.

Means for introducing genetic elements into a host cell (e.g. cloning) are well known to the skilled artisan. One can utilize an extrachromosomal multi-copy plasmid vector to insert the genetic elements in accordance with the present invention. Plasmid-borne introduction of the genetic element into host cells involves an initial cleaving of a plasmid vector with a restriction enzyme, followed by ligation of the plasmid and genetic elements encoding for the targeted enzyme species in accordance with the invention. Upon recircularization of the ligated recombinant plasmid, infection (e.g., packaging in phage lambda) or other mechanism for plasmid transfer (e.g., electroporation, microinjection, etc.) is utilized to transfer the plasmid into the host cell. Plasmids suitable for insertion of genetic elements into the host cell are well known to the skilled artisan. Other gene cloning methods include, but are not limited to, direct integration of the genetic material into the chromosome. This can occur by a variety of means, including cloning the genetic elements described herein on non-replicating plasmids flanked by homologous DNA sequences of the host chromosome; upon transforming said recombinant plasmid into a host the genetic elements can be introduced into the chromosome by DNA recombination. Such recombinant strains can be recovered if the integrating DNA fragments contain a selectable marker, such as antibiotic resistance. Alternatively, the genetic elements can be directly introduced into the chromosome of a host cell without use of a non-replicating plasmid. This can be done by synthetically producing DNA fragments of the genetic elements in accordance to the present invention that also contain homologous DNA sequences of the host chromosome. Again if these synthetic DNA fragments also contain a selectable marker, the genetic elements can be inserted into the host chromosome.

A DNA fragment, as used herein, may encode regulatory and/or structural genetic information. A DNA fragment useful in the present invention shall also include: nucleic acid molecules encoding sequences complementary to those provided; nucleic acid molecules (DNA or RNA) which hybridize under stringent conditions to those molecules that are provided; or those nucleic acid molecules that, but for the degeneracy of the genetic code, would hybridize to the molecules provided or their complementary strands. "Stringent" hybridization conditions are those that minimize formation of double stranded nucleic acid hybrids from non-complementary or mismatched single stranded nucleic acids. In addition, hybridization stringency may be affected by the various components of the hybridization reaction, including salt concentration, the presence or absence of formamide, the nucleotide composition of the nucleic acid molecules, etc. The nucleic acid molecules useful in the present invention may be either naturally or synthetically derived.

A "heterologous" or "exogenous" DNA fragment has been introduced into the host microorganism by any process such as transformation, transfection, transduction, conjugation, electroporation, etc. Additionally, it should be noted that it is possible that the host cell into which the "heterologous" DNA fragment has been inserted may itself also naturally harbour molecules encoding the same or similar sequences. A molecule such as this is referred to as a "homologous" DNA molecule.

A stably transformed microorganism is one that has had one or more DNA fragments introduced such that the introduced molecules are maintained, replicated and segregated in a growing culture. Stable transformation may be due to multiple or single chromosomal integration (s) or by (an) extrachromosomal element(s) such as (a) plasmid vector(s). A plasmid vector is capable of directing the expression of polypeptides encoded by particular DNA fragments.

Expression may be constitutive or regulated by inducible (or repressible) promoters that enable high levels of transcription of functionally associated DNA fragments encoding specific polypeptides.

Regardless of the exact mechanism utilized for expression of enzymes necessary for detoxification of lignocelluloses, it is contemplated that such expression is transferable by the introduction of genes encoding these enzymes into another host cell by methods known in the art. Genetic elements as herein defined include nucleic acids (generally DNA or RNA) having expressible coding sequences for products such as proteins, specifically enzymes, apoproteins or antisense RNA, which express or regulate expression of relevant enzymes. The expressed proteins can function as enzymes, repress or derepress enzyme activity or control expression of enzymes. Recombinant DNA encoding these expressible sequences can be either chromosomal (integrated into the host cell chromosome by, for example, homologous recombination) or extra-chromosomal (for example, carried by one or more plasmids, cosmids and other vectors capable of self replication). It is understood that the recombinant DNA utilized for transforming the host cell in accordance with this invention can include, in addition to structural genes and transcription factors, expression control sequences, including promoters, repressors and enhancers that act to control expression or derepression of coding sequences for proteins, apoproteins or antisense RNA. For example, such control sequences can be inserted into wild-type host cells to promote overexpression of selected enzymes already encoded in the host cell genome, or alternatively they can be used to control synthesis of extrachromosomally encoded enzymes.

Recombinant DNA can be introduced into the host cell by any means, including, but not limited to, plasmids, cosmids, phages, yeast artificial chromosomes or other vectors that mediate transfer of genetic elements into a host cell. These vectors can include an origin of replication, along with cis-acting control elements that control replication of the vector and the genetic elements carried by the vector. Selectable markers can be present on the vector to aid in the identification of host cells into which genetic elements have been introduced

Means for introducing genetic elements into a host cell (e.g. cloning) are well known to the skilled artisan. One can utilize an extrachromosomal multi-copy plasmid vector to insert the genetic elements in accordance with the present invention. Plasmid-borne introduction of the genetic element into host cells involves an initial cleaving of a plasmid vector with a restriction enzyme, followed by ligation of the plasmid and genetic elements encoding for the targeted enzyme species in accordance with the invention. Upon recircularization of the ligated recombinant plasmid, infection (e.g., packaging in phage lambda) or other mechanism for plasmid transfer (e.g., electroporation, microinjection, etc.) is utilized to transfer the plasmid into the host cell. Plasmids suitable for insertion of genetic elements into the host cell are well known to the skilled artisan.

Other gene cloning methods include, but are not limited to, direct integration of the genetic material into the chromosome. This can occur by a variety of means, including cloning the genetic elements described herein on non-replicating plasmids flanked by homologous DNA sequences of the host chromosome; upon transforming said recombinant plasmid into a host the genetic elements can be introduced into the chromosome by DNA recombination. Such recombinant strains can be recovered if the integrating DNA fragments contain a selectable marker, such as antibiotic resistance. Alternatively, the genetic elements can be directly introduced into the chromosome of a host cell without use of a non-replicating plasmid. This can be done by synthetically producing DNA fragments of the genetic elements in accordance to the present invention that also contain homologous DNA sequences of the host chromosome. Again if these synthetic DNA fragments also contain a selectable marker, the genetic elements can be inserted into the host chromosome.

A preferred embodiment of the invention is a host cell comprising one or more polypeptides, polynucleotides, nucleic acid constructs or vectors according to the invention. This may be a cell in which the polypeptides, polynucleotides, nucleic acid constructs or vectors can suitably be expressed. The enzymes according to the invention may be favourably expressed in a host cell. The host cell according to the invention may be any host cell. The cell may be a prokaryote cell, an eukaryote cell, a plant cell or an animal cell.

The cell may be a host microorganism, which may be an autonomous single-celled organism useful for microbial production of biofuels, such as ethanol, as well as production of chemicals, including both eukaryotic and prokaryotic microorganisms. Useful microorganisms may be prokaryotes or eukaryotes and include organisms like bacteria, yeast, and fungi and plants.

Such a host microorganism usually contains all DNA, either endogenous or heterologous, required for the digestion of furanic compounds from lignocellulose hydrolysate. It may further preferably also comprise all DNA, either endogenous or heterologous, required for the conversion of fermentable sugars from lignocellulose hydrolysate for the production of a biofuel component such as for instance ethanol, n- or iso-butanol from lignocelluloses hydrosylate. In such cell one or more gene may be deleted, knocked-out or disrupted in full or in part. According to an embodiment, the host cell according to the invention is a eukaryotic host cell. Preferably, the eukaryotic cell is a mammalian, insect, plant, fungal, or algal cell. Preferred mammalian cells include e.g. Chinese hamster ovary (CHO) cells, COS cells, 293 cells, PerC6 cells, and hybridomas. Preferred insect cells include e.g. Sf9 and Sf21 cells and derivatives thereof.

More preferably, the eukaryotic cell is a fungal cell, such as for instance a yeast cell, such as those of the Candida, Hansenula, Kluyveromyces, Pichia, Saccharomyces, Schizosaccharomyces, and/or Yarrowia strains. More preferably, it si a cell selected from Kluyveromyces lactis, S. cerevisiae, Hansenula polymorpha, Yarrowia lipolytica and Pichia pastoris, or a filamentous fungal cell. More preferably, the eukaryotic cell is a yeast cell. "Filamentous fungi" include all filamentous forms of the subdivision Eumycota and Oomycota (as defined by Hawksworth et al., In, Ainsworth and Bisby's Dictionary of The Fungi, 8th edition, 1995, CAB International, University Press, Cambridge, UK). The filamentous fungi are characterized by a mycelial wall composed of chitin, cellulose, glucan, chitosan, mannan, and other complex polysaccharides. Vegetative growth is by hyphal elongation and carbon catabolism is obligately aerobic. Filamentous fungal strains include, but are not limited to, strains of Acremonium, Agaricus, Aspergillus, Aureobasidium, Chrysosporium, Coprinus, Cryptococcus, Filibasidium, Fusarium, Humicola, Magnaporthe, Mucor, Myceliophthora, Neocallimastix, Neurospora, Paecilomyces, Penicillium, Piromyces, Panerochaete, Pleurotus, Schizophyllum, Talaromyces, Thermoascus, Thielavia, Tolypocladium, and Trichoderma.

Preferred filamentous fungal cells belong to a species of an Aspergillus, Chrysosporium, Penicillium, Talaromyces or Trichoderma genus, and most preferably a species of Aspergillus niger, Aspergillus awamori, Aspergillus foetidus, Aspergillus sojae, Aspergillus fumigatus, Talaromyces emersonii, Aspergillus oryzae, Chrysosporium lucknowense, Trichoderma reesei or Penicillium chrysogenum. When the host cell according to the invention is an Aspergillus host cell, the host cell preferably is CBS 513.88, CBS124.903 or a derivative thereof. Several strains of filamentous fungi are readily accessible to the public in a number of culture collections, such as the American Type Culture Collection (ATCC), Deutsche Sammlung von Mikroorganismen und Zellkulturen GmbH (DSM), Centraalbureau Voor Schimmelcultures (CBS), and Agricultural Research Service Patent Culture Collection, Northern Regional Research Center (NRRL) Aspergillus niger CBS 513.88, Aspergillus oryzae ATCC 20423, IFO 4177, ATCC 1011, ATCC 9576, ATCC14488-14491, ATCC 11601, ATCC12892, P. chrysogenum CBS 455.95, Penicillium citrinum ATCC 38065, Penicillium chrysogenum P2, Talaromyces emersonii CBS 124.902, Acremonium chrysogenum ATCC 36225 or ATCC 48272, Trichoderma reesei ATCC 26921 or ATCC 56765 or ATCC 26921, Aspergillus sojae ATCC11906, Chrysosporium lucknowense ATCC44006 and derivatives thereof.

According to another preferred embodiment, the host cell according to the invention is a prokaryotic cell. Preferably, the prokaryotic host cell is bacterial cell. The term "bacterial cell" includes both Gram-negative and Gram-positive microorganisms. Suitable bacteria may be selected from e.g. Escherichia, Anabaena, Caulobacter, Gluconobacter, Rhodobacter, Pseudomonas, Paracoccus, Bacillus, Brevibacterium, Corynebacterium, Rhizobium (Sinorhizobium), Flavobacterium, Klebsiella, Enterobacter, Lactobacillus, Lactococcus, Methylobacterium, Staphylococcus or Streptomyces. Preferably, the bacterial cell is selected from the group consisting of B. subtilis, B. amyloliquefaciens, B. licheniformis, B. puntis, B. megaterium, B. halodurans, B. pumilus, G. (Gluconobacter) oxydans, Caulobacter crescentus CB 15, Methylobacterium extorquens, Rhodobacter sphaeroides, Pseudomonas zeaxanthinifaciens, Pseudomonas putida, Pseudomonas putida S12, Paracoccus denitrificans, E. coli, C. glutamicum, Staphylococcus carnosus, Streptomyces lividans, Sinorhizobium melioti and Rhizobium radiobacter.

Preferred host organisms are any genus/species that are able to produce and incorporate the molydopterin cofactor required for the furoyl-CoA dehydrogenase, or comprising a molydopterin cofactor independent furoyl-CoA dehydrogenase, flavin adenine dinucleotide (FAD) or nicotine adenine dehydrogenase (NAD+).

For specific uses of a compound produced in a host cell according to the invention, the selection of the host cell may be made according to such use. Where e.g. the compound produced in a host cell according to the invention is to be used in food applications, a host cell may be selected from a food-grade organism such as Saccharomyces cerevisiae. Specific uses include, but are not limited to, food, (animal) feed, pharmaceutical, agricultural such as crop-protection, and/or personal care applications.

The invention further relates to method for the preparation of polypeptides having various enzymatic activities. This method comprises cultivating a cell according to the invention under conditions which allow for expression of the appropriate polypeptide and, optionally, recovering the expressed polypeptides and to a polypeptide obtainable by that method.

A polypeptide according to the invention having aldehyde dehydrogenase activity comprises the amino acid sequence set out in SEQ ID NO: 15 or an amino acid sequence encoded by the nucleotide sequence of SEQ ID NO: 16 or a variant polypeptide thereof, wherein the variant has at least 41% sequence identity or more with the sequence set out in SEQ ID NO: 15.

In one embodiment the variant nucleic acid molecule comprises a nucleotide sequence encoding a protein, wherein the variant nucleic acid molecule comprises a substantially homologous nucleotide sequence of at least 64%, 66%, 68%, 70%, 72%, 75%, 80%, 85%, 90%, 91%, 92%, 93%, 94%, 95%, 96%, 97%, 98%, 99% or more homologous to the nucleotide sequence shown in SEQ ID NO: 16.

In another embodiment the variant protein comprises a substantially homologous amino acid sequence of at least 41%, 45%, 47%, 50%, 55%, 60%, 65%, 70%, 75%, 80%, 85%, 90%, 91%, 92%, 93%, 94%, 95%, 96%, 97%, 98%, 99% or more homologous to the amino acid sequence shown in SEQ ID NO: 15.

An embodiment of the invention is a polynucleotide which comprises:
(a) the nucleotide sequence set out in SEQ ID NO: 16;
(b) a nucleotide sequence which hybridizes selectively
with a polynucleotide being the reverse complement of SEQ ID NO: 16; (c) a nucleotide sequence having at least 64% sequence identity or more with the nucleotide sequence of SEQ ID NO: 16; (d) a fragment of a nucleotide sequence as defined in (a), (b) or (c) which is at least about 100 nucleotides in length; (e) a sequence which is degenerate as a result of the genetic code to a sequence as defined in any one of (a), (b), (c) or (d); (f) a nucleotide sequence which is the reverse complement of a nucleotide sequence as defined in (a), (b), (c), (d) or (e).

A polypeptide according to the invention having LysR family transcriptional regulator activity comprises the amino acid sequence set out in SEQ ID NO: 17 or an amino acid sequence encoded by the nucleotide sequence of SEQ ID NO: 18 or a variant polypeptide thereof, wherein the variant has at least 47% sequence identity or more with the sequence set out in SEQ ID NO: 17.

In one embodiment the variant nucleic acid molecule comprises a nucleotide sequence encoding a protein, wherein the variant nucleic acid molecule comprises a substantially homologous nucleotide sequence of at least 72%, 75%, 80%, 85%, 90%, 91%, 92%, 93%, 94%, 95%, 96%, 97%, 98%, 99% or more homologous to the nucleotide sequence shown in SEQ ID NO: 18.

In another embodiment the variant protein comprises a substantially homologous amino acid sequence of at least 47%, 50%, 55%, 60%, 65%, 70%, 75%, 80%, 85%, 90%, 91%, 92%, 93%, 94%, 95%, 96%, 97%, 98%, 99% or more homologous to the amino acid sequence shown in SEQ ID NO: 17.

An embodiment of the invention is a polynucleotide which comprises: (a) the nucleotide sequence set out in SEQ ID NO: 18; (b) a nucleotide sequence which hybridizes selectively with a polynucleotide being the reverse complement of SEQ ID NO: 18; (c) a nucleotide sequence having at least 72% sequence identity or more with the nucleotide sequence of SEQ ID NO: 18; (d) a fragment of a nucleotides sequence as defined in (a), (b) or (c) which is at least about 100 nucleotide in length; (e) a sequence which is degenerate as a result of the genetic code to a sequence as defined in any one of (a), (b), (c) or (d); (f) a nucleotide sequence which is the reverse complement of a nucleotide sequence as defined in (a), (b), (c), (d) or (e), or encodes a polypeptide; and to the relating polypeptides.

A polypeptide according to the invention having 2,5-furan-dicarboxylic acid decarboxylase 1 activity comprises the amino acid sequence set out in SEQ ID NO: 19 or an amino acid sequence encoded by the nucleotide sequence of SEQ ID NO: 20 or a variant polypeptide thereof, wherein the variant has at least 54% sequence identity or more with the sequence set out in SEQ ID NO: 19.

An embodiment of the invention is a polynucleotide which comprises: (a) the nucleotide sequence set out in SEQ ID NO: 20; (b) a nucleotide sequence which hybridizes selectively with a polynucleotide being the reverse complement of SEQ ID NO: 20; (c) a nucleotide sequence having at least 66% sequence identity or more with the nucleotide sequence of SEQ ID NO: 20; (d) a fragment of a nucleotide sequence as defined in (a), (b) or (c) which is at least about 100 nucleotides in length; (e) a sequence which is degenerate as a result of the genetic code to a sequence as defined in any one of (a), (b), (c) or (d); (f)a nucleotide sequence which is the reverse complement of a nucleotide sequence as defined in (a), (b), (c), (d) or (e).

In one embodiment the variant nucleic acid molecule comprises a nucleotide sequence encoding a protein, wherein the variant nucleic acid molecule comprises a substantially homologous nucleotide sequence of at least 66%, 70%, 75%, 80%, 85%, 90%, 91%, 92%, 93%, 94%, 95%, 96%, 97%, 98%, 99% or more homologous to the nucleotide sequence shown in SEQ ID NO: 20.

In another embodiment the variant protein comprises a substantially homologous amino acid sequence of at least 54%, 55%, 60%, 65%, 70%, 75%, 80%, 85%, 90%, 91%, 92%, 93%, 94%, 95%, 96%, 97%, 98%, 99% or more homologous to the amino acid sequence shown in SEQ ID NO: 19.

A polypeptide according to the invention having 2,5-furan-dicarboxylic acid decarboxylase 2 activity comprises the amino acid sequence set out in SEQ ID NO: 21 or an amino acid sequence encoded by the nucleotide sequence of SEQ ID NO: 22 or a variant polypeptide thereof, wherein the variant has at least 52% sequence identity or more with the sequence set out in SEQ ID NO: 21.

An embodiment of the invention is a polynucleotide which comprises: (a) the nucleotide sequence set out in SEQ ID NO: 22; (b) a nucleotide sequence which hybridizes selectively with a polynucleotide being the reverse complement of SEQ ID NO: 22; (c) a nucleotide sequence having at least 67% sequence identity or more with the nucleotide sequence of SEQ ID NO: 22; (d) a fragment of a nucleotide sequence as defined in (a), (b) or (c) which is at least about 100 nucleotides in length; (e) a sequence which is degenerate as a result of the genetic code to a sequence as defined in any one of (a), (b), (c) or (d); (f) a nucleotide sequence which is the reverse complement of a nucleotide sequence as defined in (a), (b), (c), (d) or (e).

In one embodiment the variant nucleic acid molecule comprises a nucleotide sequence encoding a protein, wherein the variant nucleic acid molecule comprises a substantially homologous nucleotide sequence of at least 67%, 70%, 75%, 80%, 85%, 90%, 91%, 92%, 93%, 94%, 95%, 96%, 97%, 98%, 99% or more homologous to the nucleotide sequence shown in SEQ ID NO: 22.

In another embodiment the variant protein comprises a substantially homologous amino acid sequence of at least 52%, 55%, 60%, 65%, 70%, 75%, 80%, 85%, 90%, 91%, 92%, 93%, 94%, 95%, 96%, 97%, 98%, 99% or more homologous to the amino acid sequence shown in SEQ ID NO: 21.

A polypeptide according to the invention having HMF/furfural oxidoreductase activity comprises the amino acid sequence set out in SEQ ID NO: 25 or an amino acid sequence encoded by the nucleotide sequence of SEQ ID NO: 26 or a variant polypeptide thereof, wherein the variant has at least 45% sequence identity or more with the sequence set out in SEQ ID NO: 25.

An embodiment of the invention is a polynucleotide which comprises: (a) the nucleotide sequence set out in SEQ ID NO: 26; (b) a nucleotide sequence which hybridizes selectively with a polynucleotide being the reverse complement of SEQ ID NO: 26; (c) a nucleotide sequence having at least 66% sequence identity or more with the nucleotide sequence of SEQ ID NO: 26; (d) a fragment of a nucleotide sequence as defined in (a), (b) or (c) which is at least about 100 nucleotides in length; (e) a sequence which is degenerate as a result of the genetic code to a sequence as defined in any one of (a), (b), (c) or (d); (f) a nucleotide sequence which is the reverse complement of a nucleotide sequence as defined in (a), (b), (c), (d) or (e).

In one embodiment the variant nucleic acid molecules comprises a nucleotide sequence encoding a protein, wherein the variant nucleic acid molecule comprises a substantially homologous nucleotide sequence of at least 66%, 70%, 75%, 80%, 85%, 90%, 91%, 92%, 93%, 94%, 95%, 96%, 97%, 98%, 99% or more homologous to the nucleotide sequence shown in SEQ ID NO: 26.

In another embodiment the variant protein comprises a substantially homologous amino acid sequence of at least 45%, 50%, 55%, 60%, 65%, 70%, 75%, 80%, 85%, 90%, 91%, 92%, 93%, 94%, 95%, 96%, 97%, 98%, 99% or more homologous to the amino acid sequence shown in SEQ ID NO: 25.

A polypeptide according to the invention having fatty acid hydroxylase (hyd) activity comprises the amino acid sequence set out in SEQ ID NO: 27, or an amino acid sequence encoded by the nucleotide sequence of SEQ ID NO: 28 or a variant polypeptide thereof, wherein the variant has at least 66% sequence identity or more with the sequence set out in SEQ ID NO: 28.

An embodiment of the invention is a polynucleotide which comprises: (a) the nucleotide sequence set out in SEQ ID NO: 28; (b) a nucleotide sequence which hybridizes selectively with a polynucleotide being the reverse complement of SEQ ID NO: 28; (c) a nucleotides sequence having at least 66% sequence identity or more with the nucleotide sequence of SEQ ID NO: 28; (d) a fragment of a nucleotide sequence as defined in (a), (b) or (c) which is at least about 100 nucleotides in length; (e) a sequence which is degenerate as a result of the genetic code to a sequence as defined in any one of (a), (b), (c) or (d); (f) a nucleotide sequence which is the reverse complement of a nucleotide sequence as defined in (a), (b), (c), (d) or (e).

In one embodiment the variant nucleic acid molecule comprises a nucleotide sequence encoding a protein, wherein the variant nucleic acid molecule comprises a substantially homologous nucleotide sequence of at least 66%, 70%, 75%, 80%, 85%, 90%, 91%, 92%, 93%, 94%, 95%, 96%, 97%, 98%, 99% or more homologous to the nucleotide sequence shown in SEQ ID NO: 28.

In another embodiment the variant protein comprises a substantially homologous amino acid sequence of at least 31%, 33%, 35%, 40%, 45%, 50%, 55%, 60%, 65%, 70%, 75%, 80%, 85%, 90%, 91%, 92%, 93%, 94%, 95%, 96%, 97%, 98%, 99% or more homologous to the amino acid sequence shown in SEQ ID NO: 27.

A polypeptide according to the invention having truncated LysR-type transcriptional regulator LysR (hmfRt) activity activity comprises the amino acid sequence set out in SEQ ID NO: 29, or an amino acid sequence encoded by the nucleotide sequence of SEQ ID NO: 30 or a variant polypeptide thereof, wherein the variant has at least 65% sequence identity or more with the sequence set out in SEQ ID NO: 30.

An embodiment of the invention is a polynucleotide which comprises: (a) the nucleotide sequence set out in SEQ ID NO: 30; (b) a nucleotide sequence which hybridizes selectively with a polynucleotide being the reverse complement of SEQ ID NO: 30; (c) a nucleotide sequence having at least 85% sequence identity or more with the nucleotide sequence of SEQ ID NO: 30; (d) a fragment of a nucleotide sequence as defined in (a), (b) or (c) which is at least about 100 nucleotides in length; (e) a sequence which is degenerate as a result of the genetic code to a sequence as defined in any one of (a), (b), (c) or (d); (f) a nucleotide sequence which is the reverse complement of a nucleotide sequence as defined in (a), (b), (c), (d) or (e).

In one embodiment the variant nucleic acid molecule comprises a nucleotide sequence encoding a protein, wherein the variant nucleic acid molecule comprises a substantially homologous nucleotide sequence of at least 85%, 88%, 90%, 91%, 92%, 93%, 94%, 95%, 96%, 97%, 98%, 99% or more homologous to the nucleotide sequence shown in SEQ ID NO: 30.

In another embodiment the variant protein comprises a substantially homologous amino acid sequence of at least 37, 40%, 45%, 50%, 55%, 60%, 65%, 70%, 75%, 80%, 85%, 90%, 91%, 92%, 93%, 94%, 95%, 96%, 97%, 98%, 99% or more homologous to the amino acid sequence shown in SEQ ID NO: 29.

A polypeptide according to the invention having for major facilitator superfamily transporter putative furanic MFS-type transporter mfs1 activity comprises the amino acid sequence set out in SEQ ID NO: 31, or an amino acid sequence encoded by the nucleotide sequence of SEQ ID NO: 32 or a variant polypeptide thereof, wherein the variant has at least 79% sequence identity or more with the sequence set out in SEQ ID NO: 32. An embodiment of the invention is a polynucleotide which comprises: (a) the nucleotide sequence set out in SEQ ID NO: 32; (b) a nucleotide sequence which hybridizes selectively with a polynucleotide being the reverse complement of SEQ ID NO: 32; (c) a nucleotide sequence having at least 79% sequence identity or more with the nucleotide sequence of SEQ ID NO: 32; (d) a fragment of a nucleotide sequence as defined in (a), (b) or (c) which is at least about 100 nucleotide in length; (e) a sequence which is degenerate as a result of the genetic code to a sequence as defined in any one of (a), (b), (c) or (d); (f) a nucleotide sequence which is the reverse complement of a nucleotide sequence as defined in (a), (b), (c), (d) or (e).

In one embodiment the variant nucleic acid molecule comprises a nucleotide sequence encoding a protein, wherein the variant nucleic acid molecule comprises a substantially homologous nucleotide sequence of at least 79%, 80%, 85%, 90%, 91%, 92%, 93%, 94%, 95%, 96%, 97%, 98%, 99% or more homologous to the nucleotide sequence shown in SEQ ID NO: 32.

In another embodiment the variant protein comprises a substantially homologous amino acid sequence of at least 32%, 33%, 35%, 40%, 45%, 50%, 55%, 60%, 65%, 70%, 75%, 80%, 85%, 90%, 91%, 92%, 93%, 94%, 95%, 96%, 97%, 98%, 99% or more homologous to the amino acid sequence shown in SEQ ID NO: 31.

A polypeptide according to the invention having LysR type transcriptional regulator activity comprises the amino acid sequence set out in SEQ ID NO: 33 or an amino acid sequence encoded by the nucleotide sequence of SEQ ID NO: 34 or a variant polypeptide thereof, wherein the variant has at least 46% sequence identity or more with the sequence set out in SEQ ID NO: 33.

An embodiment of the invention is a polynucleotide which comprises: (a) the nucleotide sequence set out in SEQ ID NO: 34;(b) a nucleotide sequence which hybridizes selectively with a polynucleotide being the reverse complement of SEQ ID NO: 34;(c) a nucleotide sequence having at least 65% sequence identity or more with the nucleotide sequence of SEQ ID NO: 34; (d) a fragment of a nucleotide sequence as defined in (a), (b) or (c) which is at least about 100 nucleotides in length; (e) a sequence which is degenerate as a result of the genetic code to a sequence as defined in any one of (a), (b), (c) or (d); (f) a nucleotide sequence which is the reverse complement of a nucleotide sequence as defined in (a), (b), (c), (d) or (e).

In one embodiment the variant nucleic acid molecule comprises a nucleotide sequence encoding a protein, wherein the variant nucleic acid molecule comprises a substantially homologous nucleotide sequence of at least 65%, 70%, 75%, 80%, 85%, 90%, 91%, 92%, 93%, 94%, 95%, 96%, 97%, 98%, 99% or more homologous to the nucleotide sequence shown in SEQ ID NO: 34.

In another embodiment the variant protein comprises a substantially homologous amino acid sequence of at least 46%, 50%, 55%, 60%, 65%, 70%, 76%, 80%, 85%, 90%, 91%, 92%, 93%, 94%, 95%, 96%, 97%, 98%, 99% or more homologous to the amino acid sequence shown in SEQ ID NO: 33.

A polypeptide according to the invention having Furoyl-CoA dehydrogenase (large subunit) activity comprises the amino acid sequence set out in SEQ ID NO: 35 or an amino acid sequence encoded by the nucleotide sequence of SEQ ID NO: 36 or a variant polypeptide thereof, wherein the variant has at least 54% sequence identity or more with the sequence set out in SEQ ID NO: 35.

An embodiment of the invention is a polynucleotide which comprises: (a) the nucleotide sequence set out in SEQ ID NO: 36; (b) a nucleotide sequence which hybridizes selectively with a polynucleotide being the reverse complement of SEQ ID NO: 36; (c) a nucleotide sequence having at least 66% sequence identity or more with the nucleotide sequence of SEQ ID NO: 36; (d) a fragment of a nucleotides sequence as defined in (a), (b) or (c) which is at least about 100 nucleotides in length; (e) a sequence which is degenerate as a result of the genetic code to a sequence as defined in any one of (a), (b), (c) or (d); (f) a nucleotide sequence which is the reverse complement of a nucleotide sequence as defined in (a), (b), (c), (d) or (e).

In one embodiment the variant nucleic acid molecule comprises a nucleotide sequence encoding a protein, wherein the variant nucleic acid molecule comprises a substantially homologous nucleotide sequence of at least 66%, 70%, 75%, 80%, 85%, 90%, 91%, 92%, 93%, 94%, 95%, 96%, 97%, 98%, 99% or more homologous to the nucleotide sequence shown in SEQ ID NO: 36.

In another embodiment the variant protein comprises a substantially homologous amino acid sequence of at least 54%, 55%, 60%, 65%, 70%, 75%, 80%, 85%, 90%, 91%, 92%, 93%, 94%, 95%, 96%, 97%, 98%, 99% or more homologous to the amino acid sequence shown in SEQ ID NO: 35.

A polypeptide according to the invention having Furoyl-CoA dehydrogenase FAD binding subunit activity which comprises the amino acid sequence set out in SEQ ID NO: 37 or an amino acid sequence encoded by the nucleotide sequence of SEQ ID NO: 38 or a variant polypeptide thereof, wherein the variant has at least 49% sequence identity or more with the sequence set out in SEQ ID NO: 37.

An embodiment of the invention is a polynucleotide which comprises: (a) the nucleotide sequence set out in SEQ ID NO: 38; (b) a nucleotide sequence which hybridizes selectively with a polynucleotide being the reverse complement of SEQ ID NO: 38; (c) a nucleotide sequence having at least 71% sequence identity or more with the nucleotide sequence of SEQ ID NO: 38; (d) a fragment of a nucleotide sequence as defined in (a), (b) or (c) which is at least about 100 nucleotides in length; (e) a sequence which is degenerate as a result of the genetic code to a sequence as defined in any one of (a), (b), (c) or (d); (f) a nucleotide sequence which is the reverse complement of a nucleotide sequence as defined in (a), (b), (c), (d) or (e).

In one embodiment the variant nucleic acid molecule comprises a nucleotide sequence encoding a protein, wherein the variant nucleic acid molecule comprises a substantially homologous nucleotide sequence of at least 71%, 75%, 80%, 85%, 90%, 91%, 92%, 93%, 94%, 95%, 96%, 97%, 98%, 99% or more homologous to the nucleotide sequence shown in SEQ ID NO: 38.

In another embodiment the variant protein comprises a substantially homologous amino acid sequence of at least 49%, 50%, 55%, 58%, 60%, 65%, 70%, 75%, 80%, 85%, 90%, 91%, 92%, 93%, 94%, 95%, 96%, 97%, 98%, 99% or more homologous to the amino acid sequence shown in SEQ ID NO: 37.

A polypeptide according to the invention having Furoyl-CoA dehydrogenase 2Fe-2S iron sulfur subunit activity which comprises the amino acid sequence set out in SEQ ID NO: 39 or an amino acid sequence encoded by the nucleotide sequence of SEQ ID NO: 40 or a variant polypeptide thereof, wherein the variant has at least 64% sequence identity or more with the sequence set out in SEQ ID NO: 39.

An embodiment of the invention is a polynucleotide which comprises: (a) the nucleotide sequence set out in SEQ ID NO: 40; (b) a nucleotide sequence which hybridizes selectively with a polynucleotide being the reverse complement of SEQ ID NO: 40; (c) a nucleotide sequence having at least 70% sequence identity or more with the nucleotide sequence of SEQ ID NO: 40; (d) a fragment of a nucleotide sequence as defined in (a), (b) or (c) which is at least about 100 nucleotides in length; (e) a sequence which is degenerate as a result of the genetic code to a sequence as defined in any one of (a), (b), (c) or (d); (f) a nucleotide sequence which is the reverse complement of a nucleotide sequence as defined in (a), (b), (c), (d) or (e).

In one embodiment the variant nucleic acid molecule comprises a nucleotide sequence encoding a protein, wherein the variant nucleic acid molecule comprises a substantially homologous nucleotide sequence of at least 70%, 75%, 80%, 85%, 90%, 91%, 92%, 93%, 94%, 95%, 96%, 97%, 98%, 99% or more homologous to the nucleotide sequence shown in SEQ ID NO: 40.

In another embodiment the variant protein comprises a substantially homologous amino acid sequence of at least 64%, 65%, 70%, 75%, 80%, 85%, 90%, 91%, 92%, 93%, 94%, 95%, 96%, 97%, 98%, 99% or more homologous to the amino acid sequence shown in SEQ ID NO: 39.

A polypeptide according to the invention having Furoyl-CoA synthetase activity which comprises the amino acid sequence set out in SEQ ID NO: 41 or an amino acid sequence encoded by the nucleotide sequence of SEQ ID NO: 42 or a variant polypeptide thereof, wherein the variant has at least 57% sequence identity or more with the sequence set out in SEQ ID NO: 41.

An embodiment of the invention is a polynucleotide which comprises: (a) the nucleotide sequence set out in SEQ ID NO: 42; (b) a nucleotide sequence which hybridizes selectively with a polynucleotide being the reverse complement of SEQ ID NO: 42; (c) a nucleotides sequence having at least 68% sequence identity or more with the nucleotide sequence of SEQ ID NO: 42; (d) a fragment of a nucleotide sequence as defined in (a), (b) or (c) which is at least about 100 nucleotides in length; (e) a sequence which is degenerate as a result of the genetic code to a sequence as defined in any one of (a), (b), (c) or (d); (f) a nucleotide sequence which is the reverse complement of a nucleotides sequence as defined in (a), (b), (c), (d) or (e).

In one embodiment the variant nucleic acid molecule comprises a nucleotide sequence encoding a protein, wherein the variant nucleic acid molecule comprises a substantially homologous nucleotide sequence of at least 68%, 70%, 75%, 80%, 85%, 90%, 91%, 92%, 93%, 94%, 95%, 96%, 97%, 98%, 99% or more homologous to the nucleotide sequence shown in SEQ ID NO: 42.

In another embodiment the variant protein comprises a substantially homologous amino acid sequence of at least 57%, 60%, 65%, 70%, 75%, 80%, 85%, 90%, 91%, 92%, 93%, 94%, 95%, 96%, 97%, 98%, 99% or more homologous to the amino acid sequence shown in SEQ ID NO: 41.

A polypeptide according to the invention having 2-oxoglutaroyl-CoA hydrolase activity comprises the amino acid sequence set out in SEQ ID NO: 43 or an amino acid sequence encoded by the nucleotide sequence of SEQ ID NO: 44 or a variant polypeptide thereof, wherein the variant has at least 72% sequence identity or more with the sequence set out in SEQ ID NO: 44.

An embodiment of the invention is a polynucleotide which comprises: (a) the nucleotide sequence set out in SEQ ID NO: 44; (b) a nucleotide sequence which hybridizes selectively with a polynucleotide being the reverse complement of SEQ ID NO: 44; (c) a nucleotide sequence having at least 74% sequence identity or more with the nucleotide sequence of SEQ ID NO: 44; (d) a fragment of a nucleotide sequence as defined in (a), (b) or (c) which is at least about 100 nucleotides in length; (e) a sequence which is degenerate as a result of the genetic code to a sequence as defined in any one of (a), (b), (c) or (d); (f) a nucleotide sequence which is the reverse complement of a nucleotide sequence as defined in (a), (b), (c), (d) or (e) .

In one embodiment the variant nucleic acid molecule comprises a nucleotide sequence encoding a protein, wherein the variant nucleic acid molecule comprises a substantially homologous nucleotide sequence of at least 74%, 75%, 80%, 85%, 90%, 91%, 92%, 93%, 94%, 95%, 96%, 97%, 98%, 99% or more homologous to the nucleotide sequence shown in SEQ ID NO: 44.

In another embodiment the variant protein comprises a substantially homologous amino acid sequence of at least 72%, 75%, 80%, 85%, 90%, 91%, 92%, 93%, 94%, 95%, 96%, 97%, 98%, 99% or more homologous to the amino acid sequence shown in SEQ ID NO: 43.

A polypeptide according to the invention having putative furanic MFS-type transporter (major facilitator superfamily transporter mfs2) activity comprises the amino acid sequence set out in SEQ ID NO: 45, or an amino acid sequence encoded by the nucleotide sequence of SEQ ID NO: 46 or a variant polypeptide thereof, wherein the variant has at least 29% sequence identity or more with the sequence set out in SEQ ID NO: 46. An embodiment of the invention is a polynucleotide which comprises: (a) the nucleotide sequence set out in SEQ ID NO: 46; (b) a nucleotide sequence which hybridizes selectively with a polynucleotide being the reverse complement of SEQ ID NO: 46; (c) a nucleotide sequence having at least 79% sequence identity or more with the nucleotides sequence of SEQ ID NO: 46; (d) a fragment of a nucleotide sequence as defined in (a), (b) or (c) which is at least about 100 nucleotides in length; (e) a sequence which is degenerate as a result of the genetic code to a sequence as defined in any one of (a), (b), (c) or (d); (f) a nucleotide sequence which is the reverse complement of a nucleotide sequence as defined in (a), (b), (c), (d) or (e).

In one embodiment the variant nucleic acid molecule comprises a nucleotide sequence encoding a protein, wherein the variant nucleic acid molecule comprises a substantially homologous nucleotide sequence of at least 79%, 80%, 85%, 90%, 91%, 92%, 93%, 94%, 95%, 96%, 97%, 98%, 99% or more homologous to the nucleotide sequence shown in SEQ ID NO: 46.

In another embodiment the variant protein comprises a substantially homologous amino acid sequence of at least 29%, 30%, 35%, 40%, 45%, 50% 55%, 60%, 65%, 70%, 75%, 80%, 85%, 90%, 91%, 92%, 93%, 94%, 95%, 96%, 97%, 98%, 99% or more homologous to the nucleotide sequence shown in SEQ ID NO: 45.

A further embodiment is a vector incorporating the polynucleotide sequences or a nucleic acid constructs set out above.

The terms "homology", "sequence identity" and the like are used interchangeably herein. For the purpose of this invention, it is defined herein that in order to determine the degree of sequence identity shared by two amino acid sequences or by two nucleic acid sequences, the sequences are aligned for optimal comparison purposes (e.g., gaps can be introduced in the sequence of a first amino acid or nucleic acid sequence for optimal alignment with a second amino or nucleic acid sequence). Such alignment may be carried out over the full lengths of the sequences being compared. Alternatively, the alignment may be carried out over a shorter comparison length, for example over about 20, about 50, about 100 or more nucleic acids/bases or amino acids. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The degree of identity shared between sequences is typically expressed in term of percentage identity between the two sequences and is a function of the number of identical positions shared by the sequences (i.e., % identity = number of identical positions/total number of positions (i.e. overlapping positions) x 100). Preferably, the two sequences being compared are of the same or substantially the same length.

The skilled person will be aware of the fact that several different computer programs are available to determine the homology between two sequences. For instance, a comparison of sequences and determination of percent identity between two sequences can be accomplished using a mathematical algorithm. In a preferred embodiment, the percentage identity between two amino acid sequences is determined using the Needleman and Wunsch (J. Mol. Biol. (48): 444-453 (1970)) algorithm which has been incorporated into the GAP program in the Accelrys GCG software package (available at http://www.accelrys.com/products/gcg/), using either a Blossom 62 matrix or a PAM250 matrix, and a gap weight of 16, 14, 12, 10, 8, 6, or 4 and a length weight of 1, 2, 3, 4, 5, or 6. The skilled person will appreciate that all these different parameters will yield slightly different results but that the overall percentage identity of two sequences is not significantly altered when using different algorithms.

In yet another embodiment, the percent identity between two nucleotide sequences is determined using the GAP program in the Accelrys GCG software package (available at http://www.accelrys.com/products/gcg/), using a NWSgapdna.CMP matrix and a gap weight of 40, 50, 60, 70, or 80 and a length weight of 1, 2, 3, 4, 5, or 6. In another embodiment, the percent identity two amino acid or nucleotide sequence is determined using the algorithm of E. Meyers and W. Miller (CABIOS, 4:11-17 (1989) which has been incorporated into the ALIGN program (version 2.0) (available at the ALIGN Query using sequence data of the Genestream server IGH Montpellier France http://vega.igh.cnrs.fr/bin/align-guess.cgi) using a PAM120 weight residue table, a gap length penalty of 12 and a gap penalty of 4.

The nucleic acid and protein sequences of the present invention can further be used as a "query sequence" to perform a search against public databases to, for example, identify other family members or related sequences. Such searches can be performed using the NBLAST and XBLAST programs (version 2.0) of Altschul, et al. (1990) J. Mol. Biol. 215:403-10. BLAST nucleotide searches can be performed with the NBLAST program, score = 100, wordlength = 12 to obtain nucleotide sequences for instance homologous to oxidoreductase encoding nucleic acid molecules of the invention. BLAST protein searches can be performed with the XBLAST program, score = 50, wordlength = 3 to obtain amino acid sequences homologous to oxidoreductase protein molecules of the invention. To obtain gapped alignments for comparison purposes, Gapped BLAST can be utilized as described in Altschul et al., (1997) Nucleic Acids Res. 25(17): 3389-3402. When utilizing BLAST and Gapped BLAST programs, the default parameters of the respective programs (e.g., XBLAST and NBLAST, also known as BLASTn and BLASTx) can be used. See the homepage of the National Center for Biotechnology Information at http://www.ncbi.nlm.nih.gov/.

As used herein, the term "selectively hybridizing", "hybridizes selectively" and similar terms are intended to describe conditions for hybridization and washing under which nucleotide sequences at least at least 70%, at least 75%, at least 80%, more preferably at least 85%, even more preferably at least 90%, preferably at least 95%, more preferably at least 98% or more preferably at least 99% homologous to each other typically remain hybridized to each other. That is to say, such hybridizing sequences may share at least at least 70%, at least 75%, at least 80%, more preferably at least 85%, even more preferably at least 90%, more preferably at least 95%, more preferably at least 98% or more preferably at least 99% sequence identity.

A preferred, non-limiting example of such hybridization conditions is hybridization in 6X sodium chloride/sodium citrate (SSC) at about 45oC, followed by one or more washes in 1 X SSC, 0.1% SDS at about 50oC, preferably at about 55oC, preferably at about 60oC and even more preferably at about 65oC.

Highly stringent conditions include, for example, hybridization at about 68oC in 5x SSC/5x Denhardt's solution / 1.0% SDS and washing in 0.2x SSC/0.1% SDS at room temperature. Alternatively, washing may be performed at 42°C.

The skilled artisan will know which conditions to apply for stringent and highly stringent hybridization conditions. Additional guidance regarding such conditions is readily available in the art, for example, in Sambrook et al., 1989, Molecular Cloning, A Laboratory Manual, Cold Spring Harbor Press, N.Y.; and Ausubel et al. (eds.), 1995, Current Protocols in Molecular Biology, (John Wiley & Sons, N.Y.).

Of course, a polynucleotide which hybridizes only to a poly A sequence (such as the 3' terminal poly(A) tract of mRNAs), or to a complementary stretch of T (or U) resides, would not be included in a polynucleotide of the invention used to specifically hybridize to a portion of a nucleic acid of the invention, since such a polynucleotide would hybridize to any nucleic acid molecule containing a poly (A) stretch or the complement thereof (e.g., practically any double-standed cDNA clone).

In a typical approach, gene libraries constructed from other organisms, e.g. a bacterium , in particular from the micro-organism family Trichomaceae, for example from the genus Burkholderia can be screened such as Burkholderia phytofirmans.

For example, Burkholderia strains can be screened for homologous encoding polynucleotides according to the invention by Southern blot analysis. Upon detection of homologous DNA restriction fragments according to the invention, gene libraries can be constructed from chromosomal fragments of the same size from the appropriate strain, utilizing standard techniques well known to those of skill in the art. Alternatively, if the microorganism is a eukaryote, the mRNA transcript of the respective genes according to the invention can be identified by Northen hybridization and upon identification of the transcript, cDNA libraries can be prepared using total RNA isolated from the eukaryotic microorganism. Homologous gene sequences can be isolated, for example, by performing PCR using two degenerate oligonucleotide primer pools designed on the basis of nucleotide sequences as taught herein. The template for the reaction can be total chromosomal DNA from the strain know or suspected to express a polynucleotide according to the invertion. The PCR product can be subcloned and sequenced to ensure that the amplified sequences represent the sequences of a new oxidoreductase nucleic acid sequence, or a functional equivalent thereof.

Alternatively the template for the reaction can be cDNA obtained by reverse transcription of mRNA prepared from strains known or suspected to express a polynucleotide according to the invention. The PCR product can be subcloned and sequenced to ensure that the amplified sequences represent the sequences of a new oxidoreductase nucleic acid sequence, or a functional equivalent thereof.

The PCR fragment can then be used to isolate a full-length cDNA clone by a variety of known methods. For example, the amplified fragment can be labeled and used to screen a bacteriophage or cosmid cDNA library. Alternatively, the labeled fragment can be used to screen a genomic library.

PCR technology also can be used to isolate full-length cDNA sequences from other organisms. For example, RNA can be isolated, following standard procedures, from an appropriate cellular or tissue source. A reverse transcription reaction can be performed on the RNA using an oligonucleotide primer specific for the most 5' end of the amplified fragment for the priming of first strand synthesis.

The resulting RNA/DNA hybrid can then be "tailed" (e.g., with guanines) using a standard terminal transferase reaction, the hybrid can be digested with RNase H, and second strand synthesis can then be primed (e.g., with a poly-C primer). Thus, cDNA sequences upstream of the amplified fragment can easily be isolated. For a review of useful cloning strategies, see e.g.,Sambrook et al., supra; and Ausubel et al., supra.

Another aspect of the invention pertains to vectors, including cloning and expression vectors, comprising a polynucleotide of the invention encoding a oxidoreductase protein or a functional equivalent thereof and methods of growing, transforming or transfecting such vectors in a suitable host cell, for example under conditions in which expression of a polypeptide of the invention occurs. As used herein, the term "vector" refers to a nucleic acid molecule capable of transporting another nucleic acid to which it has been linked.

Polynucleotides of the invention can be incorporated into a recombinant replicable vector, for example a cloning or expression vector. The vector may be used to replicate the nucleic acid in a compatible host cell. Thus in a further embodiment, the invention provides a method of making polynucleotides of the invention by introducing a polynucleotide of the invention into a replicable vector, introducing the vector into a compatible host cell, and growing the host cell under conditions which bring about replication of the vector. The vector may be recovered from the host cell. Suitable host cells are described below.

The vector into which the expression cassette or polynucleotide of the invention is inserted may be any vector which may conveniently be subjected to recombinant DNA procedures, and the choice of the vector will often depend on the host cell into which it is to be introduced.

A vector according to the invention may be an autonomously replicating vector, i. e. a vector which exists as an extra-chromosomal entity, the replication of which is independent of chromosomal replication, e.g. a plasmid. Alternatively, the vector may be one which, when introduced into a host cell, is integrated into the host cell genome and replicated together with the chromosome (s) into which it has been integrated.

One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors are capable of directing the expression of genes to which they are operatively linked. Such vectors are referred to herein as "expression vectors". In general, expression vectors of utility in recombinant DNA techniques are often in the form of plasmids. The terms "plasmid," and "vector" can be used interchangeably herein as the plasmid is the most commonly used form of vector. However, the invention is intended to include such other forms of expression vectors, such as cosmid, viral vectors (e.g., replication defective retroviruses, adenoviruses and adeno-associated viruses) and phage vectors which serve equivalent functions.

Vectors according to the invention may be used in vitro, for example for the production of RNA or used to transfect or transform a host cell.

A vector of the invention may comprise two or more, for example three, four or five, polynucleotides of the invention, for example for overexpression.

The Recombinant expression vectors of the invention comprise a nucleic acid of the invention in a form suitable for expression of the nucleic acid in a host cell, which means that the recombinant expression vector includes one or more regulatory sequences, selected on the basis of the host cells to be used for expression, which is operably linked to the nucleic acid sequence to be expressed.

Within a vector, such as an expression vector, "operably linked" is intended to mean that the nucleotide sequence of interest is linked to the regulatory sequence(s) in a manner which allows for expression of the nucleotide sequence (e.g., in an in vitro transcription/translation system or in a host cell when the vector is introduced into the host cell), i.e. the term "operably linked" refers to a juxtaposition wherein the components described are in a relationship permitting them to function in their intended manner. A regulatory sequence such as a promoter, enhancer or other expression regulation signal "operably linked" to a coding sequence is positioned in such a way that expression of the coding sequence is achieved under condition compatible with the control sequences or the sequences are arranged so that they function in concert for their intended purpose, for example transcription initiates at a promoter and proceeds through the DNA sequence encoding the polypeptide.

The term "regulatory sequence or "control sequence" is intended to include promoters, enhancers and other expression control elements (e.g., polyadenylation signal). Such regulatory sequences are described, for example, in Goeddel; Gene Expression Technology: Methods in Enzymology 185, Academic Press, San Diego, CA (1990).

The term regulatory or control sequences includes those sequences which direct constitutive expression of a nucleotide sequence in many types of host cells and those which direct expression of the nucleotide sequence only in a certain host cell (e.g. tissue-specific regulatory sequences).

A vector or expression construct for a given host cell may thus comprise the following elements operably linked to each other in a consecutive order from the 5'-end to 3'-end relative to the coding strand of the sequence encoding the polypeptide of the first invention: (1) a promoter sequence capable of directing transcription of the nucleotide sequence encoding the polypeptide in the given host cell ; (2) optionally, a signal sequence capable of directing secretion of the polypeptide from the given host cell into a culture medium; (3) a DNA sequence of the invention encoding a mature and preferably active form of a polypeptide having cellobiohydrolase activity; and preferably also (4) a transcription termination region (terminator) capable of terminating transcription downstream of the nucleotide sequence encoding the polypeptide.

Downstream of the nucleotide sequence according to the invention there may be a 3' untranslated region containing one or more transcription termination sites (e.g. a terminator). The origin of the terminator is less critical. The terminator can, for example, be native to the DNA sequence encoding the polypeptide. However, preferably a yeast terminator is used in yeast host cells and a filamentous fungal terminator is used in filamentous fungal host cells. More preferably, the terminator is endogenous to the host cell (in which the nucleotide sequence encoding the polypeptide is to be expressed). In the transcribed region, a ribosome binding site for translation may be present. The coding portion of the mature transcripts expressed by the constructs will include a translation initiating AUG at the beginning and a termination codon appropriately positioned at the end of the polypeptide to be translated.

Enhanced expression of the polynucleotide of the invention may also be achieved by the selection of heterologous regulatory regions, e. g. promoter, secretion leader and/or terminator regions, which may serve to increase expression and, if desired, secretion levels of the protein of interest from the expression host and/or to provide for the inducible control of the expression of a polypeptide of the invention.

It will be appreciated by those skilled in the art that the design of the expression vector can depend on such factors as the choice of the host cell to be transformed, the level of expression of protein desired, etc. The vectors, such as expression vectors, of the invention can be introduced into host cells to thereby produce proteins or peptides, encoded by nucleic acids as described herein (e.g. in the case of hmfH, oxidoreductase proteins, mutant forms of oxidoreductase proteins, fragments, variants or functional equivalents thereof, fusion proteins, etc.). The same applies to the other polypeptides according to the invention.

The vectors, such as recombinant expression vectors, of the invention can be designed for expression of suitable proteins in prokaryotic or eukaryotic cells. For example oxidoreductase proteins can be expressed in bacterial cells such as E. coli, insect cells (using baculovirus expression vectors), filamentous fungi, yeast cells or mammalian cells. Suitable host cells are discussed further in Goeddel, Gene Expression Technology: Methods in Enzymology 185, Academic Press, San Diego, CA (1990). Representative examples of appropriate hosts are described hereafter. Appropriate culture mediums and conditions for the above-described host cells are known in the art.

As set out above, the term "control sequences" or "regulatory sequences" is defined herein to include at least any component which may be necessary and/or advantageous for the expression of a polypeptide. Any control sequence may be native or foreign to the nucleic acid sequence of the invention encoding a polypeptide. Such control sequences may include, but are not limited to, a promoter, a leader, optimal translation initiation sequences (as described in Kozak, 1991, J. Biol. Chem. 266:19867-19870), a secretion signal sequence, a pro-peptide sequence, a polyadenylation sequence, a transcription terminator. At a minimum, the control sequences typically include a promoter, and transcriptional and translational stop signals.

A stably transformed microorganism is one that has had one or more DNA fragments introduced such that the introduced molecules are maintained, replicated and segregated in a growing culture. Stable transformation may be due to multiple or single chromosomal integration (s) or by (an) extrachromosomal element(s) such as (a) plasmid vector(s). A plasmid vector is capable of directing the expression of polypeptides encoded by particular DNA fragments. Expression may be constitutive or regulated by inducible (or repressible) promoters that enable high levels of transcription of functionally associated DNA fragments encoding specific polypeptides.

Isolation of the polypeptides according to the invention: one or more polypeptides according to the invention or DNA material expressing the polypeptides according to the invention may be isolated from an organism, preferably a microorganism that expresses the oxidoreductase.

Preferably, the microorganism is capable of using furanic compounds, preferably HMF and/or furufal as a substrate, more preferably not using other carbon sources such as C5 and/or C6 sugars. The microorganism preferably is chosen from the group consisting of: Cupriavidus (preferably Cupriavidus basilensis, Cupriavidus Eutropha and/or Cupriavidus basilensis HMF14,), Burkholderia (prefereably Burkholderia phytofirmans and/or Burkholderia phytofirmans PsJN), Bradyhrizobium (preferably , Bradyhrizobium japonicum, and/or Bradyhrizobium japonicum USDA110), Methylobacterium (preferably Methylobacterium radiotolerans and/or Methylobacterium radiotolerans JCM2831).

Most preferred polypeptides useful in the present invention are converting furfural and/or HMF as substrate, and are polypeptides according to the invention isolated from Cupriavidus basilensis HMF 14 herein, deposited in accordance with the Budapest Treaty on International Recognition of the Deposits of Microorganisms for the Purpose of Patent Procedures at the Deutsche Sammlung von Mikroorganismen und Zellkulturen GmbH (German Collection of Microorganisms and Cell Cultures), Inhoffenstraße 7B, 38124 Braunschweig, GERMANY as strain HMF 14, having deposit number DSM 22875; deposition date of August 19, 2009.

The present invention thus also relates to the isolated HMF-utilizing bacterium, Cupriavidus basilensis strain HMF14, and its genes involved in the HMF degradative pathway.

One of the genes (herein defined as hmfH) encodes a 579-amino acid, 62 kDa FAD-dependent oxidoreductase that was found to oxidize furfuryl alcohol, furfural, HMF, and 5-hydroxymethyl-furoic acid.

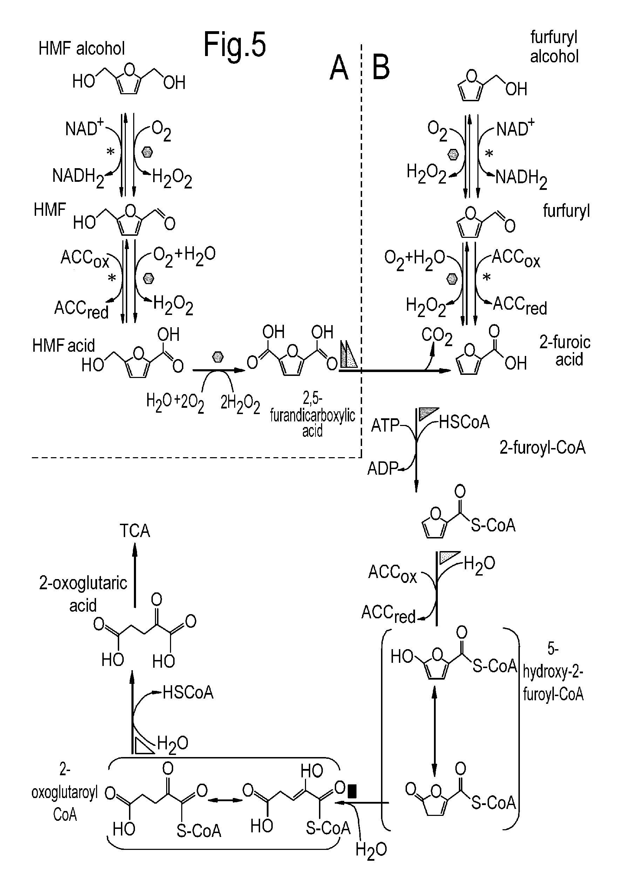

The alcohol / aldehyde groups at C2 and C5 in these molecules are oxidized, to yield i.e. furan-dicarboxylic acid from HMF, respectively, and 5-furoic acid from furfurylalcohol or furfural (see Figure 5).

The present invention thus provides polynucleotides encoding polypeptides, having the following activity: Furoyl-CoA dehydrogenase large subunit; a Furoyl-CoA dehydrogenase FAD binding subunit; Furoyl-CoA dehydrogenase 2Fe-2S iron sulfur subunit; a Furoyl-CoA synthetase; a 2-oxoglutaroyl-CoA hydrolase; a 2,5-furan-dicarboxylic acid decarboxylase 1, a 2,5-furan-dicarboxylic acid decarboxylase 2; a HMF/furfural oxidoreductase; a LysR type transcriptional regulator 1 and a LysR type transcriptional regulator 2, an aldehyde dehyrogenase and major family supertransporters 1 and 2.

Enzymes are herein understood as a subclass of polypeptides. A preferred furanic-compound-removing pathway useful in the present invention uses a novel Furoyl-CoA dehydrogenase, a novel Furoyl-CoA synthetase, a novel 2-oxoglutaroyl-CoA hydrolase, two novel 2,5-furan-dicarboxylic acid decarboxylases (1 and 2), and/or a novel HMF/furfural oxidoreductase isolated from Cupriavidus basilensis HMF 14 herein, deposited in accordance with the Budapest Treaty on International Recognition of the Deposits of Microorganisms for the Purpose of Patent Procedures as Deposit number DSM 22875.

Lignocellulose hydrolysate is the result of subjecting lignocellulosic material to a pre-treatment step. Suitable lignocellulose-containing material according to the subject invention includes, but is not limited to wood such as wood chips, saw dust; municipal waste containing lignocellulose; waste paper pulp; perennial grasses such as switchgrass (panicum virgatum); miscanthus species such as miscanthus x giganteus, miscanthus sinensis and miscanthus sacchariflorus; energy cane, sugar cane; sweet sorghum; corn cobs and corn stovers, wheat straw, rice straw and other sources of lignocellulosic material.

Lignocellulose is one of the most abundant plant material resources in the world. However, an effective pretreatment is needed to remove the rigid crystalline structure, enforced by lignin and hemicellulose to render it accessible for a subsequent hydrolysis step.

The pretreatment preferably includes both physical and chemical pretreatment steps. Physical pretreatment is often called size reduction to reduce biomass physical size. Chemical pretreatment is to remove chemical barriers so that the enzymes can access cellulose degradation.

Currently used pretreatment techniques include acid hydrolysis, steam explosion, ammonia fiber expansion, organosolve and sulfite pretreatmet, alkaline wet oxidation and ozone pretreatment.

Most pretreatment techniques result in the formation of degradation products that have inhibitory effects on subsequent hydrolysis and fermentation processes. The presence of inhibitors will not only further complicate the ethanol production but also increase the cost of production due to entailed detoxification steps.

The method most often applied includes acid hydrolysis, where the lignocellulosic material is subjected to an acid such as sulphuric acid, whereby the sugar polymers cellulose and hemicellulose are partly or completely hydrolysed to their constituent sugar monomers. Another type of lignocellulose hydrolysis is steam explosion, a process comprising heating of the lignocellulosic material by steam injection to a temperature of 190-230°C. A third method is wet oxidation wherein the material is treated with oxygen at 150-185°C. All methods may also be combined, e.g. steam explosion or oxidation in presence of acids. These pretreatments may be followed by enzymatic hydrolysis to complete the release of sugar monomers. The pretreatment steps results in the hydrolysis of cellulose into glucose while hemicellulose is transformed into the pentoses xylose and arabinose and the hexoses glucose, galactose and mannose.

The pretreatment step may in certain embodiments be supplemented with treatment resulting in further hydrolysis of the cellulose and hemicellulose. The purpose of such an additional hydrolysis treatment is to hydrolyse oligosaccharide and possibly polysaccharide species produced during the acid hydrolysis, wet oxidation, or steam explosion of cellulose and/or hemicellulose origin to form fermentable sugars (e.g. glucose, xylose and possibly other monosaccharides). Such further treatments may be either chemical or enzymatic. Chemical hydrolysis is typically achieved by treatment with an acid, such as treatment with aqueous sulphuric acid or formic acid, at a temperature in the range of about 100-150°C. Enzymatic hydrolysis is typically performed by treatment with one or more appropriate carbohydrase enzymes such as cellulases, glucosidases and hemicellulases including xylanases.

### Detailed description of the invention

Without wishing to be bound to any particular theory, it is believed that the inhibitory effect of lignocellulosic hydrolysate on fermentation comes from a concerted effect of many toxic constituents, but that furanic derivatives have a key role in this effect. It has been surprisingly found that C. basilensis HMF14 according to the invention is able to selectively degrade furanic derivatives in lignocellulosic hydrolysate, while essentially not utilizing fermentable sugars.

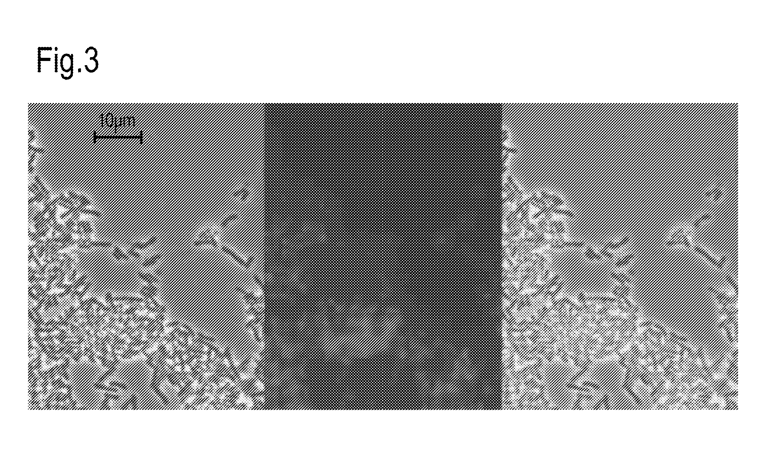

C. basilensis HMF14 was isolated on HMF, however it was found to also utilize furfural as sole carbon source. As a further beneficial effect, C. basilensis HMF14 is also able to remove the majority of non-furanic inhibitory compounds, such as acetate and formate, as illustrated in figure 3, which shows that the furanic derivatives, acetate and formate were completely removed from wheat straw hydrolysate after only ten hours of cultivation. During this period, the glucose, xylose and arabinose concentrations, i.e. fermentable sugars in lignocellulose hydrolysate, were stable. Only when the incubation was prolonged after these inhibitor compounds were consumed, the sugar concentration decreased by approximately 11 % within 15 h. Without wishing to be bound to any particular theory, it is believed that this may indicate that the sugars are not converted, but absorbed in the mucous layer around the bacterial cells.

Accrordingly, the ability to digest or convert furanic compounds without use of fermentable sugars is defined as the reduction of the inhibitor concentration while maintaining a stable fermentable sugar concentration before all furanic compounds are converted. The term "all furanic compounds are converted" refers to a concentration of furanic compounds of less than 1500 ppmw, more preferably less than 1000 ppmw, more preferably less than 500 ppmw, and yet more preferably less than 150 ppmw. Undiluted wheat straw hydrolysate was detoxified to completion as well, although an extended lag phase occurred that could be ameliorated by increasing the inoculum density.

As set out above, C. basilensis HMF14 was demonstrated to metabolize individual inhibitors in minimal medium. In addition to individual compounds, complex mixtures of toxic inhibitors were also efficiently metabolized as demonstrated by the detoxification of actual wheat straw hydrolysate. Treatment of lignocellulosic hydrolysate with C. basilensis HMF14 resulted in a solution of glucose, xylose and arabinose that is essentially free from furan aldehydes, acetate and formate. The unique substrate profile of C. basilensis HMF14 according to the invention makes this bacterium ideally suited for biological detoxification of lignocellulosic hydrolysate. Accordingly, the present invention further provides for an isolated culture of a Cupriavidus microorganism of Cupriavidus basilensis strain Deposit number DSM 22875 which, when provided with furanic compounds derivatives, preferably HMF and Furfural-derived compounds as a sole carbon source, grows on said source and expresses several novel enzymes, which act synergetically, comprising a novel 2-furoyl-CoA:acceptor 5-oxidoreductase (hydroxylating) FC 1.3.99.8), further referred to as Furoyl-CoA dehydrogenase, a novel Furoyl-CoA synthetase, a novel 2-oxoglutaroyl-CoA hydrolase, two novel 2,5-furan-dicarboxylic acid decarboxylases , and a novel HMF/furfural oxidoreductase.

An embodiment of the present invention is a vector incorporating the polynucleotide sequences or nucleic acid constructs set out herein-above, wherein the nucleotide sequences encode one or more of Furoyl-CoA dehydrogenase, Furoyl-CoA synthetase, 2-oxoglutaroyl-CoA hydrolase, 2,5-furan-dicarboxylic acid decarboxylase 1, 2,5-furan-dicarboxylic acid decarboxylase 2 and a HMF/furfural oxidoreductase from Cupriavidus basilensis HMF14 DSM 22875 in a host cell under conditions conducive for their expression.

The invention also relates to a host microorganism transformed or transfected by the isolated DNA or by a vector or plasmid comprising the isolated DNA according to the invention under conditions conducive to express one or more of a Furoyl-CoA dehydrogenase, a Furoyl-CoA synthetase, a 2-oxoglutaroyl-CoA hydrolase, a 2,5-furan-dicarboxylic acid decarboxylase 1, a 2,5-furan-dicarboxylic acid decarboxylase 2 and a HMF/furfural oxidoreductase.

The present invention also provides for a cell extract from a Cupriavidus microorganism or a host cell comprising one or more of a Furoyl-CoA dehydrogenase, a Furoyl-CoA synthetase, a 2-oxoglutaroyl-CoA hydrolase, a 2,5-furan-dicarboxylic acid decarboxylase 1, a 2,5-furan-dicarboxylic acid decarboxylase 2 and a HMF/furfural oxidoreductase.

These enzymes act synergetically when provided with the relevant cofactors. The invention also relates to a composition comprising the cell extract, which preferably is a fractionated soluble cytosolic fraction.

The present invention also relates to a process for the in-situ detoxification of lignocellulose hydrolysate comprising furanic compounds, preferably HMF, HMF alcohol and HMF carboxylic acid, and Furfurylalcohol, Furfural and/or Furoic acid with a suitable host microorganism, comprising contacting the lignocellulose hydrolysate with the host microorganism under conditions facilitating the expression of one ore more of the Furoyl-CoA dehydrogenase, a Furoyl-CoA synthetase, a 2-oxoglutaroyl-CoA hydrolase, a 2,5-furan-dicarboxylic acid decarboxylase 1, a 2,5-furan-dicarboxylic acid decarboxylase 2 and a HMF/furfural oxidoreductase, as required to for the conversion of the furanic compounds, from a microorganism from the family of Burkholderiaceae, preferably Cupriavidus, more preferably Cupriavidus basilensis, again more preferably Cupriavidus basilensis hmf 14 according to the invention to convert the furanic compounds to non-toxic components to obtain a detoxified lignocellulose hydrolysate. The term "toxic" refers to lignocellulose degradation products that are fermentation inhibitors that inhibit the growth of e.g. ethanologenic microorganisms employed in the fermentation step, thereby inhibiting a suitable performance of these organisms and reducing yields. The term "non-toxic" means that the conversion products of the toxic compounds are essentially not inhibiting the growth of microorganisms that ferment the cellulose components.

Preferably, the Cupriavidus microorganism is Cupriavidus basilensis HMF14 according to the invention. More preferably, the host microorganism comprises DNA encoding one or more of the following group: Furoyl-CoA dehydrogenase, Furoyl-CoA synthetase, 2-oxoglutaroyl-CoA hydrolase, 2,5-furan-dicarboxylic acid decarboxylase 1, 2,5-furan-dicarboxylic acid decarboxylase 2 and HMF/furfural oxidoreductase from Cupriavidus microorganism of Cupriavidus basilensis HMF14 according to the invention. The process further preferably comprises subjecting the detoxified lignocellulose hydrolysate to a simultaneous or subsequent fermentation step. It further preferably comprises a step of pre-treating lignocellulose-containing material to obtain a lignocellulose hydrolysate, more preferably under acidic conditions.

A further aspect of the subject invention is a process comprising introducing isolated DNA sequence as set out herein above and/or (b) isolated DNA sequences which are at least sufficiently identical to the DNA sequences to encode,polypeptides having the activity of of one ore more of the Furoyl-CoA dehydrogenase, a Furoyl-CoA synthetase, a 2-oxoglutaroyl-CoA hydrolase, a 2,5-furan-dicarboxylic acid decarboxylase 1, a 2,5-furan-dicarboxylic acid decarboxylase 2 and a HMF/furfural oxidoreductase, as required to for the conversion of the furanic compounds in an appropriate host cell, cultivating the obtained host cell under conditions conducive to the detoxification of lignocelluloses hydrolysate, and recovering a detoxified lignocelluloses hydrolysate from the culture. The term "detoxification" thus refers to the conversion of toxic compounds to non-toxic compounds.

The present invention also provides for a process for the production of Furoyl-CoA dehydrogenase, Furoyl-CoA synthetase, 2-oxoglutaroyl-CoA hydrolase, 2,5-furan-dicarboxylic acid decarboxylase 1, 2,5-furan-dicarboxylic acid decarboxylase 2 and/or a HMF/furfural oxidoreductase which are at least 45% identical to those expressed by Cupriavidus basilensis HMF14 DSM 22875or a host microorganism as set out above, comprising (a) culturing a microorganism in a nutrient medium containing carbon and nitrogen sources and inorganic salts; and (b) isolating the enzymes produced from the microorganism.

Preferably, in the process according to the invention, Furanic compounds such as HMF, Furfurylalcohol, Furfural and/or Furoic acid are converted to a polyhydroxyalkanoate (PHA), and/or to biofuels such as ethanol. The invention also provides for a process for the in-situ detoxification of lignocellulose hydrolysate with a suitable host microorganism, comprising cultivating the host microorganism in the presence of furfural, furfuryl alcohol, hydroxymethylfurfural and/or furoic acid under conditions facilitating the expression of the enzymes from a Cupriavidus microorganism. Preferably, the host microorganism is a Cupriavidus microorganism of Cupriavidus basilensis HMF 14 according to the invention. More preferably, the host microorganism comprises DNA encoding an enzyme complex comprising a Furoyl-CoA dehydrogenase, a Furoyl-CoA synthetase, a 2-oxoglutaroyl-CoA hydrolase, a 2,5-furan-dicarboxylic acid decarboxylase 1, a 2,5-furan-dicarboxylic acid decarboxylase 2 and a HMF/furfural oxidoreductase.

A further aspect of the subject invention preferably comprises subjecting the detoxified lignocellulose hydrolysate to a simultaneous or subsequent fermentation step.

Also described is a group of enzymes forming a synergetic enzyme pathway for the degradation of furanic compounds, further referred to as enzyme complexwhich may be isolated from Cupriavidus basilensis HFM14.

Furanic compounds, such as HMF, Furfural and furanoic acid are the primary substrates for this enzyme complex expressed from C.b. HMF 14 since this organism can grow on either compound as the sole substrate providing carbon and energy.

Also a process for producing these enzymes is provided. Thus, there is provided an improved method for the biocatalytic production of PHA and/or biofuels such as ethanol in a suitable host microorganism of the enzymatic pathway of Cupriavidus basilensis strain DSM 22875, under conditions facilitating the expression of the activity.

This embodiment preferably includes modifications of Cupriavidus basilensis strain DSM 22875 to block conversion of fermentable sugars to compounds along its degradation pathway to compounds.

The present invention further pertains to a process for the conversion of furanic compoiunds, such as furfuryl alcohol and/or furfural and/or furoic acid to 2-furoyl CoA, comprising contacting furfuryl alcohol and/or furfural and/or furoic acid with **a** furoyl-CoA dehydrogenase, furoyl-CoA synthetase, 2-oxoglutaroyl-CoA hydrolase, and 2,5-furan-dicarboxylic acid decarboxylase catalyst in the presence of one or more coenzyme cofactor. Without wqishing to be bound to any particular theory, it is believed that the furoyl-CoA synthetase converts the furanic compounds to form furoyl-CoA, while the other enzymes degrade the thus obtained furoyl-CoA. The (bio)catalyst preferably comprises a polypeptide as set out herein before for each of the polypeptide enzymes.

The present invention also pertains to a process for the conversion of furanic compounds, preferably one or more of 5-hydroxymethylfurfural (HMF), 2,5-dihydroxymethyl furan (HMF alcohol), 5-hydroxymethyl-2-furancarboxylic acid (HMF acid) and/or 2,5-furandicarboxylic acid to 2-furoyl CoA, comprising comprising contacting the Furanic compounds with a furoyl-CoA dehydrogenase, furoyl-CoA synthetase, HMF oxidoreductase and the decarboxylases catalyst in the presence of one or more coenzyme cofactor.

Preferably, the coenzyme cofactor is nicotinamide adenine dinucleotide (NAD+) and/or flavin adenine dinucleotide (FAD) and/or pyrroloquinoline quinolone (PQQ).

Yet further, its capability of producing PHA may furthermore contribute to cost effectiveness since the biomass generated may be employed for the production of bioplastics.

Similarly, conditions may be suitable to also facilitate the expression of any one or more of the enzymatic activities necessary to convert the furanic compounds to poylhydroxalkanoate.

The enzyme of the present invention is a multicomponent enzyme that can utilize nicotinamide adenine dinucleotide (NADH) or nicotinamide adenine phosphate dinucleotide (NADPH), requires flavin adenine dinucleotide (FAD) and its activity is stimulated by the presence of iron in a cell-free extract.

Also proposed is the cloning and sequencing of the gene encoding the preferred enzymes, Furoyl-CoA dehydrogenase, Furoyl-CoA synthetase, 2-oxoglutaroyl-CoA hydrolase, 2,5-furan-dicarboxylic acid decarboxylase 1, 2,5-furan-dicarboxylic acid decarboxylase 2 and HMF/furfural oxidoreductase.

### Detailed description of the Figures

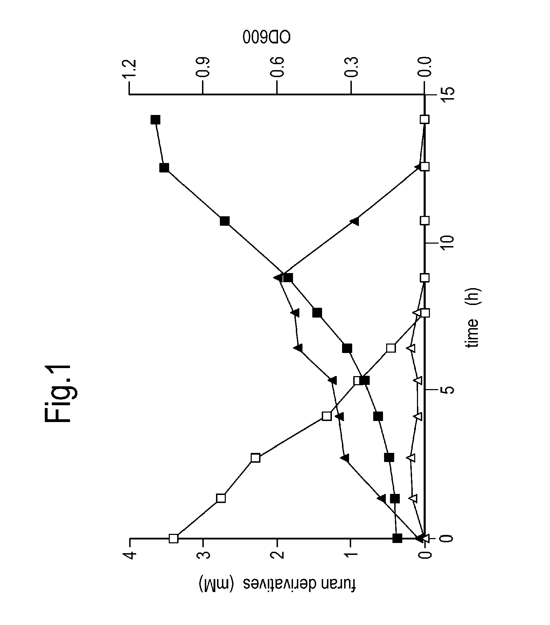

Figure 1 shows the growth of C. basilensis HMF14 on mineral salts medium with furfural as the sole carbon source. □, furfural; ▲, furfuryl alcohol; Δ, furoic acid; ■, OD600. Cultures were performed in triplicate. Variations between replicates was less than 10%.

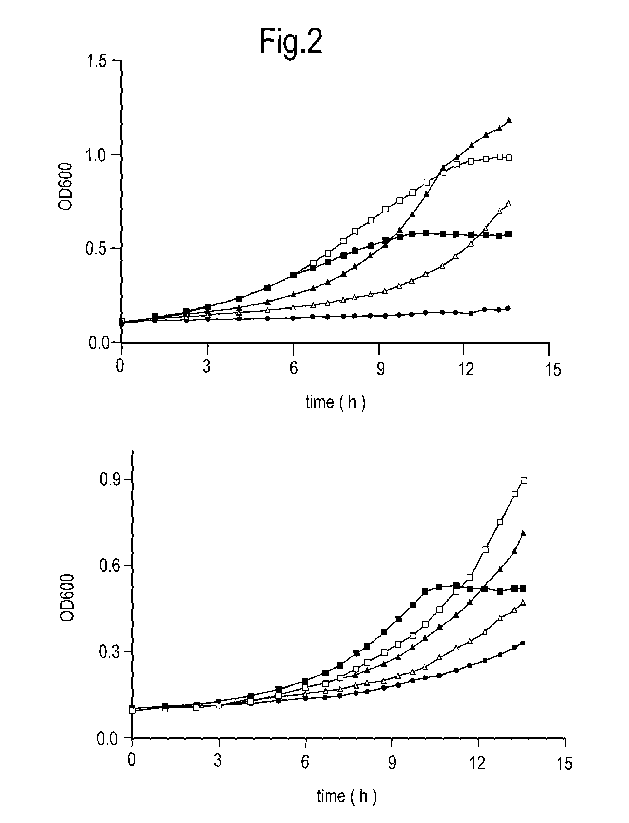

Figure 2 illustrates the growth of C. basilensis HMF14 on different concentrations of furfural (A) or HMF (B). The concentrations used were ■, 3 mM; □, 6 mM; ▲, 9 mM; Δ, 12 mM; •, 15 mM.
Figure 3 depicts the detection of PHA in cultures of C. basilensis HMF14 in minimal medium with 120 mM acetate. Left: phase contrast image. Middle: Fluorescence microscopic image of the same slide stained with Nile Blue A. Right: Overlay of the two previous images.

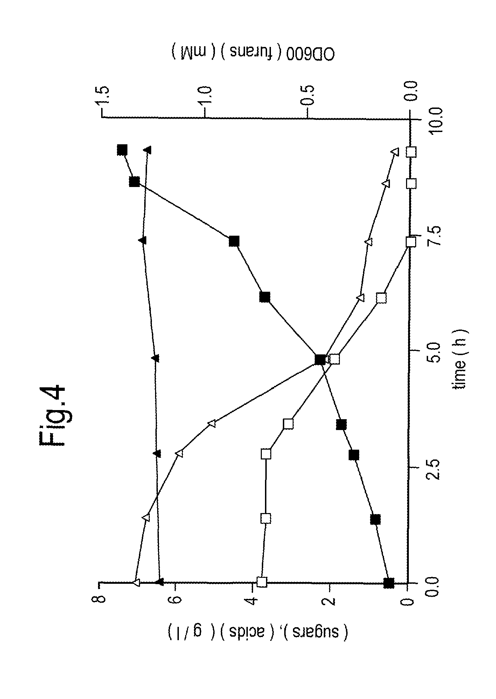

Figure 4 illustrates the detoxification of lignocellulosic hydrolysate by C. basilensis HMF14. The sugars concentration (▲) is the sum of the concentrations of glucose, xylose and arabinose. The furans concentration (Δ) is the sum of the concentrations of the alcohol, aldehyde and acid derivatives of furfural and HMF. The acids concentration (□) is the sum of the concentrations of acetic and formic acid. Biomass (■) was measured by the optical density at 600 nm.

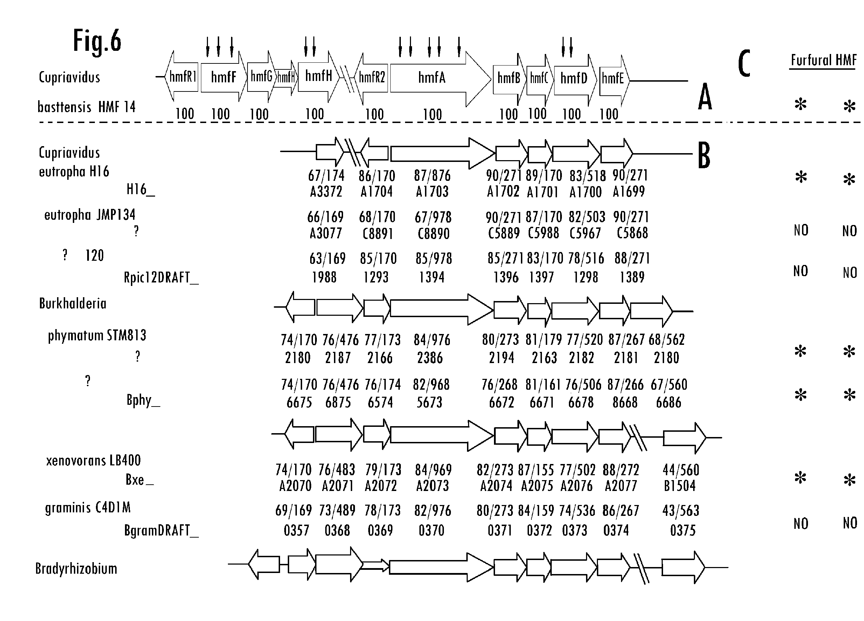

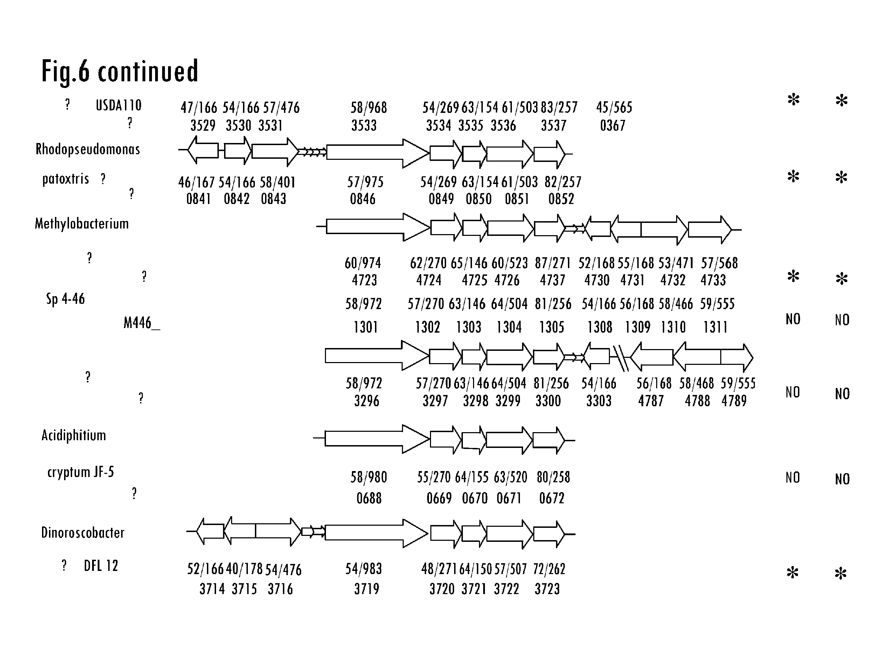

Figure 5 is a graphical representation of the HMF (A) and furfural (B) metabolic pathway in C. basilensis HMF14. Coloured hexamers and triangles indicate enzymes with the following activities: orange, furfural/HMF oxidoreductase; green and red, 2,5-furan-dicarboxylic acid decarboxylase; blue, 2-furoyl-CoA synthetase; yellow, furoyl-CoA dehydrogenase; purple, 2-oxoglutaryl-CoA hydrolase. Colours correspond to the genes depicted in figure 2A. The black square indicates a lacton hydrolysis which may occur spontaneously, or may be catalyzed by a generic lactone hydrolase. Double-pointed arrows indicate keto-enol tautomerizations. Reactions marked with (*) can be catalysed either by HmfH or by (probably non-specific) dehydrogenases. Figure 6 shows a schematic representation of the genetic organization of the furfural and HMF metabolic genes in C. basilensis HMF14 (A) and other species (B) that were identified as potential furfural and / or HMF utilizers. Colours correspond to enzyme activities in figure 1. Bold numbers (x/y) below arrows indicate the percentage identity (x) to the corresponding C. basilensis HMF14 protein in a y amino-acid stretch.

Orthologous genes were identified by BLASTx homology searches in the non-redundant protein database of the National Center for Biotechnology Information. Hits for the furfural cluster were defined as relevant when orthologues for hmfA, B, C, D and E were present in a single genome, with the hmfA orthologue encoding an enzyme that was at least 50 % identical to HmfA. The same criterion was used to define hmfF and hmfG orthologues, whereas 40 % identity to HmfH was used as the criterion for hmfH orthologues. Numbers in italics indicate genome locus tags of the indicated strain. White arrows depict genes with no metabolic function. C: Overview of growth phenotype of tested strains on mineral salts medium with either furfural or HMF (3 mM) as the sole carbon source. ND: not determined.

The following non-binding experiments illustrate the invention further.

### Culture conditions

Cultures were performed either in Luria broth (LB) or in a minimal medium (MM). Enrichment cultures were performed in MM with 20 mM HMF as the carbon source (MMH20), supplemented as indicated with 0.1 g/l yeast extract as vitamin source (MMyH20). Solid media contained 1.5 % agar. Soil and water samples were collected from the botanical garden of the Delft University of Technology and from a peat lake named 'het Kootwijkerveen' near Apeldoorn, The Netherlands. The samples were mixed and approximately 1 g was used to inoculate 50 ml MMH20 or MMyH20 in 500-ml Erlenmeyer flasks that were incubated at 30 °C for two days in a rotary shaker. One-ml samples were transferred twice to fresh medium and incubated until bacterial growth was apparent (OD600 > 1). The final enrichment cultures were streaked onto solid MMH10 and MMyH10 and incubated at 30 °C until colonies appeared.

Wheat straw hydrolysate was produced by dilute-acid hydrolysis (obtained from TNO Quality of Life, Zeist, The Netherlands). The hydrolysate was neutralized by adding 37 mM phosphate buffer (pH 7) and adjusting the pH to 7.0 with 10 M NaOH, resulting in a brown precipitate. After addition of minimal medium components, the solution was centrifuged at 10 000 x g for 5 min. The supernatant was filter sterilized through a sterile PTFE filter with a pore size of 0.22 µm. The resulting medium (MMhyd) was inoculated with an overnight pre-culture of C. basilensis HMF14 in minimal medium with 3 mM HMF, 3 mM furfural and 12 mM sodium succinate.

### Bacterial identification

Partial sequence analysis of the 16S rDNA gene was performed for preliminary identification of the bacteria isolated from the enrichment cultures. Total DNA was isolated with a FastDNA kit (QBioGene / MP Biomedicals) and the partial 16S gene was amplified by PCR using primers FD1/2, AGAGTTTGATCMTGGCTCAG and RP1/2, ACGGYTACCTTGTTACGACTT. PCR products were purified with a Qiaquick PCR purification kit (Qiagen) and sequenced by MWG Biotech AG with the same primers used for amplification.

In order to isolate the genes involved in the HMF metabolic pathway of C. basilensis HMF14, a transposon mutant library of C. basilensis HMF14 was constructed and screened for clones unable to grow on furfural and / or HMF. Twenty-five transposon mutants were selected from 14 000 clones and the chromosomal DNA flanking the transposon insertion sites was sequenced to identify the interrupted genes. Additional primer walking sequencing of up- and downstream regions of these genes revealed two distinct gene clusters. The first cluster contained five genes, designated hmfABCDE, whereas the other cluster contained four genes: hmfFGH'H. Insertion of a transposon in either of the two clusters corresponded to two distinct phenotypes. If the hmfABCDE cluster was interrupted, no growth occurred on either HMF or furfural, suggesting an -at least partly- shared degradation pathway for furfural and HMF. An insertion in the hmfFGH'H cluster resulted in loss of growth on HMF only. Mutant phenotypes of transposon mutants and BLASTx analysis {Altschul, S.F. et al. Basic local alignment search tool. J. Mol. Biol. 215, 403-410 (1990)} of the genes comprised in the two clusters are summarized in Table 1.

The resulting sequences were assigned the following functions (Table 1):

**Table 1. Growth phenotype of selected C. basilensis HMF14 transposon mutants, and BLASTx analysis and assigned function of genes involved in furfural and HMF degradation.**

| Gene | Growth phenotype of transposon mutant | | | Best BLASTx hit (Acc. No) | Assigned function |
|---|---|---|---|---|---|
| | MM + citrate | MM + fur-fural | MM + HMF | | |
| hmfA (Seq. ID No. 36) | + | - | - | Aerobic-type carbon monoxide dehydrogenase homolog, subunits L and G (YP_726196) | Furoyl-CoA dehydrogenase large subunit |
| hmfB (Seq. ID No. 38) | | | | Carbon-monoxide dehydrogenase (YP_293089) | Furoyl-CoA dehydrog enase FAD binding subunit |
| hmfC (SEQ ID NO: 40) | | | | Aerobic-type carbon monoxide dehydrogenase 2Fe-2S iron-sulfur subunit (YP_726194) | Furoyl-CoA dehydrog enase 2Fe-2S iron sulfur subunit Furoyl- |
| hmfD (SEQ ID NO: 42) | + | - | - | Acyl-CoA synthetase (YP_726193) | CoA synthetase |
| hmfE (SEQ ID NO: 44) | | | | enoyl-CoA hydratase/isomerase (YP_293086) | 2-oxoglutar oyl-CoA hydrolase |
| hmfF (SEQ ID NO: 20) | + | + | - | UbiD family decarboxylase (YP_001895811) | 2,5-furandicarboxy lic acid decarboxylapse 1 |
| hmfG (SEQ ID NO: 22) | + | + | - | 3-octaprenyl-4-hydroxybenzoate carboxylyase (ZP_02881560) | 2,5-furandicarboxy lic acid decarboxylase 2 |
| hmfH' (SEQ ID NO: 24) | | | | hypothetical protein (YP_293096) | NA |
| (SEQ ID hmfH NO: 26) | + | + | - | glucose-methanol-choline oxidoreductase (YP_001895804) | HMF / -HMF/-furfural oxido-reductase |
| hmfR1 (SEQ ID NO: 18) | | | | LysR family transcriptional regulator (YP_001862747.1) | Putative LysR type transcrip t-tonal regulator |
| hmfR2 (SEQ ID NO: 34) | | | | LysR family transcriptional regulator (YP_293091.1) | Putative LysR type transcrip tional regulator |

| | | | | | |
|---|---|---|---|---|---|
| a. The mutant phenotype was not determined since no transposon mutant was available. NA; no assigned function. | | | | | |

As such, a suitable host organism can preferably be transformed or transfected with DNA encoding one or more of the above sequences according to the invention.

### Elucidation of the furfural catabolic pathway of C. basilensis HMF14

The enzyme functions encoded by the hmfABCDE cluster of C. basilensis HMF14 were in good agreement with the enzyme activities that were reported to constitute the furoic acid degradation pathway of Pseudomonas putida strains F2 and Fu1 (see Fig. 6). The first step of this proposed pathway involves an acyl-CoA synthetase to produce 2-furoyl-CoA from 2-furoic acid, which activity matches the function of HmfD. This was supported by the accumulation of 2-furoic acid in hmfD-disrupted transposon mutants of C. basilensis HMF14 when cultured in the presence of furfuryl alcohol or furfural. Furthermore, it was established that 2-furoic acid is the substrate for ATP-dependent CoA ligation by HmfD. This activity was present in cell extracts of wildtype C. basilensis HMF14 and P. putida S12 expressing HmfD, whereas it was absent in C. basilensis HMF14 transposon mutants in which hmfD was disrupted.

In P. putida F2 and Fu1, 2-furoyl-CoA was converted into 5-hydroxy-2-furoyl-CoA by a molybdenum-dependent 2-furoyl-CoA dehydrogenase. The proteins encoded by hmfABC in C. basilensis HMF14 correspond to the three subunits that constitute a bacterial Mo-dependent dehydrogenase. The functionality of hmfABC was confirmed by demonstrating furoic-acid dependent Nitro Blue Tetrazolium (NBT) reducing activity in cell extracts of P. putida S12 co-expressing HmfABC and HmfD. The latter activity was required to generate 2-furoyl-CoA from 2-furoic acid as the substrate for HmfABC.

The HMF degradation route of C. basilensis HMF14 was reconstructed based on putative gene functions of the hmfFGH'H cluster. The hmfFG genes encode two putative decarboxylases of the UbiD/UbiX type which commonly operate concertedly 20, 21. C. basilensis HMF14 mutants with disrupted hmfFG genes accumulated HMF acid and 2,5-furan-dicarboxylic acid (FDCA) when cultured in the presence of HMF, which suggested that these carboxylic acids were the substrate for HmfFG. Cell extracts of both wildtype C. basilensis HMF14 and P. putida S12 expressing HmfFG formed 2-furoic acid when incubated with FDCA. HMF acid was not decarboxylated to furfuryl alcohol, demonstrating that FDCA was the actual substrate for HmfFG. Thus, HMF degradation in C. basilensis HMF14 proceeds obligately via its dicarboxylic acid form. No decarboxylase activity was observed in cell extract of P. putida S12 expressing HmfG only. When HmfF was expressed as a single enzyme only slight decarboxylase activity was observed, demonstrating that both proteins are required for optimal FDCA decarboxylase acitivity.

The hmfH gene encodes a putative FAD-dependent oxidoreductase.

C. basilensis HMF14 mutants with a disrupted hmfH gene accumulated HMF acid when cultured in the presence of HMF. Cell extracts of both wildtype C. basilensis HMF14 and P. putida S12 expressing HmfH formed FDCA when incubated with HMF acid, confirming that HmfH catalyzes the oxidation of the HMF-monocarboxylic acid to the dicarboxylic acid form. No FDCA was formed when oxygen was removed, demonstrating that HmfH is a true oxidase.

The hmfH' gene encodes a hypothetical protein with 49 % identity over a stretch of 296 amino acids to a probable extra-cytoplasmic solute receptor of Ralstonia eutropha H16. This gene may play a role in HMF transport, but a metabolic function was considered unlikely 22.

Analogous to the furfural pathway, no specific genes were identified for the oxidations in the upper HMF metabolic pathway leading from HMF-alcohol to HMF and HMF-acid. Also these oxidations were concluded to be performed by non-specific, redundant dehydrogenases which activities were observed both in C. basilensis HMF14 and P. putida S12 (Table 2). However, it was observed that also HmfH could oxidize HMF, furfural, and furfuryl alcohol to the corresponding acids, respectively, furfural. Apparently, this oxidase is essential for the formation of FDCA from HMF-acid but also provides an oxidase-alternative to the non-specific alcohol and aldehyde dehydrogenases that constitute the upper metabolic pathways for HMF and furfural.

Based on the above observations, the pathway depicted in Fig. 5 was constructed for HMF catabolism. First, HMF is oxidized to HMF acid, either by non-specific dehydrogenases or by HmfH. Subsequently, HMF acid is oxidized to FDCA for which conversion HmfH is essential. The HMF and the furfural catabolic pathways converge at the level of 2-furoic acid upon decarboxylation of FDCA by HmfFG.

### Analytical methods

Bacterial growth was determined by measuring optical density at 600 nm (OD600) using a Biowave Cell Density Meter (WPA Ltd) or a µQuant MQX200 universal microplate spectrophotometer (Bio-tek), using flat-bottom 96-well microplates (Greiner). Furan derivatives were analyzed on an Agilent 1100 system equipped with a diode array detector set at 230 nm. The column used was a Zorbax Eclipse XDB-C8 (length, 150 mm; internal diameter, 4.6 mm; particle size, 5 µm; Agilent) operated at 25°C. As eluent, a gradient of acetonitrile in 20 mM KH2PO4 (pH 2) with 1% acetonitrile was used at a flow of 1.2 ml / min, increasing from 0 to 5% in 3.5 min and from 5 to 40% in 2.5 min, set as smooth gradients.

Glucose, xylose and arabinose were analyzed by ion chromatography (Dionex ICS3000 system), using a CarboPac PA20 column (length, 150 mm; internal diameter, 3 mm) with 10 mM NaOH at a flow rate of 0.5 ml·min-1 as the eluent.

For production of PHA, C. basilensis HMF14 was cultured in minimal medium with 120 mM acetate as a carbon source and 6 mM (NH4)2SO4 as a nitrogen source. PHA was visualized by fluorescence microscopy using Nile Blue A staining, basically as described by Johnson et al. (8).

### Chemicals

The analytical standard of 2,5-furandicarboxylic acid was purchased from Immunosource B.V. (Halle-Zoersel, Belgium). 5-Hydroxymethyl-furoic acid (HMF acid) was purchased from Matrix Scientific (Columbia SC, United States). This compound was found to be highly esterified. Therefore, immediately prior to use, a 10 mM solution of the esterified HMF acid was boiled for two hours in 2 M H2SO4, cooled, and adjusted to pH 7.0 with NaOH after addition of 50 mM of phosphate buffer (pH 7). All other chemicals were purchased from Sigma-Aldrich Chemie B.V. (Zwijndrecht, The Netherlands). 5-Hydroxy-2-methylfurfuryl alcohol was identified based on its UV-VIS spectrum (3).

### Enrichment and characterization of HMF degrading bacteria

Enrichment cultures on minimal medium with HMF as the sole carbon source were inoculated with soil and water samples. After 2 transfers into fresh medium, the cultures were plated on solid HMF medium to isolate individual bacteria capable of degrading HMF. Fourteen individual colonies were selected and initial identification was performed by partial 16S rDNA sequencing. Only one isolate (CB HMF14) was the only isolate incapable of utilizing glucose. In addition, HMF14 was easily culturable.

### Cupriavidus sp. HMF14 (according to the invention)

Cupriavidus sp. HMF14 was able to grow on gluconate, succinate, citrate, acetate, benzene, toluene and phenol. No growth was observed on glucose, xylose, arabinose and mannose. Cells were short rods, either single, in pairs or in short chains. On LB agar plates round colonies were formed that had a mucous appearance and formation of mucus was also observed in liquid cultures. Strain HMF14 could be cultured at temperatures up to 41 °C and did not show anaerobic nitrate respiration. The phenotypic characteristics of Cupriavidus sp. HMF14 best match the type species of Cupriavidus basilensis (DSMZ 11853T). Therefore, the strain was designated Cupriavidus basilensis HMF14.

The genus Cupriavidus is well known for its ability to efficiently produce PHA (24, 31). In order to verify PHA production by the newly isolated C. basilensis HMF14, this strain was cultivated in minimal medium with acetate as a carbon source until an OD600 of 2.4 was reached. Fluorescence microscopic analysis showed PHA granules within the cells of C. basilensis (Figure 3).

### Degradation of furan derivatives by C. basilensis HMF14

In addition to HMF, other furan derivatives are present in lignocellulosic hydrolysates. In order to demonstrate whether C. basilensis HMF14 was capable of utilizing furan derivatives other than HMF, growth was assessed on minimal medium with 3.5 mM HMF, furfural, furfuryl alcohol or furoic acid as sole carbon source. Growth was observed on all tested furan derivatives, with slightly different growth characteristics (table 1). In cultures on furfural, furfural was initially rapidly converted to furfuryl alcohol, while also a small amount of furoic acid was formed (Fig. 1). Without wishing to be bound to any particular theory, conversion of furfural to its alcohol and / or acid form appears to be a common mechanism of furfural detoxification. At the onset of logarithmic growth, furfuryl alcohol production decreased in favour of biomass formation, which likely occurs via furoic acid. Similarly, HMF acid and -alcohol were formed in cultures with HMF as the carbon source. In addition, trace amounts of 2,5-furandicarboxylic acid and furoic acid were found in the HMF cultures (not shown).

**Table 2. Growth characteristics of C. basilensis HMF14 on furan derivatives.**

| Carbon source | µmax (h-1) | Max. OD600 |
|---|---|---|
| HMF | 0.25^{a} | 0.95 |
| Furfural | 0.22 | 1.09 |
| Furfuryl alcohol | 0.22 | 1.08 |
| Furoic acid | 0.29 | 0.99 |

| | | |
|---|---|---|
| ^{a} This culture did not reach stable exponential phase, since the growth rate increased continuously. | | |

C. basilensis HMF14 grew in the presence of 5 mM of furfural or HMF (0.48 g / 1, and 0.63 g / 1 respectively). However, since the concentration of these toxic compounds is often higher in lignocellulosic hydrolysates, with values ranging from 0 to 3.5 g / 1 for furfural, and from 0 to 5.9 g / 1 for HMF, the tolerance of C. basilensis HMF14 towards furfural and HMF was determined in shake-flask cultures with 3-15 mM furfural or HMF (Fig. 2, table 2). The lag phase was found to increase with increasing concentrations of HMF or furfural (Fig. 2), likely as a result of substrate toxicity. Nevertheless, after 24 h of cultivation growth was observed at all concentrations tested (not shown). No stable exponential phase was reached at furfural concentrations above 6 mM, but increased apparent growth rates were found at higher concentrations of furfural (results not shown). Also in the HMF cultures, no stable exponential growth was observed, but the apparent growth rate decreased rather than increased with increasing HMF concentrations.

### Detoxification of lignocellulosic hydrolysate by C. basilensis HMF14

In addition to the furan derivatives, lignocellulosic hydrolysate also contains many other components that can inhibit fermentative production of biochemicals. While C. basilensis HMF14 is unable to degrade the sugars present in lignocellulosic hydrolysate, it preferably degrades many of the other toxic constituents, as illustrated in shake-flask cultures with each compound as a single carbon source (table 3).

**Table 3. Degradation of constituents of lignocellulosic hydrolysate by C. basilensis HMF14.**

| Compound | | Substrate utilization by C. basilensis |
|---|---|---|
| | | HMF14^{a} |
| Sugars | | |
| | Glucose | N |
| | Xylose | N |
| | Arabinose | N |
| | Mannose | N |
| | Furans | |
| | Furfural | Y |
| | Furfuryl alcohol | Y |
| | | Y |
| Hydroxymethylfurfural | | |
| | Furoic acid | |
| Organic acids | | |
| | Acetic acid | Y |
| | Formic acid | Y^{b} |
| | Levulinic acid | Y |
| | Ferulic acid | Y |
| Aromatics | | |
| | 4-hydroxybenzoic acid | Y |
| | Vanillic acid | Y |
| | Syringic acid | N |
| | Phenol | Y |
| | 4- hydroxybenzaldehyde | Y |
| | 4-hydroxybenzyl alcohol | Y |
| | Guaiacol | Y |
| | Vanillin | Y |
| | Vanillyl alcohol | Y |
| | Syringol | N |
| | Syringaldehyde | N |

| | | |
|---|---|---|
| ^{a} Y, Yes; N, No. ^{b} Formic acid was only co-utilized with a different carbon source. | | |

This unique substrate specificity makes C. basilensis HMF14 ideally suited for the biological detoxification of lignocellulosic hydrolysate.

### Heterologous expression of the furfural and HMF degradation pathways in P. putida S12

The functional characterization of the furfural and HMF catabolic genes of C. basilensis HMF14 enabled a reconstruction of the complete catabolic pathway for these furanic compounds. For a final verification of the functionality of the reconstructed pathway, the encoding genes were expressed in a heterologous host, P. putida S12.

First, the furfural cluster hmfABCDE was introduced into P. putida S12. As expected, the resulting strain, P. putida S12 pJT'hmfABCDE, was able to utilize furoic acid, furfural and furfuryl alcohol as sole carbon sources, although growth was initially poor. Therefore, strain S12 pJT'hmfABCDE was repeatedly transferred to fresh mineral salts medium with furfural as the sole carbon source. After 10 serial transfers, P. putida strain S12_fur was obtained which showed a reproducible growth rate of 0.30 h-1 on furfural as a sole carbon source with a biomass yield of 51 % (C-mol biomass / C-mol substrate). P. putida S12 strains expressing only HmfABCD or HmfABC were also constructed, but these strains failed to grow on furoic acid. These results confirmed that all genes required for furfural metabolism are located in the furfural cluster hmfABCDE and that all genes in this cluster are essential for furfural metabolism, including the hmfE-encoded CoA thioester hydrolase.

Subsequently, the hmfFGH genes were cloned into P. putida S12_fur. The resulting strain, P. putida S12_HMF, was able to utilize either furfural or HMF as the sole carbon source, at a growth rate of 0.23 h-1 and a yield of 40 % (C-mol biomass / C-mol substrate). Gene hmfH' was apparently dispensable for growth on HMF, confirming that the encoded gene had no function in HMF metabolism. Nor was the gene essential for HMF transport in P. putida S12. Thus, also all genes required for the utilization of HMF were characterized, and their functionality was reconfirmed by functional expression in a heterologous host.

## Claims

1. An isolated microorganism which is Cupriavidus basilensis strain HMF14 Deposit number DSM 22875.

2. A bacterial culture comprising a Cupriavidus microorganism of Cupriavidus basilensis HMF 14 strain Deposit number DSM 22875 according to claim 1.

3. An isolated microorganism or culture according to claim 1 or claim 2, which, when provided with HMF, Furfurylalcohol, Furfural and/or Furoic acid as a carbon source, grows on said source.

4. An isolated microorganism or culture according to anyone of claims 1 to 3, which expresses the following enzymes: a Furoyl-CoA dehydrogenase, a Furoyl-CoA synthetase, a 2-oxoglutaroyl-CoA hydrolase, a 2,5-furan-dicarboxylic acid decarboxylase 1, a 2,5-furan-dicarboxylic acid decarboxylase 2 and a HMF/furfural oxidoreductase.

5. A polypeptide having aldehyde dehydrogenase activity which comprises the amino acid sequence set out in SEQ ID NO: 15 or an amino acid sequence encoded by the nucleotide sequence of SEQ ID NO: 16 or a variant polypeptide thereof, wherein the variant has at least 64% sequence identity or more with the sequence set out in SEQ ID NO: 16.

6. A polynucleotide which comprises:
(a) the nucleotide sequence set out in SEQ ID NO: 16;
(b) a nucleotide sequence which hybridizes selectively with a polynucleotide being the reverse complement of SEQ ID NO: 16;
(c) a nucleotide sequence having at least 64% sequence identity or more with the nucleotide sequence of SEQ ID NO: 16;
(d) a fragment of a nucleotide sequence as defined in (a), (b) or (c) which is at least about 100 nucleotides in length;
(e) a sequence which is degenerate as a result of the genetic code to a sequence as defined in any one of (a), (b), (c) or (d);
(f) a nucleotide sequence which is the reverse complement of a nucleotide sequence as defined in (a), (b), (c), (d) or (e).

7. A polypeptide having LysR family transcriptional regulator activity which comprises the amino acid sequence set out in SEQ ID NO: 17 or an amino acid sequence encoded by the nucleotide sequence of SEQ ID NO: 18 or a variant polypeptide thereof, wherein the variant has at least 47% sequence identity or more with the sequence set out in SEQ ID NO: 17.

8. A polynucleotide which comprises:
(a) the nucleotide sequence set out in SEQ ID NO: 18;
(b) a nucleotide sequence which hybridizes selectively with a polynucleotide being the reverse complement of SEQ ID NO: 18;
(c) a nucleotide sequence having at least 72% sequence identity or more with the nucleotide sequence of SEQ ID NO: 18;
(d) a fragment of a nucleotide sequence as defined in (a), (b) or (c) which is at least about 100 nucleotides in length;
(e) a sequence which is degenerate as a result of the genetic code to a sequence as defined in any one of (a), (b), (c) or (d);
(f) a nucleotide sequence which is the reverse complement of a nucleotide sequence as defined in (a), (b), (c), (d) or (e).

9. A polypeptide having 2,5-furan-dicarboxylic acid decarboxylase 1 activity which comprises the amino acid sequence set out in SEQ ID NO: 19 or an amino acid sequence encoded by the nucleotide sequence of SEQ ID NO: 20 or a variant polypeptide thereof, wherein the variant has at least 54% sequence identity or more with the sequence set out in SEQ ID NO: 19.

10. A polynucleotide which comprises:
(a) the nucleotide sequence set out in SEQ ID NO: 20;
(b) a nucleotide sequence which hybridizes selectively with a polynucleotide being the reverse complement of SEQ ID NO: 20;
(c) a nucleotide sequence having at least 66% sequence identity or more with the nucleotide sequence of SEQ ID NO: 20;
(d) a fragment of a nucleotide sequence as defined in (a), (b) or (c) which is at least about 100 nucleotides in length;
(e) a sequence which is degenerate as a result of the genetic code to a sequence as defined in any one of (a), (b), (c) or (d);
(f) a nucleotide sequence which is the reverse complement of a nucleotide sequence as defined in (a), (b), (c), (d) or (e).

11. A polypeptide having 2,5-furan-dicarboxylic acid decarboxylase 2 activity which comprises the amino acid sequence set out in SEQ ID NO: 21 or an amino acid sequence encoded by the nucleotide sequence of SEQ ID NO: 22 or a variant polypeptide thereof, wherein the variant has at least 52% sequence identity or more with the sequence set out in SEQ ID NO: 21.

12. A polynucleotide which comprises:
(a) the nucleotide sequence set out in SEQ ID NO: 22;
(b) a nucleotide sequence which hybridizes selectively with a polynucleotide being the reverse complement of SEQ ID NO: 22;
(c) a nucleotide sequence having at least 67% sequence identity or more with the nucleotide sequence of SEQ ID NO: 22;
(d) a fragment of a nucleotide sequence as defined in (a), (b) or (c) which is at least about 100 nucleotides in length;
(e) a sequence which is degenerate as a result of the genetic code to a sequence as defined in any one of (a), (b), (c) or (d);
(f) a nucleotide sequence which is the reverse complement of a nucleotide sequence as defined in (a), (b), (c), (d) or (e).

13. A polypeptide having HMF/furfural oxidoreductase activity which comprises the amino acid sequence set out in SEQ ID NO: 25 or an amino acid sequence encoded by the nucleotide sequence of SEQ ID NO: 26 or a variant polypeptide thereof, wherein the variant has at least 45% sequence identity or more with the sequence set out in SEQ ID NO: 25.

14. A polynucleotide which comprises:
(a) the nucleotide sequence set out in SEQ ID NO: 26;
(b) a nucleotide sequence which hybridizes selectively with a polynucleotide being the reverse complement of SEQ ID NO: 26;
(c) a nucleotide sequence having at least 66% sequence identity or more with the nucleotide sequence of SEQ ID NO: 26;
(d) a fragment of a nucleotide sequence as defined in (a), (b) or (c) which is at least about 100 nucleotides in length;
(e) a sequence which is degenerate as a result of the genetic code to a sequence as defined in any one of (a), (b), (c) or (d);
(f) a nucleotide sequence which is the reverse complement of a nucleotide sequence as defined in (a), (b), (c), (d) or (e).

15. A polypeptide having LysR type transcriptional regulator activity which comprises the amino acid sequence set out in SEQ ID NO: 33 or an amino acid sequence encoded by the nucleotide sequence of SEQ ID NO: 34 or a variant polypeptide thereof, wherein the variant has at least 46% sequence identity or more with the sequence set out in SEQ ID NO: 33.

16. A polynucleotide which comprises:
(a) the nucleotide sequence set out in SEQ ID NO: 34;
(b) a nucleotide sequence which hybridizes selectively with a polynucleotide being the reverse complement of SEQ ID NO: 34;
(c) a nucleotide sequence having at least 65% sequence identity or more with the nucleotide sequence of SEQ ID NO: 34;
(d) a fragment of a nucleotide sequence as defined in (a), (b) or (c) which is at least about 100 nucleotides in length;
(e) a sequence which is degenerate as a result of the genetic code to a sequence as defined in any one of (a), (b), (c) or (d);
(f) a nucleotide sequence which is the reverse complement of a nucleotide sequence as defined in (a), (b), (c), (d) or (e).

17. A polypeptide having Furoyl-CoA dehydrogenase (large subunit) activity which comprises the amino acid sequence set out in SEQ ID NO: 35 or an amino acid sequence encoded by the nucleotide sequence of SEQ ID NO: 36 or a variant polypeptide thereof, wherein the variant has at least 54% sequence identity or more with the sequence set out in SEQ ID NO: 35.

18. A polynucleotide which comprises:
(a) the nucleotide sequence set out in SEQ ID NO: 36;
(b) a nucleotide sequence which hybridizes selectively with a polynucleotide being the reverse complement of SEQ ID NO: 36;
(c) a nucleotide sequence having at least 66% sequence identity or more with the nucleotide sequence of SEQ ID NO: 36;
(d) a fragment of a nucleotide sequence as defined in (a), (b) or (c) which is at least about 100 nucleotides in length;
(e) a sequence which is degenerate as a result of the genetic code to a sequence as defined in any one of (a), (b), (c) or (d);
(f) a nucleotide sequence which is the reverse complement of a nucleotide sequence as defined in (a), (b), (c), (d) or (e).

19. A polypeptide having Furoyl-CoA dehydrogenase (FAD binding subunit) activity which comprises the amino acid sequence set out in SEQ ID NO: 37 or an amino acid sequence encoded by the nucleotide sequence of SEQ ID NO: 38 or a variant polypeptide thereof, wherein the variant has at least 49% sequence identity or more with the sequence set out in SEQ ID NO: 37.

20. A polynucleotide which comprises:
(a) the nucleotide sequence set out in SEQ ID NO: 38;
(b) a nucleotide sequence which hybridizes selectively with a polynucleotide being the reverse complement of SEQ ID NO: 38;
(c) a nucleotide sequence having at least 71% sequence identity or more with the nucleotide sequence of SEQ ID NO: 38;
(d) a fragment of a nucleotide sequence as defined in (a), (b) or (c) which is at least about 100 nucleotides in length;
(e) a sequence which is degenerate as a result of the genetic code to a sequence as defined in any one of (a), (b), (c) or (d);
(f) a nucleotide sequence which is the reverse complement of a nucleotide sequence as defined in (a), (b), (c), (d) or (e).

21. A polypeptide having Furoyl-CoA dehydrogenase 2Fe-2S iron sulfur subunit activity which comprises the amino acid sequence set out in SEQ ID NO: 39 or an amino acid sequence encoded by the nucleotide sequence of SEQ ID NO: 40 or a variant polypeptide thereof, wherein the variant has at least 64% sequence identity or more with the sequence set out in SEQ ID NO: 39.

22. A polynucleotide which comprises:
(a) the nucleotide sequence set out in SEQ ID NO: 40;
(b) a nucleotide sequence which hybridizes selectively with a polynucleotide being the reverse complement of SEQ ID NO: 40;
(c) a nucleotide sequence having at least 70% sequence identity or more with the nucleotide sequence of SEQ ID NO: 40; (d) a fragment of a nucleotide sequence as defined in (a), (b) or (c) which is at least about 100 nucleotides in length;
(e) a sequence which is degenerate as a result of the genetic code to a sequence as defined in any one of (a), (b), (c) or (d);
(f) a nucleotide sequence which is the reverse complement of a nucleotide sequence as defined in (a), (b), (c), (d) or (e).

23. A polypeptide having Furoyl-CoA synthetase activity which comprises the amino acid sequence set out in SEQ ID NO: 41 or an amino acid sequence encoded by the nucleotide sequence of SEQ ID NO: 42 or a variant polypeptide thereof, wherein the variant has at least 57% sequence identity or more with the sequence set out in SEQ ID NO: 41.

24. A polynucleotide which comprises:
(a) the nucleotide sequence set out in SEQ ID NO: 42;
(b) a nucleotide sequence which hybridizes selectively with a polynucleotide being the reverse complement of SEQ ID NO: 42;
(c) a nucleotide sequence having at least 68% sequence identity or more with the nucleotide sequence of SEQ ID NO: 42;
(d) a fragment of a nucleotide sequence as defined in (a), (b) or (c) which is at least about 100 nucleotides in length;
(e) a sequence which is degenerate as a result of the genetic code to a sequence as defined in any one of (a), (b), (c) or (d);
(f) a nucleotide sequence which is the reverse complement of a nucleotide sequence as defined in (a), (b), (c), (d) or (e).

25. A polypeptide having 2-oxoglutaroyl-CoA hydrolase activity which comprises the amino acid sequence set out in SEQ ID NO: 43 or an amino acid sequence encoded by the nucleotide sequence of SEQ ID NO: 44 or a variant polypeptide thereof, wherein the variant has at least 72% sequence identity or more with the sequence set out in SEQ ID NO: 43.

26. A polynucleotide which comprises:
(a) the nucleotide sequence set out in SEQ ID NO: 44;
(b) a nucleotide sequence which hybridizes selectively with a polynucleotide being the reverse complement of SEQ ID NO: 44;
(c) a nucleotide sequence having at least 74% sequence identity or more with the nucleotide sequence of SEQ ID NO: 44;
(d) a fragment of a nucleotide sequence as defined in (a), (b) or (c) which is at least about 100 nucleotides in length;
(e)a sequence which is degenerate as a result of the genetic code to a sequence as defined in any one of (a), (b), (c) or (d);
(f)a nucleotide sequence which is the reverse complement of a nucleotide sequence as defined in (a), (b), (c), (d) or (e).

27. A polynucleotide which encodes a polypeptide according to anyone of claims 5 to 26.

28. A nucleic acid construct comprising the polynucleotide according to any one of claims 5 to 26.

29. A vector incorporating a polynucleotide sequence according to any of claims 5 to 26 or a nucleic acid construct according to claim 27 or 28.

30. A cell comprising a polypeptide according to any one of claims 5 to 26, a polynucleotide according to claim 25, a nucleic acid construct according to claim 28 or a vector according to claim 29.

31. A host microorganism cell transformed or transfected by the polynucleotide sequence according to any of claims 5 to 26 or a nucleic acid construct according to claim 27 or 28 or by a vector according to claim 29 under conditions conducive to express one or more of a Furoyl-CoA dehydrogenase, a Furoyl-CoA synthetase, a 2-oxoglutaroyl-CoA hydrolase, a 2,5-furan-dicarboxylic acid decarboxylase 1, a 2,5-furan-dicarboxylic acid decarboxylase 2 and a HMF/furfural oxidoreductase.

32. A cell according to claim 31, wherein the cell is a prokaryote cell, an eukaryote cell, a plant cell or an animal cell.

33. A cell according to claim 32, wherein the cell is a bacterium chosen from the group of Escherichia, Anabaena, Caulobactert, Gluconobacter, Rhodobacter, Pseudomonas, Paracoccus, Bacillus, Brevibacterium, Corynebacterium, Rhizobium (Sinorhizobium), Flavobacterium, Klebsiella, Enterobacter, Lactobacillus, Lactococcus, Methylobacterium, Staphylococcus or Streptomyces genus; or B. subtilis, B. amyloliquefaciens, B. licheniformis, B. puntis, B. megaterium, B. halodurans, B. pumilus, G. oxydans, Caulobactert crescentus CB 15, Methylobacterium extorquens, Rhodobacter sphaeroides, Pseudomonas zeaxanthinifaciens, Paracoccus denitrificans, E. coli, C. glutamicum, Staphylococcus carnosus, Streptomyces lividans, Sinorhizobium melioti and Rhizobium radiobacter species.

34. A cell according to claim 32, wherein the cell is a yeast cell from the group consisting of the Candida, Hansenula, Kluyveromyces, Pichia, Saccharomyces, Schizosaccharomyces, or Yarrowia genus; or Kluyveromyces lactis, S. cerevisiae, Hansenula polymorpha, Yarrowia lipolytica and Pichia pastoris species or a filamentous fungal cell from the group Aspergillus, Chrysosporium, Penicillium, Talaromyces or Trichoderma genus; or Aspergillus niger, Aspergillus awamori, Aspergillus foetidus, Aspergillus sojae, Aspergillus fumigatus, Talaromyces emersonii, Aspergillus oryzae, Chrysosporium lucknowense, Trichoderma reesei or Penicillium chrysogenum species.

35. A cell according to any one of claims 33 to 34, wherein one or more gene is deleted, knocked-out or disrupted in full or in part, wherein optionally the gene encodes for a protease.

36. A cell extract from a Cupriavidus microorganism according to claim 1 or claim 2 or a host cell according to claims 32 to 34, comprising a Furoyl-CoA dehydrogenase, a Furoyl-CoA synthetase, a 2-oxoglutaroyl-CoA hydrolase, a 2,5-furan-dicarboxylic acid decarboxylase 1, a 2,5-furan-dicarboxylic acid decarboxylase 2 and a HMF/furfural oxidoreductase.

37. A process for the in-situ detoxification of lignocellulose hydrolysate containing furanic compounds, such as preferably one or more of HMF, Furfurylalcohol, Furfural and/or Furoic acid, with a suitable host microorganism, comprising contacting the lignocellulose hydrolysate with the host microorganism under conditions facilitating the expression of one or more of a Furoyl-CoA dehydrogenase, a Furoyl-CoA synthetase, a 2-oxoglutaroyl-CoA hydrolase, a 2,5-furan-dicarboxylic acid decarboxylase 1, a 2,5-furan-dicarboxylic acid decarboxylase 2 and a HMF/furfural oxidoreductase from a microorganism from the family of Burkholderiaceae to convert furanic compounds, such as HMF, Furfurylalcohol, Furfural and/or Furoic acid, to non-toxic components to obtain a detoxified lignocellulose hydrolysate.

38. A process according to claim 37, wherein the Cupriavidus microorganism is Cupriavidus basilensis, preferably Cupriavidus basilensis HMF14 DSM 22875 according to claim 1.

39. A process according to claim 37, wherein the host microorganism is a microorganism according to any one of claims 32 to 34.

40. A process according to anyone of claims 37 to 39, further comprising subjecting the detoxified lignocellulose hydrolysate to a simultaneous or subsequent fermentation step.

41. A process according to anyone of claims 37 to 40, further comprising a step of pre-treating lignocellulose-containing material to obtain a lignocellulose hydrolysate, preferably under acidic conditions.

42. A process according to anyone of claims 37 to 41, comprising introducing (a) a polynucleotide according to claim 27 in an appropriate host cell, cultivating the obtained host cell under conditions conducive to the detoxification of lignocelluloses hydrolysate, and recovering a detoxified lignocelluloses hydrolysate from the culture.

43. A process for the production of Furoyl-CoA dehydrogenase, Furoyl-CoA synthetase, 2-oxoglutaroyl-CoA hydrolase, 2,5-furan-dicarboxylic acid decarboxylase 1, 2,5-furan-dicarboxylic acid decarboxylase 2 and/or a HMF/furfural oxidoreductase which are at least 45% identical to those expressed by Cupriavidus basilensis HMF14 DSM 22875, or a host microorganism according to claim 4, comprising
(a) culturing a microorganism in a nutrient medium containing carbon and nitrogen sources and inorganic salts; and
(b) isolating the enzymes produced from the microorganism.

44. A process for the conversion of 5-hydroxymethylfurfural (HMF), 2,5-dihydroxymethyl furan (HMF alcohol), 5-hydroxymethyl-2-furancarboxylic acid (HMF acid) and/or 2,5-furandicarboxylic acid to 2-furoyl CoA, comprising comprising contacting furfuryl alcohol and/or furfural with **a** furoyl-CoA dehydrogenase, furoyl-CoA synthetase, 2-oxoglutaroyl-CoA hydrolase, and 2,5-furan-dicarboxylic acid decarboxylase catalyst in the presence of one or more coenzyme cofactor.

45. A process according to claim 44, wherein the catalyst comprises a polypeptide according to claims 9, 11, 17, 19 and/or 21.
